(19) Europäisches Patentamt / European Patent Office / Office européen des brevets

(11) EP 2 281 606 A1

(12) **EUROPEAN PATENT APPLICATION**
published in accordance with Art. 153(4) EPC

(43) Date of publication:
09.02.2011 Bulletin 2011/06

(51) Int Cl.:
*A61N 5/06* (2006.01)

(21) Application number: 09738613.0

(22) Date of filing: 23.04.2009

(86) International application number:
PCT/JP2009/001874

(87) International publication number:
WO 2009/133676 (05.11.2009 Gazette 2009/45)

(84) Designated Contracting States:
AT BE BG CH CY CZ DE DK EE ES FI FR GB GR HR HU IE IS IT LI LT LU LV MC MK MT NL NO PL PT RO SE SI SK TR
Designated Extension States:
AL BA RS

(30) Priority: 28.04.2008 JP 2008117511

(71) Applicant: Panasonic Corporation
Kadoma-shi
Osaka 571-8501 (JP)

(72) Inventors:
• YAGI, Hiroshi
Chuo-ku, Osaka 540-6207 (JP)
• SHIMIZU, Masanori
Chuo-ku, Osaka 540-6207 (JP)
• HIGASHI, Toru
Chuo-ku, Osaka 540-6207 (JP)
• KOBAYASHI, Yuki
Chuo-ku, Osaka 540-6207 (JP)

(74) Representative: Unland, Jochen Hermann
Eisenführ, Speiser & Partner
P.O. Box 10 60 78
28060 Bremen (DE)

(54) **FLUORESCENT LAMP**

(57)    Fluorescent lamp 100 has a light color with a correlated color temperature higher than 10000 [K] and lower than 17000 [K] and Duv in a range of -2.5 to 5, comprising arc tube 10 and phosphor layer 20 which includes at least: a red emitting rare-earth phosphor with a main emission peak (11) in 605-625 [nm]; a green emitting rare-earth phosphor with a main emission peak (12) in 540-550 [nm]; and a blue emitting rare-earth phosphor with a main emission peak (13) in 440-460 [nm], an action function efficiency of melatonin suppression for per unit luminous flux is higher than 1.0, and a general color rendering index Ra is 80 or higher. With the stated structure, the fluorescent lamp 100 suppresses melatonin secretion thereby achieving effects of adjusting biological rhythms and promoting wakefulness of living bodies, and also remedies the shortcomings in color rendering characteristics of the light source.

FIG. 11

EP 2 281 606 A1

Printed by Jouve, 75001 PARIS (FR)

**Description**

**[Technical Field]**

The present invention relates to a fluorescent lamp which provides photostimulation pertaining to secretion and suppression of melatonin of a living body.

A fluorescent lamp of the present invention is a low-pressure lamp which has a phosphor applied therein, such as a compact fluorescent lamp, a hot cathode fluorescent lamp, a cold cathode fluorescent lamp, an electrodeless fluorescent lamp, and a dielectric barrier fluorescent lamp. A lighting apparatus of the present invention is a lighting apparatus including an irradiation apparatus in which the fluorescent lamp of the present invention is incorporated.

**[Background Art]**

In recent years, melanopsin which contributes to regulation of photoperiodic biological rhythms was discovered in human bodies, and a study is conducted to develop a technology to realize lighting sources that are able to suppress melatonin secretion by photostimulating melanopsin, thereby adjusting the biological rhythms and promoting wakefulness of living bodies.

The spectral absorption sensitivity of melanopsin is considered to be high to short-wavelength visible light. This fits conventional knowledge that when giving photostimulation to a human being to suppress melatonin secretion, use of short-wavelength visible light is effective.

With such a technical background, an illuminating light source which, as a result of its spectral power of short-wavelength visible light being enhanced, has a bluish-white high-color-temperature light color corresponding to a high correlated color temperature is nearly realized. This illumination light source is intended to suppress melatonin secretion, thereby adjusting biological rhythms and promoting wakefulness of living bodies.

Conventional fluorescent lamps and lighting apparatuses which provide photostimulation related to secretion and suppression of melatonin of living bodies are disclosed by, for example, Patent Literature 1, i.e. Japanese Laid-Open Patent Application Publication No. 2005-529462, Patent Literature 2, i.e. Japanese Laid-Open Patent Application Publication No. 2004-508106, and Patent Literature 3, i.e. Japanese Laid-Open Patent Application Publication No. H6-314595.

Patent Literatures 1 and 2 each disclose a lighting source with a correlated color temperature of 6500 [K] or higher and a general color rendering index Ra of 65 or higher, as a lighting source intended to suppress secretion of melatonin and accordingly adjust biological rhythms and promote wakefulness of living bodies.

Patent Literature 3, which precedes Patent Literatures 1 and 2, also discloses a lighting source with a correlated color temperature of 6000 [K] or higher, as a lighting source intended to suppress secretion of melatonin and accordingly adjust biological rhythms and promote wakefulness of living bodies. In view of an issue discussed therein on reducing discomfort which arises from changing the light color of lighting located indoor (houses, offices, etc.) to that of a high color temperature, one skilled in the art would easily presume, based on general knowledge, that the general color rendering index is close to a recommended value of the indoor lighting (for example, the general color rendering index Ra of 60 or higher).

However, concerning an action spectrum indicating suppression of melatonin secretion for adjusting biological rhythms and promoting wakefulness of living bodies, there are currently still various views, and no unique determination has been made.

Besides, although not intended to suppress melatonin secretion to adjust biological rhythms and promote wakefulness of living bodies, some light sources mainly emit short-wavelength visible light and have a bluish-white light color with a high correlated color temperature, and some light sources have an ultra-high color temperature and high color rendition.

As an example, Patent Literature 4, i.e. Japanese Laid-Open Patent Application Publication H4-284347 discloses a broad-band fluorescent lamp. This fluorescent lamp is intended to reproduce a color tone of clear north sky, and has a correlated color temperature of 9000 [K] to 13000 [K] and achieves the high 90s for the general color rendering index and special color rendering indexes.

However, in general, a blue-emitting phosphor is low in emission efficiency. Accordingly, the emission efficiency becomes lower at an ultra-high color temperature. In addition, because the fluorescent lamp according to Patent Literature 4 is a broad-band fluorescent lamp, the emission efficiency becomes even lower. Thus, in view of the emission efficiency, it is desirable that a fluorescent lamp be a narrow-band fluorescent lamp with improved emission efficiency.

Yet, conventionally, a desirable emission spectrum by a narrow-band light source at an ultra-high color temperature, problems related to the color rendering thereof and the like have not been found out. Also, an influence of a positional relationship with the Planckian locus and a chromaticity range of a light color corresponding to the correlated color temperature have been unknown.

Accordingly, it has been necessary to study these using a value indicated by Duv and the like. Duv is a value calculated according to the calculation procedures specified by JIS Z8725-1999 and obtained by multiplying deviation of u and v values from the Planckian Locus having the corresponding color temperature, by 1000, on the CIE 1960 UCS chromaticity coordinates. Duv takes on a negative value when located below the Planckian Locus.

On the other hand, with regard to action spectra for suppression of melatonin secretion, there are various theories and no unique determination has been made. Accordingly, there exists no clear means to optimize the action spectra for lighting sources currently. However, the inventors of the present invention have found out that concepts with respect to the action spectra can be roughly categorized into some groups.

The first concept includes relatively simple action spectra determined based on the spectral absorption characteristics of melanopsin which is the most fundamental photoreceptive material. The second concept includes relatively complex action spectra determined by directly measuring effects on melatonin suppression in cases where actual spectra are given to human beings. Patent Literatures 1 to 3 are based on one of these two concepts on action spectra.

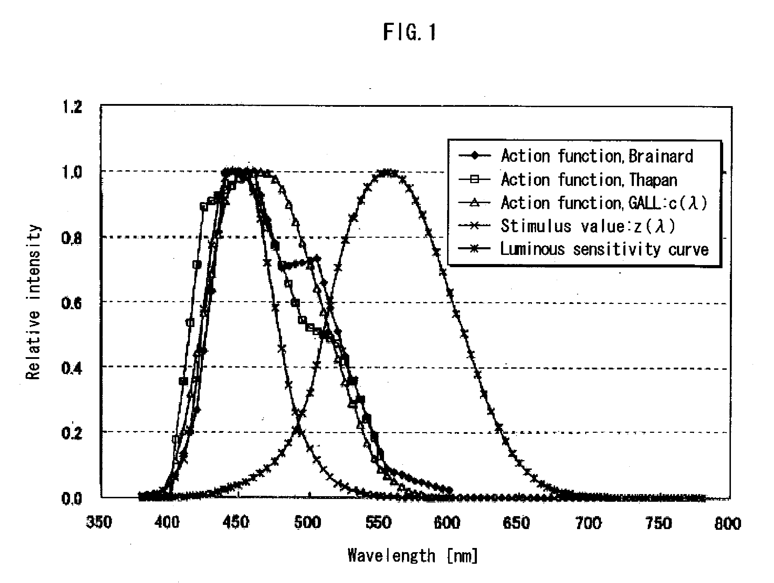

FIG. 1 shows a representative action function of the spectral absorption of melanopsin, action functions of melatonin suppression, and a standard spectral luminous efficiency curve.

With regard to action spectra for suppressing melatonin secretion and accordingly adjusting biological rhythms and promoting wakefulness of living bodies, the first concept is a relatively simple spectral sensitivity model (for example, GALL:C($\lambda$) in the figure). This model is based on the spectral absorption characteristics of melanopsin, the spectral sensitivity of photo-sensitive cells, and is approximated using, for example, visual pigment templates.

The second concept is sometimes regarded as a model which considers the following in an integrated manner (for example, Brainard and Thapan in the figure) : complex influence of cone cells and rod cells of eyes on the spectral sensitivity; effects of ophthalmological optics with spectral transmission characteristics of, such as, pupil contraction, crystalline lens, and vitreum of eyeballs; high-order reaction within living bodies; and complex biological reaction as light spectral reaction including optic nerves and hormone systems.

As is apparent from the above, action spectra are currently under technical study. Consequently, conventionally, the correlated color temperature is simply increased based on the knowledge that enhancing blue emission of short-wavelength visible light is preferable, to achieve a light source intended to suppress melatonin section at a high color temperature and accordingly adjusts biological rhythms and promotes wakefulness of living bodies.

Accordingly, conventionally, there has been no specific discussion on preferable deviation from the Planckian locus (Duv), preferable spectral distributions and the like.

Furthermore, no discussion has been made on deterioration of a visual environment that occurs as a counteraction from a decrease in color rendering properties due to a higher color temperature in application to actual illumination. Accordingly, problems arising from color rendering properties unique to the ultra-high color temperature, which cannot be expressed simply by values of the general color rendering index Ra and the color rendering indexes Ri, bases of the phenomenon, and measures for improvement have not been found.

In addition, in view of the fact that conditions for lamp use such as illumination intensity and environments are various, relatively simple action spectra (hereinafter, referred to as first-type action spectra) determined based on the spectral absorption characteristics of melanopsin which is the most fundamental photoreceptive material, and relatively complex action spectra (hereinafter, referred to as second-type action spectra) determined by directly measuring effects on melatonin suppression in cases where actual spectra are given to human beings should be optimized in an integrated manner. However, there has been no attempt for such optimization.

**[Citation List]**

[Patent Literature]

    [Patent Literature 1] Japanese Laid-Open Patent Application Publication No. 2005-529462
    [Patent Literature 2] Japanese Laid-Open Patent Application Publication No. 2004-508106
    [Patent Literature 3] Japanese Laid-Open Patent Application Publication No. H6-314595
    [Patent Literature 4] Japanese Laid-Open Patent Application Publication No. H4-284347

**[Summary of Invention]**

**[Technical Problem]**

In view of the above, the present invention aims to provide an ultra-high color temperature fluorescent lamp that has a color temperature higher than the highest lamp color 7100 [K] specified for fluorescent lamps for general lighting by JIS Z9112:1990, IEC 60081-1997 and the like. Here, the ultra-high color temperature fluorescent lamp suppresses melatonin secretion and accordingly achieves function effects of adjusting biological rhythms and promoting wakefulness of living bodies. Furthermore, the nature of the weakness in terms of color rendering properties of such a light source is found, and consequently, the present invention aims to provide the above-mentioned ultra-high color temperature fluorescent lamp as an illumination light source while preventing deterioration of the emission efficiency and further improving the emission efficiency.

One essential problem is that increasing blue emitting components at a high correlated color temperature in order to suppress melatonin section efficiently renders the light color bluish white, which decreases color rendering properties. On the other hand, improving the color rendering properties results in a decrease in the emission efficiency of the light source. Additionally, in the ultra-high color temperature region, simply increasing the general color rendering index Ra (CRI), special color rendering indexes Ri and the like will not improve the color appearance of the illuminated object correspondingly.

The present invention has found out that the nature of the deterioration of the color rendering properties of an ultra-high color temperature light source is lack of red color emission in the ultra-high color temperature light. However, merely adding red color emission in order to improve the red appearance under the bluish white light with the ultra-high color temperature leads to a further decrease in the emission efficiency, which already has decreased due to the ultra-high color temperature, and would not be able to increase the efficiency of melatonin suppression which is sensitive to short-wavelength visible light.

Thus, the present invention aims to improve the efficiency of melatonin suppression, which is highly sensitive to short-wavelength visible light, while also improving the color rendering characteristics (especially color rendering properties of red) by enhancing emission of green which is a supplementary color of red, instead of enhancing the red-emitting spectral band. In the present invention, particularly, emission of a blue green region is enhanced.

Here, in an attempt to improve the lack of red emission, which is the essential cause of the deterioration of the color rendering properties of the ultra-high color temperature light source, it has been found out that conventional evaluation indexes such as Ra and Ri are not capable of evaluating this essential problem. This is because the reference light source (reference light) itself varies with the correlated color temperature.

In view of the above, the present invention realizes ultra-high temperature illumination similar to light from a blue sky by optimizing unconventional color rendering characteristics at an ultra-high color temperature, including not only the simple Ra and Ri but also the color gamuts. For the above-stated realization, a new evaluation method has been developed in the present invention. This new evaluation method optimizes not only color gamuts defined by color chips used for evaluating and calculating Ra, which represent appearance of objects with medium chroma often found among natural objects, but also color gamuts defined by color chips used for evaluating and calculating Ri, which represent appearance of colors used with intention to render objects appear vivid. According to this method, a further improvement is achieved on a conventionally unrecognized state where although the appearance of medium chroma colors is improved, the appearance of high chroma colors is not improved.

Accordingly, in the embodiments of the present invention, melatonin suppression and an improvement of color rendering properties are both optimized multidimensionally. In addition, unlike in cases where an ultra-high color temperature is simply realized using a conventional three-wavelength light source, Duv takes on a positive value, and color rendering indexes such as Ra and Ri are high, or improved (Duv is a value obtained by multiplying deviation of u and v values from the Planckian Locus having the corresponding color temperature, by 1000, on the CIE 1960 UCS chromaticity coordinates defined by JIS Z8725-1999. Duv takes on a negative value when located below the Planckian Locus. In general, among lamps with the same structure, those with a higher Duv value are likely to emit greenish light, which increases luminous efficiency, and accordingly, emission efficiency).

Furthermore, the light source according to the present invention achieves a value higher than the reference gamut area of 100 for a gamut area Ga4 (a newly developed index which is described later) defined by high-chroma color chips for color rendering evaluation, in addition to the gamut area Ga defined by medium chroma color chips for color rendering evaluation. This contributes to ability to perform more preferable and vivid color rendition than the light for reference (reference light). Conventionally, it is difficult for an ordinary ultra-high color temperature fluorescent lamp developed based on a three-wavelength light source to achieve a value above 100 of the reference gamut area for the gamut area Ga4.

In the ultra-high color temperature region, Duv of natural light (skylight) often has a positive value. Thus, the above-described features, i.e., Duv of a positive value and improvement on the color rendering characteristics are extremely advantageous for realizing the present invention in a preferable manner, and also achieves an effect that the emission efficiency of the lump increases when Duv is a positive value.

Presently, light sources for realizing ultra-high color temperature fluorescent lamps and suppressing melanopsin secretion, thereby being capable of adjusting biological rhythms and promoting wakefulness of living bodies include the following: a fluorescent lamp with a correlated color temperature of 8000 [K] and a fluorescent lamp with a correlated color temperature of 17000 [K], both having spectral distributions corresponding to three-wavelength fluorescent lamps.

In general, a three-wavelength fluorescent lamp manufactured in a conventional manner approximately has the following values, where the correlated color temperature is 8000 [K] and Duv = 0:

general color rendering index Ra = 83; and
special color rendering indexes R9 = 40, R10 = 47, R11 = 67, R12 = 60, R13 = 94, R14 = 71, and R15 = 98.
When the correlated color temperature is 17000 [K] and Duv = 0, the fluorescent lamp approximately has the following values:

general color rendering index Ra = 82; and
special color rendering indexes R9 = 42, R10 = 46, R11 = 63, R12 = 59, R13 = 92, R14 = 72, and R15 = 94.
In the above cases, the color rendering indexes represented by ordinary Ra and Ri indicate high values, and the value of R9 which is an index for appearance of vivid red improves as the correlated color temperature rises. However, there is a problem here.

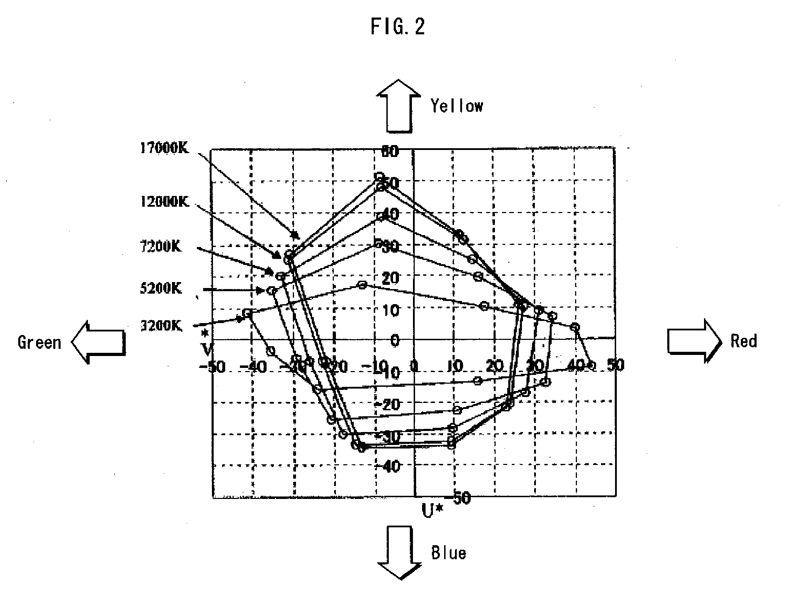

FIG. 2 shows chromaticities of R1 to R8, which are color chips used for calculating the general color rendering index at various correlated color temperatures, on U*V* chromaticity coordinates used for calculating color rendering indexes. In the figure, each set of eight color chips defines a color gamut.

In the figure, the rightward direction, the leftward direction, the upward direction, and the downward direction indicate chromas of red, green, yellow, and blue, respectively, and the further away from the origin, the more vividly the color of the color chip is reproduced.

When the reference light source (reference light) has a strongly yellowish light color with a correlated color temperature of 3200 [K], the reference light source itself has little short-wavelength visible light spectrum, the appearance (chroma) of colors in the blue and yellow directions is low, and the color gamut is in a vertically compressed shape.

When the reference light source (reference light) has a bluish white light color with a correlated color temperature of 17000 [K], the reference light source itself has little short-wavelength visible light spectrum, the appearance (chroma) of colors in the red and green directions is low, and the color gamut is in a horizontally compressed shape.

The general color rendering index Ra and the special color rendering indexes Ri numerically evaluate whether faithful color reproduction is realized or not with respect to the reference light source of the corresponding correlated color temperature, based on a color difference. However, because the appearance of the reference color is different for each correlated color temperature, appearances of the colors of the light sources which largely differ from each other in correlated color temperature cannot be evaluated by simple comparison.

At an ultra-high color temperature, the chromas of the color chips for evaluation illuminated with the reference light are low in the red and green directions. Consequently, as the correlated color temperature of the light source to be evaluated becomes higher, the evaluation values such as Ra and Ri tend to be high despite the actual appearances.

Here, it has been further found that the gamut areas which are subjectively determined to be preferable at an ultra-high color temperature are in a shape that is, like an ordinary light source with a relatively low correlated color temperature, stretched in the horizontal direction. This shape increases vivid appearance of red and green.

Particularly, in the case of the special color rendering index R9 for red, the actual appearance dulls at an ultra-high color temperature due to a decrease in chroma if the numerical values are merely maintained. The dull appearance of red under an ultra-high color temperature of bluish light leads to an uncomfortable visual environment peculiar to ultra-high color temperature light sources.

When using the conventional R9, it is more preferable that the higher the correlated color temperature becomes, the higher the value of R9.

The CIE (International Commission on Illumination) specifies grades of light sources in terms of color rendering properties. According to the grade specification, a three-wavelength light source of a color rendering group 2 has the general color rendering index Ra of 80 or higher. However, the results of the experiments on the subjective appearance evaluation indicated the following as the minimum requirements to be met for a light source having a correlated color temperature exceeding the highest light color 7100 [K] specified for fluorescent lamps for general lighting: the general color rendering index Ra of 80 or higher; and the value of the special color rendering index R9, which indicates appearance of red, exceeding a range of 28 to 46 and reaching 50 when rounded off, where the range of 28 to 46 being realized in general at a correlated color temperature around a range of 6000 [k] to 7100 [K] specified as the highest light color range for fluorescent lamp for general lighting.

Furthermore, the subjective evaluation results indicated that, in comparison to an ultra-high color temperature simply realized with an ordinary three-wavelength light source, a more significant and more preferable value of R9 was 60 or higher, and optimally, 80 or higher.

Also, according to a more preferable practice of the present invention, an extremely high color rendering level can be realized with Duv of a positive value, as follows: the general color rendering level Ra of 90 or higher, which corresponds to color rendering group 1A specified by the CIE (International Commission on Illumination).

This clearly differs in color rendering property from cases where a high color temperature is realized simply by a general three-wavelength light source. The evaluation is made not merely with numerical figures such as Ra and Ri; more preferably, Duv is positive, and the gamut area Ga is equal to or higher than 100, which is the gamut area value of the reference light.

Further preferably, the gamut area Ga4 defined by the newly configured special color rendering indexes R9 to R12 indicate 95 or higher. Being close to 100, this value is hardly achievable in a case where a high color temperature is simply realized by a general three-wavelength light source, and indicates that vivid colors can be reproduced almost as if under the reference light.

When the gamut areas Ga and Ga4 indicate values equal to or higher than 100, which is the value assigned to the appearance under the reference light, the numerical values such as Ra and Ri may apparently decrease. This is because Ra and Ri are evaluated based on the color difference from the values indicated on the chromaticity coordinates by the color chips illuminated with the reference light of the corresponding correlated color temperature; thus, even when the colors are rendered more vividly in a preferable manner, they are evaluated negatively as mere color deviation. A preferable case of the present invention derives a relationship between the features of the spectra and color rendering effects from a numerical decrease due to the preferable color deviation as described above. Conventionally, such a relationship is ignored because Ra and Ri have deteriorated.

As described above, the following problem has been found in the present invention: for a light source with an ultra-high color temperature that exceeds the highest light color 7100 [K] of fluorescent lamps for general lighting, the general color rendering index Ra is maintained high and the special color rendering index R9, which indicates appearance of red, tends to be high, in terms of numerical values; in reality, however, red becomes less perceivable at an ultra-high color temperature. Thus, even if an ultra-high color temperature fluorescent lamp is realized to suppress melatonin secretion and accordingly adjust biological rhythms and promote wakefulness of living bodies, and the numerical values such as Ra and Ri are high, the realized fluorescent lamp provides unnatural illumination in actual visual environments. This is because red and its supplementary color green look faded under a bluish white light color. For example, such illumination renders the colors of the complexion and lips appear blood-drained, giving a pasty-faced impression, and makes natural objects such as plants and flowers look faded without sufficient green and red colors.

The present invention solves these problems related to the color rendering of an ultra-high color temperature fluorescent lamp.

On the other hand, when an ultra-high color temperature fluorescent lamp having a high spectral power in the short-wavelength visible light region is to be realized, the emission efficiency deteriorates with an increase in the color temperature, since in general, the emission efficiency of a blue emitting phosphor is low.

In the current situation described above, when leaving the emission efficiency out of consideration, it is possible to improve Ra and R9 by reproducing a continuous spectrum of sunlight using a phosphor which emits a broad-band continuous spectrum, to maximize the color rendering properties, as disclosed by Patent Literature 4, i.e. Japanese Laid-Open Patent Application Publication H4-284347.

However, the emission efficiency of the broad-band continuous spectrum is lower than that of a narrow-band light source. In addition, it is difficult to improve the appearance (chroma) of red and green colors efficiently based on a broad-band continuous spectrum, compared to a narrow-band light source which concentrates the emission spectrum in a specific wavelength band.

On the other hand, in a case of realizing an ultra-high color temperature light source for achieving effects of adjusting biological rhythms and promoting wakefulness of living bodies by suppressing melatonin secretion, using a narrow-band light source corresponding to a three-wavelength light source, it is difficult to affect an action function for melatonin suppression as much as sunlight of the corresponding correlated color temperature does. This is because with its spectrum focused to stimulate cone cells, the narrow-band light source is not able to focus emission to the spectral absorption band of the melanopsin visual pigments efficiently.

How the emission spectrum can be intensified while also improving the color rendering properties efficiently, instead of simply focusing emission at a peak of the melatonin suppression action at an ultra-high color temperature, remains unknown conventionally.

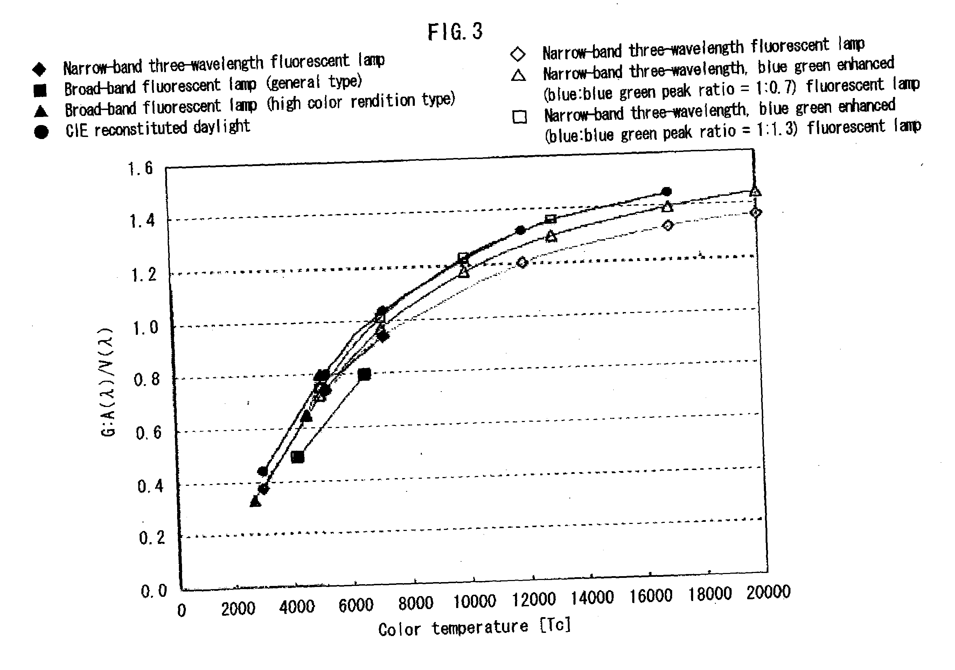

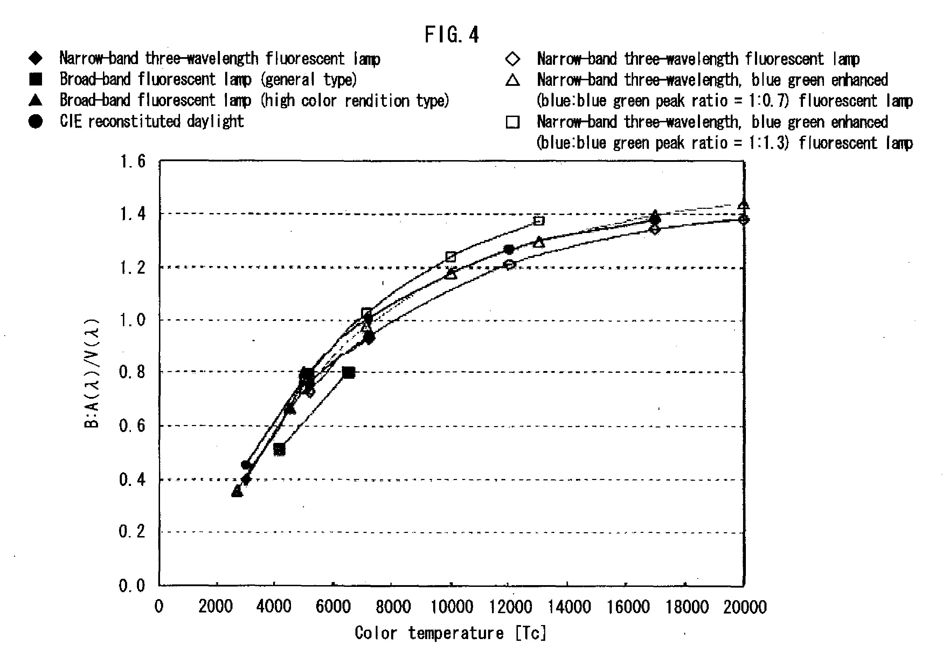

Next, FIGs. 3 and 4 show relationships between a correlated color temperature and action functions A ($\lambda$) of melatonin suppression. FIGs. 3 and 4 show comparison results with a first-type action function G(C($\lambda$)) and a second-type action function B(Brainard) as representatives, respectively.

The vertical axes in these figures each represent a value obtained by (light source spectral distribution $\times$ melatonin suppression action function A($\lambda$) )/(light source spectrum distributions $\times$ spectral luminous efficiency curve V($\lambda$)), where A($\lambda$)/ V($\lambda$) is equivalent to melatonin suppression efficiency for per unit luminous flux.

It is understood from FIGs. 3 and 4 that for both the broad-band light source and narrow-band light source, there is tendency that the stimulation ratio of the action function rises with an increase of the correlated color temperature.

As described above, the characteristic that an increase of short-wavelength visible light components intensifies melatonin suppression has been confirmed individually for various action functions of melatonin suppression. Accordingly, even conventionally, light sources with a relatively high color temperature have been realized to achieve the effect of this characteristic. Yet, conventionally it has not been found out that although spectral sensitivity characteristics of the various first-type action functions and second-type action functions differ among one another significantly, the results thereof have common tendencies.

In general, tracing the spectral absorption of visual pigments more directly, the first-type action functions form a simple curve with a relatively narrow half width; on the other hand, reflecting complex reactions in a living body in an integrated manner, the second-type action functions form a complex curve with a relatively wide half width.

In addition, as a result of adjusting and integrating the concept of the first-type action functions and the concept of the second-type action functions and conducting simulations using various action functions, the present invention has found out that although the spectral sensitivity characteristics of these two types differ from each other significantly, the results thereof have common tendencies. Furthermore, it has been found out that the tendencies of the actions functions of the two types often coincide in light sources based on narrow-band fluorescent lamps, in the ultra-high color temperature region.

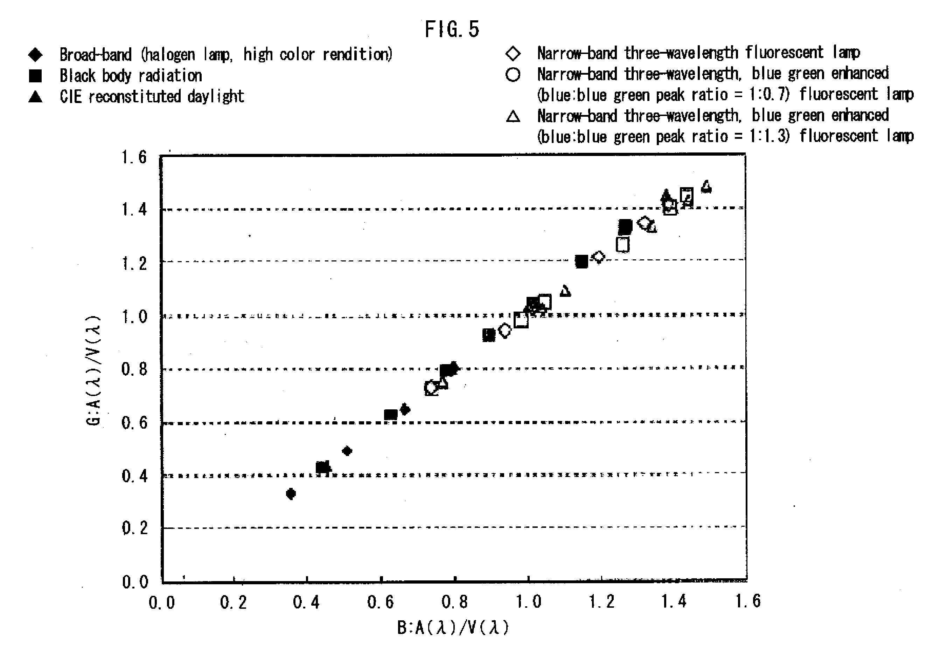

FIG. 5 shows, as an example, a correlation between the first-type action function G and the second-type action function B. It should be noted here that other action functions also exhibit high correlations. This is considered to mainly attribute to the following two aspects: bases of the various action functions are essentially correlated with the spectral sensitivity of melanopsin; and the spectral distributions are of light sources based on narrow-band fluorescent lamps which efficiently stimulate color channels of cone cells. Looking into the spectral distributions of the lamps classified according to features thereof, the spectral distributions sharing the same feature show very high correlations.

Also, while the second-type action functions particularly exhibit a large margin of errors near an extremely-short-wavelength visible light region around 400 [nm], these errors do not affect the calculation results largely as the region has little significance in the calculation of color rendition and emission efficiency.

Based on this knowledge, the present invention optimizes the characteristics of the color rendition, the light color and the like of an ultra-high color temperature fluorescent lamp as an illuminated visual environment, where the light color of the ultra-high color temperature fluorescent lamp exceeds the highest light color specified by JIS or IEC for fluorescent lamp for general lighting. This is a new unconventional approach.

Conventionally, a simple attempt to enhance melatonin suppression leads to deterioration of the color rendering properties (especially of red) as well as a decrease in the emission efficiency, resulting from the spectrum enhancement of short-wavelength visible light in the ultra-high color temperature region. Here, there is an obstructive factor for solving this problem: increasing the red emission spectrum in the long-wavelength visible light in an attempt to compensate for the insufficiency and to improve the color rendering properties leads to reduction of the melatonin suppression efficiency and the emission efficiency.

In order to solve the above problems, the present invention enhances emission in a blue-green region of a three-wavelength light source at an ultra-high color temperature. The purpose thereof is to realize an ultra-high color temperature fluorescent lamp with a light color exceeding the highest light color of the correlated color temperature 7100 [K] specified by JIS Z9112:1990, IEC 60081-1007 and the like for fluorescent lamp for general lighting. The realized ultra-high color temperature fluorescent lamp suppresses melatonin secretion and accordingly achieving effects of adjusting biological rhythms and promoting wakefulness of living bodies, and at the same time addresses the shortcomings of the color rendering characteristics unique to such an ultra-high color temperature light source, i.e., deterioration of appearance (chroma) of red and green colors.

Also, the present invention is able to achieve significant effects such as follows: when Duv has a positive value, Ra and Ri are high and Ga and Ga4 (described later) exceed 100. As a result, like skylight which is a natural ultra-high color temperature light, a light source which has high color rendition when Duv is positive and which has high emission efficiency because of Duv being positive can be realized. As a result, the present invention is able to provide a high-efficiency fluorescent lamp and a high-efficiency lighting apparatus that achieve both a biological function pertaining to melanopsin stimulation and unconventional color rendering properties in an efficient and natural visual environment.

**[Solution to Problem]**

In order to solve the conventional problems, the present invention provides the following measures. Note that Duv is a value obtained by multiplying deviation of u and v values from the Planckian Locus having the corresponding color temperature, by 1000, on the CIE 1960 UCS chromaticity coordinates defined by JIS Z8725-1999. Duv takes on a negative value when located below the Planckian Locus. Note also that the action function efficiency of melatonin suppression for per unit luminous flux is a ratio (action function efficiency/visibility efficiency) of an action function efficiency obtained by weighing, with an action function of melatonin suppression, the spectral power of the emission spectrum of the lamp and integrating the weighed value, to the visibility efficiency obtained by weighing, with the CIE spectral luminous efficiency $V(\lambda)$, the spectral power of the emission spectrum of the lamp and integrating the weighed value.

One aspect of the present invention is a fluorescent lamp having a light color with a correlated color temperature higher than 10000 [K] and lower than 17000 [K] and Duv in a range of -2.5 to 5, inclusive, the fluorescent lamp comprising: an arc tube; and a phosphor layer formed on an inner surface of the arc tube, wherein the phosphor layer includes at least: a red emitting rare-earth phosphor containing a rare-earth element as a light emission center with a main emission peak located in a range of 605 [nm] to 625 [nm], inclusive; a green emitting rare-earth phosphor containing a rare-earth element as a light emission center with a main emission peak located in a range of 540 [nm] to 550 [nm], inclusive; and a blue emitting rare-earth phosphor containing a rare-earth element as a light emission center with a main emission peak located in a range of 440 [nm] to 460 [nm], inclusive, an action function efficiency of melatonin suppression for per unit luminous flux is higher than 1.0, and a general color rendering index Ra is 80 or higher.

Another aspect of the present invention is a fluorescent lamp having a light color with a correlated color temperature higher than 7100 [K] and Duv in a range of -2.5 to 5, inclusive, the fluorescent lamp comprising: an arc tube; and a phosphor layer formed on an inner surface of the arc tube, wherein the phosphor layer includes at least: a red emitting rare-earth phosphor containing a rare-earth element as a light emission center with a main emission peak located in a range of 605 [nm] to 625 [nm], inclusive; a green emitting rare-earth phosphor containing a rare-earth element as a light emission center with a main emission peak located in a range of 540 [nm] to 550 [nm], inclusive; a blue emitting rare-earth phosphor containing a rare-earth element as a light emission center with a main emission peak located in a range of 440 [nm] to 460 [nm] inclusive; and a phosphor containing an element as a light emission center with a main emission peak located in a range of 480 [nm] to 520 [nm], inclusive, between the main emission peak of the blue emitting rare-earth phosphor and the main emission peak of the green emitting rare-earth phosphor, wherein the phosphor containing the element as the light emission center with the main emission peak located in the range of 480 [nm] to 520 [nm], inclusive, photostimulates melanopsin by enhancing emission between the main emission peak of the blue emitting rare-earth phosphor and the main emission peak of the green emitting rare-earth phosphor, thereby increasing a melatonin suppression efficiency, a general color rendering index Ra is 80 or higher, and a special color rendering index R9 is 50 or higher.

Yet another aspect of the present invention is a fluorescent lamp having a light color with a correlated color temperature higher than 7100 [K] and Duv in a range of -2.5 to 5, inclusive, the fluorescent lamp comprising: an arc tube; and a phosphor layer formed on an inner surface of the arc tube, wherein the phosphor layer includes at least: a red emitting rare-earth phosphor containing a rare-earth element as a light emission center with a main emission peak located in a range of 605 [nm] to 625 [nm], inclusive; a green emitting rare-earth phosphor containing a rare-earth element as a light emission center with a main emission peak located in a range of 540 [nm] to 550 [nm], inclusive; and a blue emitting rare-earth phosphor containing a rare-earth element as a light emission center with a main emission peak located in a range of 440 [nm] to 460 [nm] inclusive, wherein the blue emitting rare-earth phosphor containing the rare-earth element as the light emission center with the main emission peak located in the range of 480 [nm] to 520 [nm], inclusive, has a half width of 50 [nm] or greater from an emission peak toward a longer wavelength and photostimulates melanopsin by enhancing emission between the main emission peak of the blue emitting rare-earth phosphor and the main emission peak of the green emitting rare-earth phosphor, thereby increasing a melatonin suppression efficiency, a general color rendering index Ra is 80 or higher, and a special color rendering index R9 is 50 or higher.

As a preferred embodiment, the correlated color temperature of the above-described fluorescent lamp may be in a range of 10000 [K] to 20000 [K], inclusive.

As another preferred embodiment, the correlated color temperature of the above-described fluorescent lamp may be in a range of 11000 [K] to 13000 [K], inclusive.

As another preferred embodiment, Duv of the above-described fluorescent lamp may be greater than 0.

As another preferred embodiment, at least one of the phosphors of the above-described fluorescent lamp is composed of SCA.

As another preferred embodiment, in the above-described fluorescent lamp, the blue emitting rare-earth phosphor containing the rare-earth element as the light emission center with the main emission peak located in the range of 440 [nm] to 460 [nm], inclusive, may be composed of one or more of BAM:Mn, BAM and SCA, and the blue emitting rare-earth phosphor containing the rare-earth element as the light emission center with the main emission peak located in the range of 480 [nm] to 520 [nm], inclusive, is composed of one or more of BAM:Mn, BAM, SAE, CMZ, and BCA.

As another preferred embodiment, in the above-described fluorescent lamp, the blue emitting rare-earth phosphor containing the rare-earth element as the light emission center with the main emission peak located in the range of 440 [nm] to 460 [nm], inclusive, and the phosphor containing the element as the light emission center with the main emission peak located in the range of 480 [nm] to 520 [nm], inclusive, may be each composed of a BAM:Mn phosphor.

As another preferred embodiment, in the above-described fluorescent lamp, the red emitting rare-earth phosphor containing the rare-earth element as the light emission center with the main emission peak located in the range of 605 [nm] to 625 [nm], inclusive, may be composed of one or a plurality of YOX, YVO, and YOS, the green emitting rare-earth phosphor containing the rare-earth element as the light emission center with the main emission peak located in the range of 540 [nm] to 550 [nm], inclusive, is composed of one or a plurality of LAP, CAT, CAT:Mn, and CBT, the phosphor containing the element as the light emission center with the main emission peak located in the range of 480 [nm] to 520 [nm], inclusive, is composed of one or a plurality of BAM:Mn, SAE, CMZ, BCA, CAT:Mn, and the blue emitting rare-earth phosphor containing the rare-earth element as the light emission center with the main emission peak located in the range of 440 [nm] to 460 [nm], inclusive, is composed of one or a plurality of BAM:Mn, BAM, and SCA.

**[Advantageous Effects of Invention]**

The present invention realizes an ultra-high color temperature fluorescent lamp with improved color rendering properties and a high efficiency by enhancing emission between blue emission and green emission of a fluorescent lamp which is constructed based on a narrow-band fluorescent lamp, the ultra-high color temperature fluorescent lamp achieving function effects of adjusting biological rhythms and promoting wakefulness of living bodies by suppressing melatonin secretion.

Enhancing at least emission between a main emission peak of a blue emitting rare-earth phosphor and a main emission peak of a green emitting rare-earth phosphor achieves higher melatonin suppression and at the same time improving the color rendering characteristics , that is, deterioration of appearance (chroma) of red and green, with respect to a narrow-band light source corresponding to a three-wavelength light source with the corresponding correlated color temperature.

This enables providing a fluorescent lamp and a lighting apparatus that achieve effects of adjusting biological rhythms and promoting wakefulness of living bodies by suppressing melatonin secretion in an efficient and natural visual environment while maintaining color rendering properties.

There are roughly two ways to enhance emission between the main emission peaks of the blue emitting rare-earth phosphor and the green emitting rare-earth phosphor.

The first way is to extend the half width from the emission peak of the blue emitting rare-earth phosphor (preferably 50 [nm] or more) toward a longer wavelength, whereby the above-mentioned effect can be achieved. The second way is to add a phosphor with an emission center whose main emission peak is located in a range between the emission peaks of the blue emitting rare-earth phosphor and the green emitting rare-earth phosphor (preferably from 480 [nm] to 520 [nm]).

It has been found out that especially by use of the latter way, preferably concentrating emission in the range of 480 [nm] to 520 [nm] when enhancing the emission between the main emission peaks between the blue emitting rare-earth phosphor and the green emitting rare-earth phosphor enables increasing Ra and Ri (especially R9) in a high Duv region, and it has been further found out that problems related to color rendition which these Ra, Ri and the like cannot express can be solved.

The region around 480 [nm] to 520 [nm] enhanced by the first and second ways is a region which has a relatively high luminosity sensitivity and contributes to relatively efficient melatonin suppression. Accordingly, a light source with an even higher efficiency is realizable, and also, the following can be improved : the ratio (action function efficiency/visibility efficiency) of the action function efficiency obtained by weighing, with the action function of melatonin suppression, the spectral power of the emission spectrum of the lamp and integrating the weighed value, to the visibility efficiency obtained by weighing, with the CIE spectral luminous efficiency $V(\lambda)$, the spectral power of the emission spectrum of the lamp and integrating the weighed value.

Also, in the present invention, evaluation indexes include, in addition to the color gamut Ga defined by R1 to R8 which are medium chroma color chips used for evaluating the general color rendering index, the color gamut Ga4 defined by high chroma color chips R9 to R12 used for evaluating the special color rendering indexes. With use of Ga4, a more appropriate evaluation can be performed on visual objects having high chroma.

Consequently, according to the present invention, a special effect can be achieved, that is, appearance of high chroma colors, as well as of medium chroma colors, can be improved. This effect is also significant when Duv is positive. Thus, it is possible to reproduce vivid colors as if under skylight, which is natural light having an ultra-high color temperature, and also achieve the melatonin suppression effect; and in addition, since Duv of skylight often takes on a positive value, the effect of the present invention being especially significant when Duv is positive is an advantage.

The following describes the effects of the present invention.

First, the melatonin suppression action is explained in detail.

FIGs. 3 and 4 show the relationships between the correlated color temperature and the action functions A ($\lambda$) of melatonin suppression. The vertical axes in these figures each represent a value obtained by (light source spectral distribution $\times$ melatonin suppression action function $A(\lambda)$ )/(light source spectrum distributions $\times$ spectral luminous efficiency curve $V(\lambda)$), where $A(\lambda)/ V(\lambda)$ is equivalent to melatonin suppression efficiency for per unit luminous flux.

It is understood from FIGs. 3 and 4 that for both the broad-band light source and narrow-band light source, there is tendency that the stimulation ratio of the action function rises with an increase of the correlated color temperature. Furthermore, this correlation is higher for light sources of the same type, such as broad-band light sources and narrow-band light sources.

At approximately 8000 [K], the spectral efficiency A($\lambda$)/V($\lambda$) of the action functions A ($\lambda$) of melatonin suppression and the spectral luminous efficiency curve V($\lambda$) strike a balance at approximately 1 to 1. Thus, it can be presumed that for a conventional three-wavelength fluorescent lamp, a correlated color temperature of 8000 [K] or higher enables an efficient achievement of the melatonin suppression effect.

Conventionally, when realizing an ultra-high color temperature fluorescent lamp that exceeds the highest light color 7100 [K] specified by JIS and IEC for fluorescent lamps for general lighting, the characteristic that the melatonin suppression intensifies with an increase of short-wavelength visible light components is not considered in an integrated manner. That is to say, the characteristic is considered from the viewpoint based on melanopsin visual pigments absorption, the viewpoint based on melatonin suppression effect action curve and the like individually. The present invention has found correlations among various action functions of melatonin suppression, and found out that these tendencies correlate and approximate with each other even after integrating these individual consideration.

For (a) the first-type action functions which reflect relatively simple concepts of action spectrum determined based on the spectral absorption characteristics of melanopsin, which is a photoreceptive material, and (b) the second-type action functions which reflect relatively complex action spectrum determined by directly measuring effects pertaining to melatonin suppression when specific spectra are given to a human being, melatonin suppression action functions A ($\lambda$) are verified, and furthermore, analysis is made separately for respective types of lamp spectral distributions, such as the broad-band type and the narrow-band type. As a result, it has been found out that the number of relative errors is small in a practical light source based on a narrow-band light source.

FIG. 5 indicates correlations for the respective light sources between the first-type action function G(C ($\lambda$)) represented by the vertical axis and the second-type action function B (Brainard) represented by the horizontal axis.

Despite significant differences in basic shapes of the spectral distributions of the black-body radiation, reconstituted daylight, and three-wavelength light sources, the figure shows high correlations among them. Correlations of the other action functions also show similar results, and when looking into the correlations in a more detailed manner for each basic shape of the spectral distributions of light sources, each result has been found to exhibit a high correlation and linearity.

It is understood from FIGs. 3 and 4 that A($\lambda$)/ V($\lambda$) constituted from a generally typical action function becomes 1 or greater when the correlated color temperature reaches about 8000 [K] or higher, exhibiting an increase in melatonin suppression effects with an increase of the correlated color temperature. However, the figures also indicate that this increase is close to saturation at around 20000 [K].

Here, an excessively high color temperature results in negative effects. Specifically, the light color becomes unnatural from being rendered extremely bluish white, and the spectral luminous efficiency of the lamp itself falls. It has been found that the emission efficiency significantly falls in the following manner due to an excessive high color temperature for a narrow-band light source corresponding to a three-wavelength light source, with respect to an ordinary light color around 5000 [K]: approximately 5% at 8000 [K], approximately 10% at 13000 [K], and approximately 15% at 17000 [K].

In general, in terms of industrial products, the emission efficiency of daylight D (corresponding to F6500) falls approximately 5% with respect to that of daylight white N (corresponding to F5000) as a result of an increase in color temperature. Accordingly, an emission efficiency fall of approximately 5% can be estimated for a fluorescent lamp having a color temperature which is one level higher than daylight D (corresponding to F6500). Based on the emission efficiency fall of approximately 5%, the light color one level higher than daylight D (corresponding to F6500) can be determined to have a correlated color temperature around 13000 [K] by backward calculation. In this case, a correlated color temperature around 17000 [K] would cause an emission efficiency fall of approximately 10%, and thus is determined to be a light color two level higher than daylight D (corresponding to F6500).

With differences of phosphors, the lamp structure, and so on of a fluorescent lamp that realizes these high color temperatures taken into consideration as error factors, about 15000 [K] between 13000 [K] and 17000 [K] is determined to be the highest allowable color temperature from the viewpoint of an emission efficiency fall resulting from an increase of the color temperature.

In addition, in FIGs. 3 and 4, the efficiency A($\lambda$)/ V($\lambda$) of typical natural light, namely CIE reconstituted daylight, for each correlated color temperature is plotted. The value of the efficiency A($\lambda$)/ V($\lambda$) of an ordinary three-wavelength fluorescent lamp is always lower than that of CIE reconstituted daylight when Duv = 0 at the corresponding correlated color temperature. This indicates that mere changes of the correlated color temperature do not enable a conventional three-wavelength fluorescent lamp to achieve as high A($\lambda$)/ V($\lambda$) as that of natural light having the corresponding correlated color temperature.

However, as in the present invention, enhancing at least the emission in the range of 480 [nm] to 520 [nm] between the main emission peaks between the blue emitting rare-earth phosphor and the green emitting rare-earth phosphor results in achieving a higher value for A($\lambda$)/ V($\lambda$) with respect to that of an ordinary three-wavelength fluorescent lamp with the corresponding correlated color temperature and Duv, and in an optimal case, achieving as high A($\lambda$)/ V ($\lambda$) as that of the natural light with the correlated color temperature corresponding to two significant digits.

Also, a value corresponding to A(λ)/ V(λ) of a light source with a higher correlated color temperature can be realized at a lower correlated color temperature, thereby preventing deterioration of appearance and emission efficiency due to an excessively high color temperature.

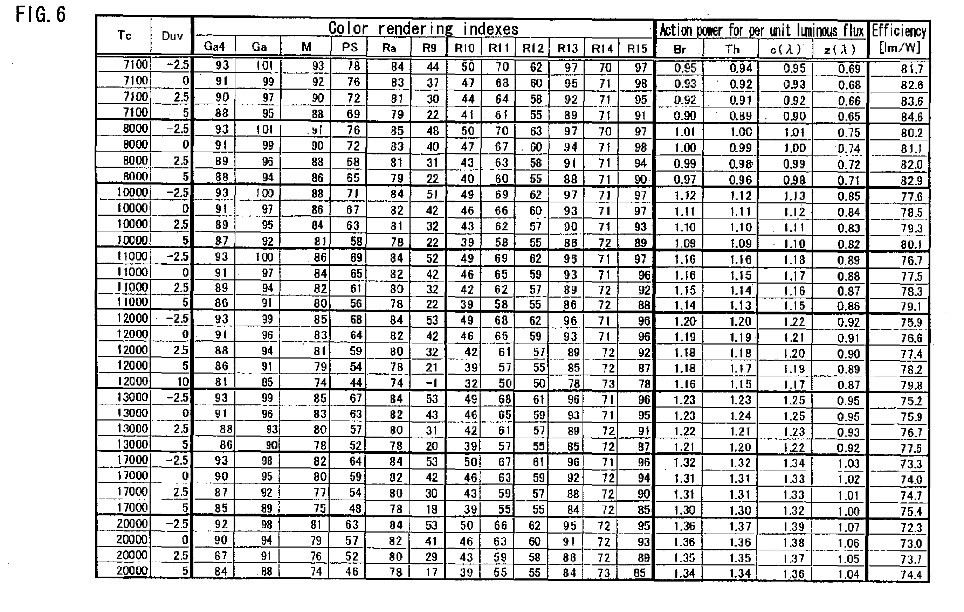

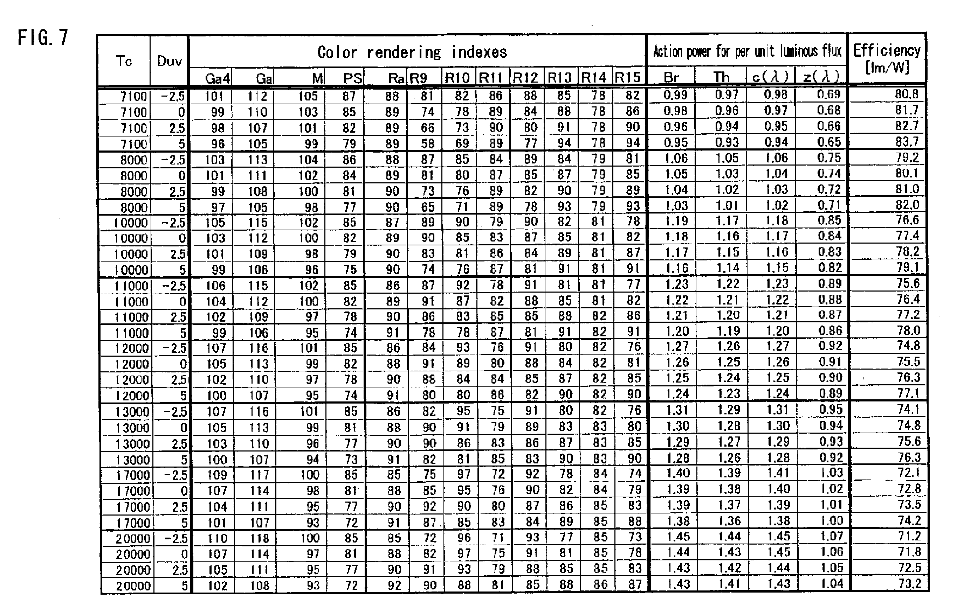

Here, comparison is made, at the correlated color temperature of 12000 [K] as an example, between an ordinary case based on a narrow-band light source corresponding to a three-wavelength fluorescent lamp, and a case, as with the present invention, based on a narrow-band light source corresponding to a three-wavelength fluorescent lamp, where the emission in the range of 505 [nm] to 525 [nm] between the emission peaks of the green emitting rare-earth phosphor and the blue emitting rare-earth phosphor is enhanced, with reference to tables shown in FIGs. 6 and 7.

FIG. 6 shows a case where a blue emitting phosphor BAM, a green emitting phosphor LAP, and a red emitting phosphor YOX are used in an ordinary three-wavelength fluorescent lamp. While it is also common to use a SCA phosphor having a half width of 50 [nm] or greater as the blue emitting phosphor, since the resulting value is substantially the same, description is omitted here.

FIG. 7 shows a case where a blue emitting phosphor BAM:Mn (also emits blue green), the green emitting phosphor LAP, and the red emitting phosphor YOX are used. In this case, the emission peak intensity ratio of the emission centers of the BAM:MN phosphor is as follows: when the emission intensity peak value of a rare-earth element Eu whose main emission peak is located in the range of 440 [nm] to 460 [nm] is assumed to be approximately 1.0, the emission intensity peak value of a Mn element emission center whose main emission peak is located in the range of 480 [nm] to 520 [nm] is approximately 0.7.

In the tables shown in FIGs. 6 and 7, the color rendering indexes are calculated according to the computation procedures specified by JIS Z8726-1990. In addition, Ga is calculated according to the computation procedure of gamut areas specified in "Evaluation method of color rendering properties other than color rendering indexes" of JIS Z8726-1990, and Ga4 is calculated according to a similar computation procedure similar to Ga. Generally, Ga is a gamut area on the U*V* chromaticity coordinates that is defined by medium chroma color chips R1 to R8 used for calculating the general color rendering index; and Ga4 is a gamut area defined by high croma color chips R9 to R12 which are newly-defined special color rendering indexes.

As a result, the gamut area defined by the high chroma color chips can be also taken into consideration in addition to the gamut area which is defined by the medium chroma color chips and is conventionally the only gamut area considered. Because the high chroma color chips are consistent with the conventional standards, the calculation thereof is consistent with the calculation of the widely applied gamut area Ga defined by the medium chroma color chips.

Regarding Ri of the high chroma color chips R9 to R12 which particularly require vividness, even if the color appears to have high chroma with Ri exceeding 100, conventionally it is only possible to determine that the index decreases based on the numerical value of Ri, due to lack of supplementary indexes. Consequently, although indexes for evaluating vividness of vivid colors are in greater need, there has been no support available.

Additionally, it has been found out that improving the medium chroma color gamut does not always improve the high-chroma color gamut, and this finding has led to the significant effect resulting from the configuration of the supplementary indexes which are consistent with the conventional standards and require minimum changes in the computation procedure. Specifically, with the supplementary indexes, it has become possible to accurately judge the appearance of the color chip R9, which deteriorates at an ultra-high temperature, without totally relying on the numerical value of R9.

Another reference index M in the tables is a value indicating vividness of the color rendition and can be derived according to a process disclosed in the following non-patent document: Kenjiro Hashimoto et. al., New Method for Specifying Color-Rendering Properties of Light Sources Based on Feeling of Contrast, Color research and application, Vol. 32, No. 5, October, P.361, 2007.

"PS" in the tables in FIGs. 6 and 7 is a value indicating preferability of the appearance of complexion color, and can be derived according to a process disclosed in the following non-patent document: Kenjiro Hashimoto et. al., Preference Index for Japanese Complexion Color under Illumination, Journal of the Illuminating Engineering Institute of Japan, Vol. 82, No. 11, p. 895, 1998.

"Br (Brainard)" in the tables in FIGs. 6 and 7 represents an action power of per unit luminous flux obtained using the second-type action function B as a representative, and is a typical value of the second-type action functions. "Th" is an action power for per unit luminous flux belonging to the second-type action functions, and is another value of the second-type action functions.

C(λ)" in FIG.s 6 and 7 represents an action power of per unit luminous flux obtained using the first-type action function G, and is a typical value of the first-type action functions. "Z(λ)" represents a referential action power of per unit luminous flux obtained using a first-type action function, and is another referential value of the first-type action functions. Z(λ) is an extreme case where the sensitivity peak is located in a shorter-wavelength region and the half width is narrow. This case is provided as a referential extreme case of which the sensitivity is close to that of cones which are highly sensitive to short-wavelength visible light (blue).

"Efficiency" in the tables shown in FIGs. 6 and 7 indicates an emission efficiency obtained with a 40W straight rapid-start type fluorescent lamp with a glass diameter of 32.5 [mm] and a glass length of 1198 [mm].

When Tc = 12000 [K] and Duv = 0, Ra of the conventional case shown in FIG. 6 is 82, while Ra of the present invention shown in FIG. 7 is 88.

Because the first-type action functions and the second-type action functions are correlated, assuming that Br of the second-type action function is a representative value, Br is 1.19 in the conventional case and 1.26 in the case of the present invention, where the correlated color temperature is 12000 [K] and Duv = 0. These indicate that the present invention improves the general color rendering index while also increasing the action power for per unit luminous flux of the efficiency $A(\lambda) / V(\lambda)$.

Moreover, the special color rendering index R9 indicating the appearance of red is 42 in the conventional case and 91 in the present invention, exhibiting a significant improvement of the appearance of red resulting from the structure of the present invention.

Additionally, according to the conventional structure of the fluorescent lamp realized based on a narrow-band light source corresponding to a three-wavelength light source, although the emission efficiency increases with an increase of Duv, Ra and Ri (especially R9) simply decrease.

However, according to the structure of the present invention based on a narrow-band light source corresponding to a three-wavelength fluorescent lamp, in which at least the emission in the range of 480 [nm] to 520 [nm] between the main emission peaks of the blue emitting rare-earth phosphor and the green emitting rare-earth phosphor is enhanced, Ra is high in regions where Duv is high, and simply put, Ra increases by contraries with an increase of Duv.

Also, with an increase of the correlated color temperature, R9 shows a high value when Duv is positive. Furthermore, other Ri such as R11 for green, R13 for leaf-green, and R15 for Japanese complexion clearly show behavior other than a simple decrease of the special color rendering indexes resulting from the change of Duv.

Conventionally, when realizing a fluorescent lamp based on a narrow-band light source corresponding to a three-wavelength fluorescent lamp, Duv is set to a negative value in order to secure high values for Ra and Ri. However, according to the present invention, the color rendering indexes increase even when Duv is a positive value. Accordingly, the synergistic effect of the increase of the emission efficiency and the improvement of Ra, Ri and the like can be attained.

In the cases with the correlated color temperature of 12000 [K], Ra, Ri and the like can be significantly improved while the emission efficiency which corresponds to Duv of about -2.5 according to the conventional structure is achieved at Duv of about 0 in the present invention. Also, conventionally, there is a case where the lowest value of Ra of the three-wavelength fluorescent lamp falls below 80 when Duv is around +2.5. In the present invention, however, Ra is maintained close to 90 even when Duv is +5, thus it is possible to set Duv at a higher value in order to achieve a high emission efficiency. Considering that Duv of the skylight having an ultra-high color temperature is positive, the above-mentioned characteristics are preferable for an ultra-high color temperature lamp.

The case shown in FIG. 7 pertaining to the present invention clearly indicates that the numerical value of R9 rises significantly with an increase of Duv especially in a case where the correlated color temperature is high. Also, according to FIG. 7, in a case where the correlated color temperature is relatively low, the numerical value of R9 rises once when Duv is around 0, and as the correlated color temperature increases, Ra starts increases at Duv of a higher value. Conventionally, even in cases where Ra, Ri and the like are considered, the effect of such changes in Duv are not taken into consideration.

Next, the effect of the present invention on color rendering is described in detail.

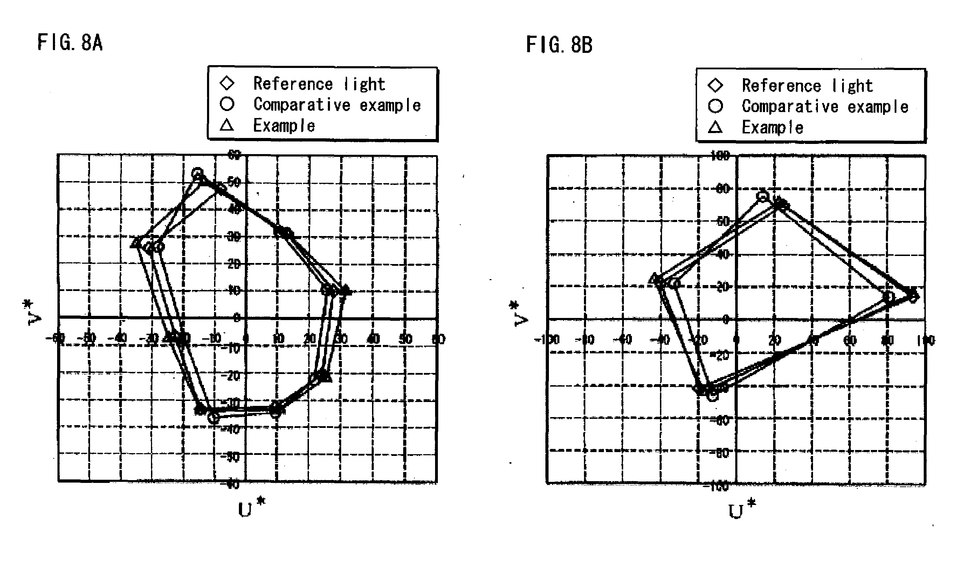

FIGs. 8 show color gamuts defined by the color chips R1 to R8 for calculating the general color rendering index (FIG. 8A) and color gamuts defined by the color chips for calculating the special color rendering indexes (FIG. 8B) on the U*V* chromaticity coordinates. In each figure, the color gamuts are constituted under the following combination of spectra, respectively: reference light (light for reference), a comparative example, i.e. a conventional structure (combination of BAM, LAP, and YOX phosphors), and the example, i.e. the structure of the present invention (combination of BAM:Mn, LAP, and YOX phosphors, with the ratio of the Eu peak of BAM:Mn to the Mn peak of BAM:MN being approximately 1.0 : 0.7). For all the cases in FIGs. 8, the correlated color temperature is 12000 [K] and Duv = 0.

Regarding the color gamuts defined by the medium chroma color chips R1 to R8 for calculating the general color rendering index, the color gamut pertaining to the conventional structure is narrow in the horizontal direction in the figure than that pertaining to the reference light having the corresponding correlated color temperature. In other words, the chroma of the appearance of the red and green color chips decreases.

In the case of the structure pertaining to the present invention, the color gamuts are wider in the horizontal direction in the figure than those pertaining to the reference light. In other words, the chroma of the appearance of red and green increases, and the shapes of the color gamuts become closer and even become larger than those pertaining to the reference light, as the distortion of the shapes of the gamut distributions decrease.

Especially the color gamut having the structure of the present invention defined by the high chroma color chips for calculating the special color rendering indexes R9 to R12 is significantly wider in the red direction, and also in the green direction than that having the conventional structure.

The general color rendering index Ra, the special color rendering indexes Ri and the like are indexes which are decreased based on how much the colors deviate with respect to the color reproductions by the reference light, which are given 100, having the corresponding correlated color temperature. Accordingly, regardless of how the colors are reproduced more vividly and more preferably with respect to those by the reference light of the corresponding correlated color temperature, the numerical values are decreased. According to the structure of the present invention, it is understood that 91 of R9 at the correlated color temperature of 12000 [K] is a number obtained as a result of reduction due to the colors such as red appearing more vivid than the color rendition using the reference light.

In the present invention, although the values of Ra, Ri and the like improve simply according to the evaluation of conventional Ra, Ri and the like, in the above-mentioned aspect, the solution the present invention offers with respect to the color rendering properties not only simply increase the numerical values of Ra, Ri and the like, but also achieve a higher level of improvement with respect to the color rendering properties.

Conventionally, JIS Z8726-1990 discloses, as reference, a method for calculating a color gamut defined by the eight kinds of medium chroma color chips R1 to R8 as a gamut area Ga.

According to this method, in the case of the conventional structure, Ga is 96; the gamut is smaller than the color gamut pertaining to the reference light having Ga of 100, and the color chips appear dull-colored. On the other hand, Ga pertaining to the present invention is 113; the color gamut is larger than the gamut pertaining to the reference light having Ga of 100, realizing vivid color reproduction.

Furthermore, in the present invention, a method for evaluating appearance of red in the ultra-high color temperature region is developed by structuring the value of the gamut ratio Ga4 defined by the four kinds of high chroma color chips R9 to R12.

Note that as is the case with Ga, information in the W* direction is not used for gamut area calculation. This is in order to share the calculation method (procedure) and make Ga a color gamut area pertaining to a hue and chroma on a plane, as is the case with Ga.

In a case of including increase and decrease of the gamut area in the brightness direction denoted as W*, an increase of the gamut area can be calculated three-dimensionally, for example when blue is rendered dark and yellow is rendered bright. However, to keep consistency with Ga, the gamut area is viewed as pertaining to the hue and chroma on the plane, and a difference due to such an increase of the gamut area is removed as a error factor.

According to the above-mentioned method, in the case of the conventional structure, Ga4 is 91; the gamut is smaller than the color gamut pertaining to the reference light having Ga4 of 100, and the color chips appear dull-colored. On the other hand, Ga4 pertaining to the present invention is 105; the color gamut is larger than the gamut pertaining to the reference light having Ga4 of 100, realizing vivid color reproduction.

In general, the high-chroma color gamut is not as easily improved as the medium chroma color gamut indicated with the gamut area Ga. The present invention, however, has found out that enhancing emission in a narrow band in the band of 480 [nm] to 520 [nm] especially at an ultra-high color temperature increases excitation purity of green, and as a result, the gamut area Ga4 significantly increases, including red, which is the supplementary color of green.

According to the conventional structure, when Duv is a positive value, Ga and Ga4 are both unlikely to exceed 100. However, in the present invention, when Duv is a positive number, Ga, Ga4 and the like are likely to be close to 100 or exceed 100, indicating that the present invention enables vivid color rendition which is more vivid than the values of Ra, Ri and the like imply.

Also, conventionally, variance in the Duv direction (especially in the positive range of Duv) for the same high correlated color temperature is not considered. The present invention intends to realize optimization in this aspect as well.

In a case of optimizing the spectrum of the present invention using unconventional evaluative viewpoints and evaluative indexes and then evaluating the addition of the optimized blue green emission according to the evaluation of the values of conventional Ra, Ri and the like, the evaluation results show the following: the color gamuts which became smaller as a result of the change in shape due to the ultra-high color temperature extend in the red and green directions and exhibit preferable color rendering properties even when Duv is high.

On the other hand, in the case of a conventional invention that simply increases Ra, Ri and the like without taking variance of Duv into consideration, the optimization of the spectrum cannot be performed in expectation of the above-mentioned effects because phenomena such as the following appear: when the color gamut becomes larger than the color gamut pertaining to the reference light, Ra, Ri and the like decrease; and the higher Duv, the lower Ra, Ri and the like.

Furthermore, conventionally, even when the conventional indexes such as Ra and Ri are high and Ga is high, there are cases where vivid red appears dull-colored. Thus, in the present invention, Ga4, i.e. the index for the color gamut defined by the vivid color chips that require vivid color rendition is configured, and with this structure, the appearance of the vivid colors can also improved.

The gamut area Ga4, composed of the color chips more vivid than the medium chroma color chips which make up the gamut area Ga, is not likely to improve in a conventional three-wavelength fluorescent lamp, and therefore it is difficult to find the above-mentioned tendencies only with this conventional Ga.

This is because that the spectral reflectivity of vivid colors have a steep rising portion and a steep trailing portion in the spectral curve thereof, and when the spectral curve is illuminated with a narrow-band light source, whether the spectrum hits the regions with high spectrum reflectivity or not makes a large difference. Consequently, the spectral setting of the narrow-band light source affects the color rendering properties more significantly.

In the present invention, the range of wavelength to be enhanced is set to 480 [nm] to 520 [nm], with the above-mentioned effect taken into account. As a result, the color gamut defined by R1 to R8, the color gamut defined by R9 to R12, or both of these color gamuts exhibit the features in terms of color reproduction, that is, extend in the green direction (left side), the red direction (right side), or in both of the directions farther with respect to the color gamuts pertaining to the reference light of the corresponding correlated color temperature.

Both Ga and Ga4 being higher than 100 indicates the result that the colors are reproduced vividly and preferably, exceeding 100 for the color reproduction by the reference light of the corresponding correlated color temperature, despite Ra, R9 and the like being small. However, while extreme improvements of Ga, Ga4, etc. increase the vividness of the colors, the result may be excessive from the viewpoint of faithful reproduction of the colors. Thus, it is desirable to increase Ga, Ga4 and the like when Ra, Ri and the like are high while also securing sufficiently faithful color rendition. From this viewpoint also, Duv of a positive value is a preferable range of the invention.

More specifically, focusing on the shape of the gamuts as well as on the size of the numerical values of the calculation results of the gamut areas reveals that gamuts similar in shape with those of the reference light with the corresponding correlated color temperature indicate that a balance is achieved among the colors. Furthermore, the shape of the color gamuts extending in the horizontal direction as is the shape of the color gamuts of the reference light of a lower correlated color temperature indicates that the colors are rendered in a manner to compensate the chroma decrease in the red-green directions due to the ultra-high color temperature, as if rendered by the reference light of a relatively low correlated color.

This enables the color of the object illuminated by bluish white light of a high correlated color temperature to appear natural as if illuminated by the reference light of a relatively low correlated color temperature, indicating that the light color with the high color temperature and the color rendition of the illuminated object are individually and appropriately determined.

Thus, the present invention does not constitute a simple design matter according to which the color rendition is determined as the light color is determined. According to the present invention, cases where the appearance of the colors improve in spite of decrease of Ra, Ri and the like are accurately judged. On the other hand, according to the conventional cases where designing is performed simply based on the level of Ra, Ri and the like, the above-described cases are not taken into consideration.

Also, Ra, Ri and the like evaluate whether the color reproduction pertaining to the gamuts are close to those pertaining to the corresponding reference light based on the degree of color difference. Accordingly, even in a case where the color rendition is more vivid in a preferable way than that pertaining to the reference light, Ra, Ri and the like can only indicate values lower than 100, which is the value for the color rendition of the reference light. For instance, Ra and Ri (R9 to R12) being 90 in a case where the colors appear dull with respect to the reference light and thus are not preferable, and Ra and Ri (R9 to R12) being 90 in a case where the colors are more vivid than those pertaining to the reference light and thus are more preferable both exist. The present invention is able to distinguish between these cases. The present invention solves special phenomena of the color rendition that pertain to the light color of the special ultra-high color temperature and that cannot be grasped by simply increasing Ra and Ri.

Based on the above, it has been found out that in the ultra-high color temperature region, enhancing the emission in the range of 480 [nm] to 520 [nm] between the main emission peaks of the blue emitting rare-earth phosphor and the green emitting rare-earth phosphor, which are high in luminosity not only improves the appearance of the blue green color, to which the spectrum is added, but also improves the appearance of red which is the supplementary color thereof.

The present invention differs from a concept of simply adding red emission, which is low in luminosity, to improve the appearance of red, which one skilled in the art would easily arrive at. The present invention thus can provide a fluorescent lamp and a lighting apparatus able to suppress melatonin secretion efficiently in a natural visual environment without reducing the emission efficiency, thereby achieving effects of adjusting biological rhythms and promoting wakefulness as well as achieving color rendering characteristics.

In order to yield these advantageous effects more significantly, it is preferable that the added emission has narrow-band characteristics so that the energy can be concentrated in a targeted region more efficiently, allowing more preferable implementation. While conventional Ra, R9 and the like are not able to achieve these effects, the present invention has found a method of evaluating these effects easily using the gamut areas Ga and Ga4 and shapes thereof, and is realized by performing quantitative evaluation.

Even in a case where the features of the present invention that extend the color gamuts especially in the red and green directions even when Duv is a positive value and enable Ga, Ga4, and the like to come close to or exceed 100 are explained merely in connection with conventional Ra, Ri and the like, and the following unconventional features still exist: when a conventional structure and the structure of the present invention are compared in detail in a case of varying Duv while keeping the correlated color temperature constant, according to the present invention, unlike the unconventional case, the indexes such as Ra, Ri and the like improve when Duv is a positive value.

This tendency is more significant when the correlated color temperature and the high color temperature are not lower than 8000 [K], especially not lower than 10000 [K] , and it is further significant when the added emission in the range of 480 [nm] to 520 [nm] has a high peak in a narrow band.

Thus the present invention has a special advantageous effect of realizing sufficient color rendering indexes Ra and Ri even when increasing Duv to a positive value in order to improve the emission efficiency in the ultra-high color temperature region and bring Duv closer to that of the skylight, which is natural light.

Unlike a concept of simply adding red emission, which is low in luminosity, to improve the appearance of red, this method adds blue green, which is relatively high in luminosity. Accordingly, the present invention thus can provide a fluorescent lamp and a lighting apparatus able to suppress melatonin secretion efficiently in a natural visual environment without reducing the emission efficiency, thereby achieving effects of adjusting biological rhythms and promoting wakefulness as well as achieving color rendering characteristics.

In order to attain this effect more significantly, it is preferable that the added emission has a narrow-band half width of 50 [nm] or smaller defined as a narrow-band emission. This is because the energy can be concentrated in a specific region more efficiently.

Next, the correlated color temperature of the present invention is described. Skylight from a blue sky is what one would easily assume as a natural ultra-high color temperature light which human beings encounter in everyday life. The correlated color temperature of direct sunlight components is known to be around 5500 [K], and the correlated color temperature of the skylight from a clear sky is higher.

Conventionally, northern sky daylight is often considered as a representative high color temperature skylight. In this case, the correlated color temperature of the skylight is highest in the sky located opposite and away from the sun (northern sky in the northern hemisphere), partially exceeding 20000 [K] in some cases.

However, the skylight that reaches shaded places, such as a shade of a tree, where the direct sunlight components are blocked is rarely affected by the partial high color temperature and is an integral of skylight from a wide range of the entire sky, which approximately ranges from 11000 [K] to 13000 [K].

Consequently, high color temperature light naturally available in everyday life, such as under a tree shade on a clear day, takes on the above-mentioned value, and is presumed to correspond to a preferable range of correlated color temperature of an ultra-high color temperature light source which is easily acceptable in actual life.

In the present invention, the direct sunlight components and skylight components are considered separately, and a natural high correlated color temperature is chosen. As a result, extraction and setting of a fresh and natural light color such as light from a blue sky becomes possible. Additionally, it is advantageous to separately consider direct sunlight components which have a high directivity and skylight components which have a high diffusivity, in terms of illumination design.

It is easy for a fluorescent lamp with a relatively large emission area to reproduce diffusion light, and accordingly, is able to constitute a surface light source such as a luminous ceiling and realize diffusion illumination of natural skylight Here, an illumination design for blending light from a light source which reproduces direct sunlight having a high directivity and a correlated color temperature of 5500 [K], and light from an ultra-high color temperature fluorescent lamp of the present invention with high diffusivity enables an individual reproduction for direct sunlight high in directivity and low in correlated color temperature and for skylight high in diffusivity and high in correlated color temperature. This enables more faithful reproduction of the skylight.

The following can be stated based on further detailed analysis of daylight, skylight, and the like.

The following non-patent document recites measurement results of the skylight from the entire sky except sunlight: Seishi Sekine, Spectral Distributions of Clear Sky and Their Chromaticities, Journal of the Illuminating Engineering Institute of Japan, Vol. 73, No.2, P.3, 1989. An integral of light from the entire sky except from direct sunlight is approximately 8500 [K] under ordinary fine-weather conditions, and approximately 20000 [K] in clear-sky conditions with extremely clear air.

Setting the correlated color temperature to 8500 [K] or higher is appropriate for simulating skylight from a blue sky in a fine weather in general, and setting the correlated color temperature to 20000 [K] or lower is appropriate for simulating the upper limit of the skylight from an actual clear sky. When the correlated color temperature is high, it is likely that the chromaticity of the measured skylight projects farther in the green direction with respect to the Planckian Locus and Duv is a positive value.

Here, the inverse of a correlated color temperature multiplied by one million is called "mired (micro reciprocal degree)", and an interval of two mired values corresponds to a subjective difference between appearances of two light colors. Approximately, 8500 [K] is 118 [mired], and 20000 [K] is 50 [mired]. The integral of light from the entire sky under fine-weather conditions except for the direct sunlight is presumed to fall in this range. The intermediate correlated color temperature of the integral of the light from the entire sky under clear-sky conditions is calculated to represent an intermediary value thereof; The result is approximately 84 [mired] ≈ 12000 [K]. Because the light colors having the difference of 10 [mired] are visually perceived as substantially the same light color, and accordingly, when a practical light source is used to simulate this, 12000 [K] + 10 [mired] is approximately 10700 [K], while 12000 [K] - 10 [mired] is approximately 13600 [K], and thus, an approximate range of 11000 [K] to 13000 [K] is considered to correspond to a middle value.

Additionally, because the spectral distributions of the skylight is basically under strong influence by the spectral distributions of the sunlight, the CIE reconstituted daylight is often used as a practical approximation.

According to the following non-patent document: Okada Kiyoshi, Report of the Investigative Committee for Daylight Standard Establishment, Journal of the Illuminating Engineering Institute of Japan, Viol.54, No.3, P.15 (1970), it is disclosed that at a high-color temperature above 8500 [K], the measured skylight chromaticity is likely to take on Duv equal to or higher than that of the CIE reconstituted daylight.

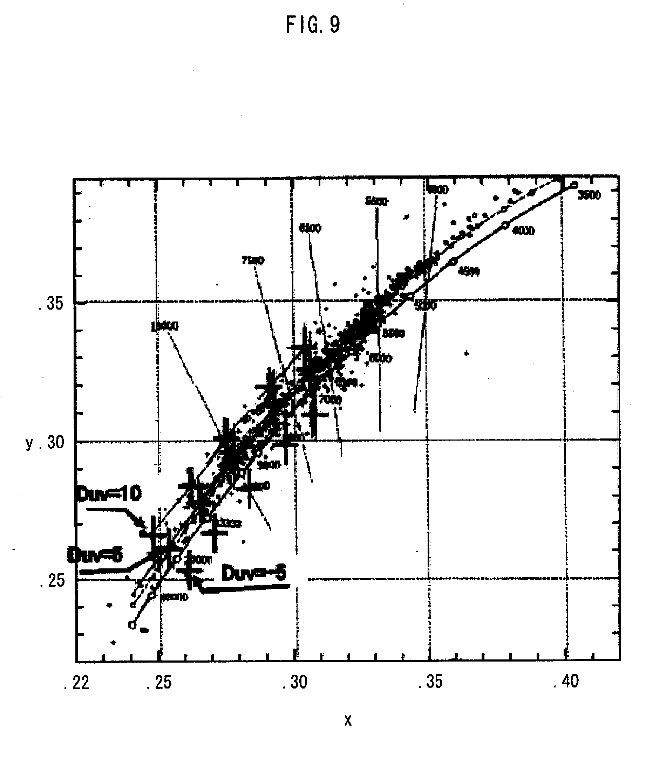

FIG. 9 is a diagram in which the correlations between Duv and the chromaticity coordinates are added to the diagram from a non-patent document "New Edition Color Science Handbook", The Color Science Association of Japan, Fifth ed., p. 67, 1985.

Looking at the distributions of the chromaticity coordinates of natural daylight based on which CIE defined the CIE daylight, approximately 90% of the measured data is included in a range of Duv up to about 10, and Duv of approximately 5 is a middle value close to the value corresponding to the CIE reconstituted daylight.

Here, looking at only the distributions when Duv is a positive value, almost all the measured data is included in a range of Duv up to about 10, and when Duv is approximately 5, a majority of the measured data is included. Because the light color is perceived as bluish green when Duv is excessively high, 7.5 is the practical upper limit of Duv in the viewpoint of discomfort of the luminance color.

As to low Duv, approximately 90% of the measured data is included in a range of Duv down to about 0, and almost all the measured data is included in a range down to about -5, with the exception of special cases.

Here, looking at only the distributions when Duv is a negative value, almost all the measured data is included in a range of Duv down to about -2.5. Based on these, when the light source is applied in a practical environment where windows exist as light sources, the highest preferable value is 10 to 5, and the lowest preferable value is -5 to -2.5, further to 0.

In addition, Duv of the measured daylight often exceeds the CIE daylight locus in the ultra-high color temperature region, and the Planckian Locus where Duv is 0 has a lower value of Duv than natural skylight. Accordingly, it is preferable that the light color of the present invention has Duv of 0 or greater, more preferably Duv greater than 0 in the viewpoint of color match with the skylight.

A conventional lamp based on a narrow-band light source corresponding to a three-wavelength fluorescent lamp is often designed to have Duv lower than 0 and equal to or higher than -2.5 in order to increase Ra. According to the present invention, the color rendering properties do not deteriorate even when Duv is a positive value, whereby the emission efficiency is maintained high, and accordingly, the preferable appearance of the colors can be maintained while matching the light color with the skylight.

In a case where Duv of an embodiment of the present invention is set to take a negative value, as is the case of a conventional three-wavelength fluorescent lamp, higher color rendering properties with respect to those in a case where the corresponding high correlated color temperature is realized in a fluorescent lamp based on a narrow-band light source corresponding to a conventional three-wavelength light source.

In an embodiment of the present invention, it is meaningful, from the viewpoint of consistency of the light color with that of a conventional three-wavelength fluorescent lamp, to set Duv to have a negative value to have consistency in light color image with other light sources which are set to have a purplish light color. When the present invention is applied in an actual environment where a conventional fluorescent lamp also exists, the preferable lower limit of Duv is around -2.5, or equal to or higher than -1.0 which is a value Duv of the conventional fluorescent lamp often takes on.

On the other hand, in order to clearly distinguish the color in the Duv direction from the conventional three-wavelength fluorescent lamp, it is preferable to set Duv to a positive value, thereby excluding the range of Duv of the conventional fluorescent lamp.

For example, when a positive value is to be set for Duv which is greater than 0, specifically, in a case where the digit after the decimal point is rounded off, Duv can be set to 0.5 or higher, and in a case where the digit after the decimal point is truncated, Duv can be set to 1 or higher.

Visual evaluation experiments were performed in an ultra-high color temperature region, and the results thereof indicated that when these light sources were placed side by side and compared with each other, the difference between the two light colors was unable to be perceived when the difference between the light colors in Duv was lower than 0.5. When the difference in Duv is 1 or greater, most people perceived the difference in light color.

On the other hand, one factor that restricts the correlated color temperature in an actual visual environment is consistency in light color between a self-light emitting display device and illuminating light. Here, one practical issue is that at an office or home, the white point of a display monitor is often set to around 11000 [K], and if an ultra-high color temperature light source having a color temperature higher than that is used, the white color of the monitor appears yellowish, becoming unsuitable for monitor work, video watching and the like.

In general, the white point of a display monitor is set at a higher value than the correlated color temperature of the surrounding illumination environment. Hence, the above-mentioned phenomenon does not occur from the light color of a conventional light source for general illumination but occurs from the use of the ultra-high color temperature for illumination.

Empirically, the yellowish appearance of the display monitor was not perceived below the correlated color temperature of about 13000 [K]. From the theoretical viewpoint, this result fits the conventional knowledge that a difference of 10 [mired] (micro reciprocal degree) or smaller in light color is unlikely to be perceived. This is because when the difference with the white point of the monitor is 10 [mired], the color temperature of the illumination light falls in a range of 10000 [K], obtained by 11000 [K] + 10 [mired], to 12500 [K], obtained by 11000 [K] - 10 [mired] (13000 [K] by rounding off the fraction).

In addition, it is possible to set an acceptable range of the light color of the present invention by performing, with mired intervals, extrapolation of higher light color ranges with respect to daylight color and F6500 that each are the highest-color temperature chromaticity specification of the light color classification for JIS and IEC fluorescent lamps, respectively. In this case, the range would be 8000 [K] to 17000 [K] for JIS, and F8774 and F12655 for IEC.

While it is possible to simply set a range of 8774 [K] to 12655 [K] using the values obtained from the IEC specification, it is also possible to set an acceptable chromaticity range for F8774 and F12655 individually, or to set a range of the correlated color temperature of the present invention using the maximum correlated color temperature and the minimum correlated color temperature of these values.

The inventors of the present invention obtained psychological estimation from subjective evaluation experiments that an approximate range of 10000 [K] to 14000 [K], more preferably a range of 11000 [K] to 13000 [K] is recognized as a light color group distinguished clearly as having a higher level of color temperature with respect to the daylight color and F6500.

In addition, the following non-patent document suggests other effects by ultra-high color temperature illumination: Takashi Kanai, Effects of High Color Temperature Illumination on Psychological and Physiological Function in Working, Master's Thesis, Chiba University Graduate School of Science and Technology, 2000.

The results of brain wave measurements for different correlated color temperatures of illumination light indicate that the bandwidth rate of Alpha waves which are spontaneous brain waves decreases between the correlated color temperature of 3000 [K] and 9000 [K], and starts increasing at 11000 [K] or higher. Meanwhile, the initial component value of CNV which is an event-related potential is high at the correlated color temperature of 9000 [K] and begins to drop at 11000 [K].

In general, the Alpha wave indexes are considered to be associated with a relaxed state of a human being, and show a generally recognized tendency that human beings are in a relaxed state under low color temperature light and the state weakens at high color temperature light. However, the relaxed state starts intensifying at around 10000 [K] between 9000 [K] and 11000 [K].

On the other hand, in general, the CNV indexes are considered to be associated with attention concentration and tension, and fit a generally recognized tendency that attention concentration and tension increase under high color temperature light rather than under low color temperature light. However, the attention concentration and tension begin to ease around 10000 [K] between 9000 [K] and 11000 [K].

These brain wave indexes suggest that the tension of the brain activities that intensify as the color temperature rises being to ease at around 10000 [K] again. The brain wave indexes showed a clearer change at 11000 [K] after this inflection point.

According to psychological evaluations conducted at the same time showed a tendency that "sleepiness" and "focus on work" increase even after the color temperature exceeds 1000 [K], indicating different results from the brain wave indexes. In other words, this state is physiologically relaxed according to the brain index while concentration is heightened psychologically, and thus, it is expected at an ultra-high color temperature of over 10000 [K] that a light color which heightens concentration without excessive tension can be obtained.

In general, a blue light color with high color purity is said to have a relaxing and calming effect on human beings. It is supposed that such an effect statrt appearing at an ultra-high color temperature exceeding 10000 [K] at which blue emitting components are enhanced.

According to the psychological experiments conducted by the inventors of the present invention, psychological exhilaration was felt at a bluish white light color exceeding 10000 [K]. This indicates that according to the present invention which is able to realize higher color rendering properties than those in a case where the corresponding high correlated color temperature is realized in a fluorescent lamp based on a narrow-band light source corresponding to a general three-wavelength fluorescent lamp, reducing discomfort in the visual environment that inhibits such an effect due to the ultra-high color temperature can realize a more comfortable environment.

As is apparent from the above, various correlated color temperatures and lower and upper limits of Duv can be combined in various ways to combine the above-mentioned physiological effects and color rendering effects preferably and optimally within the scope of the present invention in order to realize a natural light color while improving the color rendering properties and the melanopsin suppression effect.

For example, it is an option to set the lower limit of the correlated color temperature in view of the psychological and physiological effects and set the upper limit of the correlated color temperature in view of the color rendering effects. One skilled in the art can change these combinations in accordance with the purpose.

**[Brief Description of Drawings]**

FIG. 1 is a graph showing an action function of spectral absorption of melanopsin, action functions of melatonin suppression, and a standard spectral luminous efficiency curve..

FIG. 2 is a graph showing chromaticity of each of color chips R1 to R8 on U*V* chromaticity coordinates:

FIG. 3 is a graph showing relationships between a correlated color temperature and action functions A($\lambda$) of melatonin suppression using a first action function G(C($\lambda$)) as a representative.

FIG. 4 is a graph showing relationships between a correlated color temperature and the action functions A($\lambda$) of melatonin suppression with a second action function B (Brainard) as a representative.

FIG. 5 is a graph showing correlation between the first action function G and the second action function B.

FIG. 6 is a table showing results in a case where a blue emitting phosphor BAM, a green emitting phosphor LAP, and a red emitting phosphor YOX are used as phosphors for a conventional three-band fluorescent lamp.

FIG. 7 is a table showing results in a case where a blue emitting phosphor BAM:Mn (also emits blue green), the green emitting phosphor LAP, and the red emitting phosphor YOX are used in an embodiment of the present invention.

FIGs. 8A and 8B are graphs showing an eight-color gamut defined by color chips R1 to R8 and a four-color gamut defined by color chips R9 to R12 on U*V* chromaticity coordinates, respectively, for a base light, a comparative case having an ordinary structure, and an example, where the correlated color temperature is 12000 [K] and Duv = 0.

FIG. 9 is a graph showing Duv on color chromaticity coordinates.

FIGs. 10a and 10b are a partially cutaway perspective view and a sectional view each showing a fluorescent lamp 100 of the embodiment of the present invention.

FIG. 11 is a graph showing spectral distribution of the fluorescent lamp 100 of the embodiment of the present invention.

FIGs. 12A and 12B are graphs showing an eight-color gamut defined by the color chips R1 to R8 and a four-color gamut defined by the color chips R9 to R12 on the U*V* chromaticity coordinates, respectively.

FIG. 13 is a graph showing spectral distributions of BAM, LAP, and YOX used for a conventional narrow-band fluorescent lamp.

FIGs. 14A and 14B are graphs showing an eight-color gamut defined by the color chips R1 to R8 and a four-color gamut defined by the color chips R9 to R12 on the U*V* chromaticity coordinates, respectively, of a fluorescent lamp with a conventional structure.

FIG. 15 is a graph showing spectral distributions of a conventional narrow-band fluorescent lamp (BAM) and a narrow-band fluorescent lamp of an example (BAM:Mn) for comparison, where the correlated color temperature is 12000 [K] and Duv = 0.

FIG. 16 is a graph showing changes in a peak ratio of YOX to LAP for different Duv with respect to each correlated color temperature.

FIG. 17 is a table showing values of color rendering indexes when emission peak intensity 2/emission peak intensity 1 is approximately 0.5.

FIG. 18 is a table showing values of the color rendering indexes when emission peak intensity 2/emission peak intensity 1 is approximately 0.7.

FIG. 19 is a table showing values of the color rendering indexes when emission peak intensity 2/emission peak intensity 1 is approximately 1.0.

FIG. 20 is a table showing values of the color rendering indexes when emission peak intensity 2/emission peak intensity 1 is approximately 1.3.

FIGs. 21A to 21C are graphs showing a spectral distribution, color gamuts defined by the color chips R1 to R8, and color gamuts defined by the color chips R9 to R12, respectively, where the correlated color temperature is 7100 [K].

FIGs. 22A to 22C are graphs showing a spectral distribution, color gamuts defined by the color chips R1 to R8, and color gamuts defined by the color chips R9 to R12, respectively, where the correlated color temperature is 11000 [K].

FIGs. 23A to 23C are graphs showing spectral distributions, color gamuts defined by the color chips R1 to R8, and color gamuts defined by the color chips R9 to R12, respectively, where the correlated color temperature is 20000 [K].

FIGs. 24A to 24C are graphs showing a spectral distribution, color gamuts defined by the color chips R1 to R8, and color gamuts defined by the color chips R9 to R12, respectively, of a conventional narrow-band fluorescent lamp, where the correlated color temperature is 7100 [K].

FIGs. 25A to 25C are graphs showing a spectral distribution, color gamuts defined by the color chips R1 to R8, and color gamuts defined by the color chips R9 to R12, respectively, of a conventional narrow-band fluorescent lamp, where the correlated color temperature is 11000 [K].

FIGs. 26A to 26C are graphs showing a spectral distribution, color gamuts defined by the color chips R1 to R8, and color gamuts defined by the color chips R9 to R12, respectively, of a conventional narrow-band fluorescent lamp, where the correlated color temperature is 20000 [K].

FIGs. 27A to 27C are graphs showing a spectral distribution, color gamuts defined by the color chips R1 to R8, and color gamuts defined by the color chips R9 to R12, respectively, where (emission peak intensity 2/emission peak intensity 1) is 0.5 and the correlated color temperature is 7100 [K].

FIGs. 28A to 28C are graphs showing a spectral distribution, color gamuts defined by the color chips R1 to R8, and color gamuts defined by the color chips R9 to R12, respectively, where (emission peak intensity 2/emission peak intensity 1) is 0.5 and the correlated color temperature is 11000 [K].

FIGs. 29A to 29C are graphs showing a spectral distribution, color gamuts defined by the color chips R1 to R8, and color gamuts defined by the color chips R9 to R12, respectively, where (emission peak intensity 2/emission peak intensity 1) is 0.5 and the correlated color temperature is 20000 [K].

FIGs. 30A to 30C are graphs showing a spectral distribution, color gamuts defined by the color chips R1 to R8, and color gamuts defined by the color chips R9 to R12, respectively, where (emission peak intensity 2/emission peak intensity 1) is 0.75 and the correlated color temperature is 7100 [K].

FIGs. 31A to 31C are graphs showing a spectral distribution, color gamuts defined by the color chips R1 to R8, and color gamuts defined by the color chips R9 to R12, respectively, where (emission peak intensity 2/emission peak intensity 1) is 0.75 and the correlated color temperature is 11000 [K].

FIGs. 32A to 32C are graphs showing a spectral distribution, color gamuts defined by the color chips R1 to R8, and color gamuts defined by the color chips R9 to R12, respectively, where (emission peak intensity 2/emission peak intensity 1) is 0.75 and the correlated color temperature is 20000 [K].

FIGs. 33A to 33C are graphs showing a spectral distribution, color gamuts defined by the color chips R1 to R8, and color gamuts defined by the color chips R9 to R12, respectively, where (emission peak intensity 2/emission peak intensity 1) is 1.0 and the correlated color temperature is 7100 [K].

FIGs. 34A to 34C are graphs showing a spectral distribution, color gamuts defined by the color chips R1 to R8, and color gamuts defined by the color chips R9 to R12, respectively, where (emission peak intensity 2/emission peak intensity 1) is 1.0 and the correlated color temperature is 11000 [K].

FIGs. 35A to 35C are graphs showing a spectral distribution, color gamuts defined by the color chips R1 to R8, and color gamuts defined by the color chips R9 to R12, respectively, where (emission peak intensity 2/emission peak intensity 1) is 1.0 and the correlated color temperature is 20000 [K].

FIGs. 36A to 36C are graphs showing a spectral distribution, color gamuts defined by the color chips R1 to R8, and color gamuts defined by the color chips R9 to R12, respectively, where the correlated color temperature is 7100 [K].

FIGs. 37A to 37C are graphs showing a spectral distribution, color gamuts defined by the color chips R1 to R8, and color gamuts defined by the color chips R9 to R12, respectively, where the correlated color temperature is 11000 [K].

FIGs. 38A to 38C are graphs showing spectral distributions, color gamuts defined by the color chips R1 to R8, and color gamuts defined by the color chips R9 to R12, respectively, where the correlated color temperature is 20000 [K].

FIGs. 39A to 39C are graphs showing a spectral distribution, color gamuts defined by the color chips R1 to R8, and color gamuts defined by the color chips R9 to R12, respectively, where the correlated color temperature is 7100 [K].

FIGs. 40A to 40C are graphs showing a spectral distribution, color gamuts defined by the color chips R1 to R8, and color gamuts defined by the color chips R9 to R12, respectively, where the correlated color temperature is 11000 [K].

FIGs. 41A to 41C are graphs showing spectral distributions, color gamuts defined by the color chips R1 to R8, and color gamuts defined by the color chips R9 to R12, respectively, where the correlated color temperature is 20000 [K].

FIGs. 42A to 42C are graphs showing a spectral distribution, color gamuts defined by the color chips R1 to R8, and color gamuts defined by the color chips R9 to R12, respectively, where the correlated color temperature is 7100 [K].

FIGs. 43A to 43C are graphs showing a spectral distribution, color gamuts defined by the color chips R1 to R8, and color gamuts defined by the color chips R9 to R12, respectively, where the correlated color temperature is 11000 [K].

FIGs. 44A to 44C are graphs showing spectral distributions, color gamuts defined by the color chips R1 to R8, and color gamuts defined by the color chips R9 to R12, respectively, where the correlated color temperature is 20000 [K].

FIGs. 45A to 45C are graphs showing a spectral distribution (SCA), color gamuts defined by the color chips R1 to R8, and color gamuts defined by the color chips R9 to R12, respectively, where the correlated color temperature is 11000 [K].

FIGs. 46A to 46C are graphs showing a spectral distribution (SCA broad), color gamuts defined by the color chips R1 to R8, and color gamuts defined by the color chips R9 to R12, respectively, where the correlated color temperature is 11000 [K].

FIGs. 47A and 47B are graphs showing comparison results of color gamuts defined by the color chips R1 to R8, and color gamuts defined by the color chips R9 to R12.

FIGs. 48A to 48C are graphs showing a spectral distribution, color gamuts defined by the color chips R1 to R8, and color gamuts defined by the color chips R9 to R12, respectively.

FIGs. 49A to 49C are graphs showing a spectral distribution, color gamuts defined by the color chips R1 to R8, and color gamuts defined by the color chips R9 to R12, respectively.

FIG. 50 is a graph showsin a spectral distribution of a fluorescent lamp pertaining to a fourth embodiment of the present invention.

FIG. 51 is a table showing values of color rendering indexes when Duv is varied for each correlated color temperature.

FIGs. 52A and 52B are graphs showing comparison results of color gamuts defined by the color chips R1 to R8, and color gamuts defined by the color chips R9 to R12.

FIG. 53 is a graph showing a spectral distribution of a fluorescent lamp pertaining to a fifth embodiment of the present invention.

FIG. 54 is a table showing values of color rendering indexes when Duv is varied for each correlated color temperature.

FIGs. 55A and 55B are graphs showing comparison results of color gamuts defined by the color chips R1 to R8, and color gamuts defined by the color chips R9 to R12.

FIG. 56 is a graph showing a spectral distribution of a fluorescent lamp pertaining to a sixth embodiment of the present invention.

FIG. 57 is a table showing values of color rendering indexes when Duv is varied for each correlated color temperature.

FIGs. 58A and 58B are graphs showing comparison results of color gamuts defined by the color chips R1 to R8, and color gamuts defined by the color chips R9 to R12.

FIG. 59 is a graph showing a spectral distribution of CAT:Mn phosphor of a fluorescent lamp pertaining to a sixth embodiment of the present invention.

FIGs. 60A and 60B are graphs showing comparison results of color gamuts defined by the color chips R1 to R8, and color gamuts defined by the color chips R9 to R12.

FIG. 61 is a perspective view schematically showing a lighting apparatus 1000 of an embodiment of the present invention.

**[Reference Signs List]**

| | |
|---|---|
| 10 | lamp arc tube (bulb) |
| 20 | phosphor layer |
| 24 | lead wire |
| 30 | electrode |
| 32 | filament |
| 34 | lead wire |
| 36 | stem |
| 40 | base |
| 42 | base pin |
| 45 | mercury |
| 46 | ultraviolet ray |
| 47 | electron |
| 48 | visible light |
| 49 | rare gas |
| 50 | melatonin secretion suppressing unit |
| 100 | fluorescent lamp |
| 1000 | lighting apparatus |

**[Description of Embodiments]**

The following describes embodiments of the present invention with reference to the drawings. In the drawings described below, components having substantially the same function are indicated using the same reference sign. It should be noted that the present invention is not limited the embodiments described below.

**<First Embodiment>**

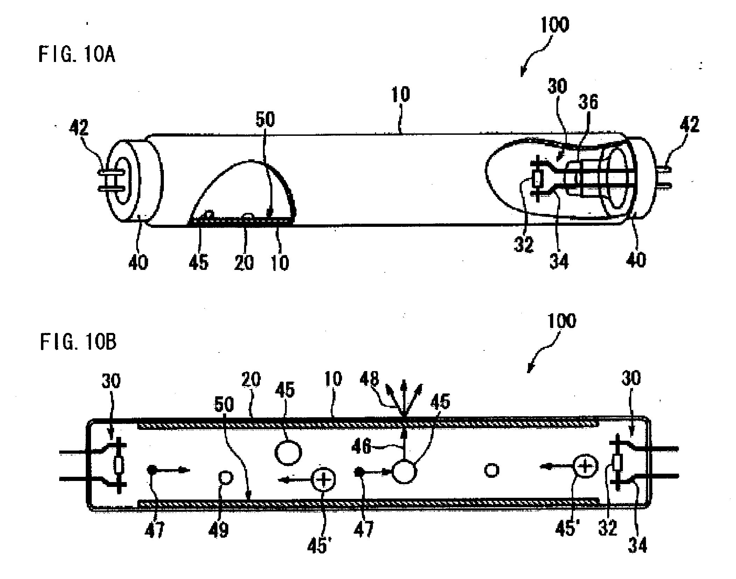

FIG. 10A schematically shows a fluorescent lamp 100 of an embodiment pertaining to the present invention.

FIG. 10B schematically shows a longitudinal sectional view of a structure of the fluorescent lamp 100 of the present embodiment.

The fluorescent lamp 100 of the present embodiment includes a bulb 10 (lamp arc tube) composed of a glass tube and a phosphor layer 20 formed on an inner surface of the bulb 10. In the present embodiment, light emitted from the fluorescent lamp 100 has a high color temperature which is a correlated color temperature above 7100 [K]. In other words, the fluorescent lamp 100 of the present embodiment is a fluorescent lamp that has an ultra-high color temperature exceeding the highest light color 7100 [K] specified by JIS Z9112:1990 (or IEC 60081-1997) for fluorescent lamps for general lighting.

The phosphor layer 20 of the present embodiment includes a red emitting rare-earth phosphor, a green emitting rare-earth phosphor, and a blue emitting rare-earth phosphor. In other words, the fluorescent lamp 100 of the present embodiment is a narrow-band light source corresponding to a three-wavelength light source. In the configuration of the present embodiment, the red emitting rare-earth phosphor is a phosphor which contains a rare-earth element as an emission center whose main emission peak is located in a range of 605 [nm] to 625 [nm]; the green emitting rare-earth phosphor is a phosphor which contains a rare-earth element as an emission center whose main emission peak is located in a range of 540 [nm] to 550 [nm]; and the blue emitting rare-earth phosphor is a phosphor which contains a rare-earth element as an emission center whose main emission peak is located in a range of 440 [nm] to 460 [nm].

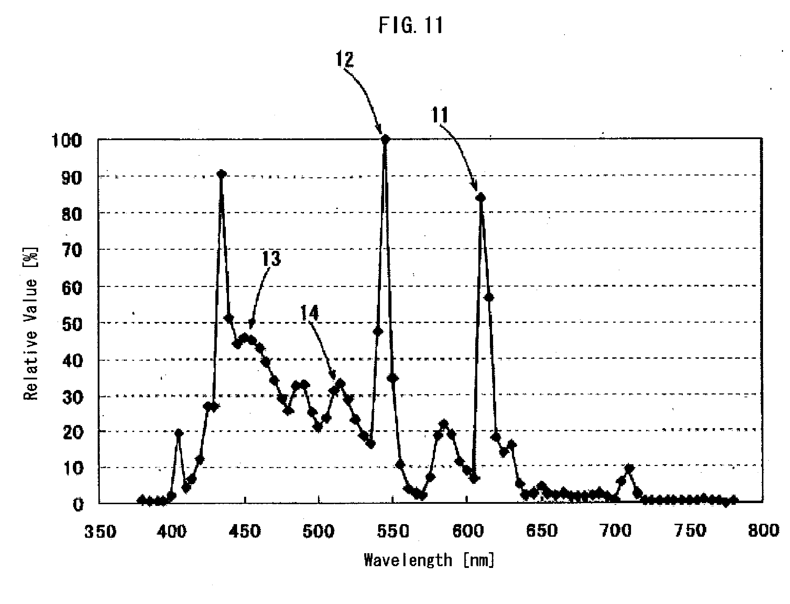

The fluorescent lamp 100 includes a melatonin secretion suppressing unit 50 which suppresses secretion of melatonin by photostimulating melanopsin, and the melatonin secretion suppressing unit 50 is realized by the phosphor layer 20. The suppression action by the melatonin secretion suppressing unit 50 is described later. The phosphor layer 20 further includes a phosphor (a further phosphor) which contains an element as an emission center whose main emission peak is located between the main emission peak of the blue emitting rare-earth phosphor and the main emission peak of the green emitting rare-earth phosphor (i.e. between blue and green). The relationship among the emission peaks of the phosphors constituting the phosphor layer 20 is described later with reference to FIG. 11.

A pair of electrodes 30 is provided in the bulb 10 of the fluorescent lamp 100. The fluorescent lamp 100 shown in FIG. 10 is a hot cathode fluorescent lamp, and the electrodes 30 each have a filament 32. Each filament 32 is mainly made of a tungsten line and is in a shape of a double coil or a triple coil (or a quadruple coil). The surface of each filament 32 is coated with an electron emitting material (emitter). The filaments 32 of the electrodes 30 are respectively supported by lead wires 34 that each are fixed by a stem 36.

The glass tube constituting the bulb 10 is determined based on the electrical characteristics (current, voltage, power consumption, etc.) of the lamp. The bulb 10 is formed in a shape such as a straight shape, an annular shape, or a bent shape to correspond with various use. The bulb 10 shown in FIG. 10 as an example is in a straight shape (straight circular tube). Bases 40 are respectively provided at ends of the bulb 10. Base pins 42 are attached to each base 40, and the base pins 42 are electrically connected with the corresponding lead wires 34 in the bulb 10, respectively.

Mercury 45 for obtaining saturated mercury vapor pressure, and rare gas 49 are enclosed in the bulb 10. The rare gas is, for example, Argon. Alternatively, the rare gas is a mixed gas of argon, and crypton, neon, or the like. The mercury 45 is introduced into the lamp in a state of being enclosed in a glass capsule or introduced in a state of alloy such as zinc mercury or titanium mercury. Mercury amalgam may be enclosed to prevent a decrease in the light emitting efficiency due to an excessive rise of the mercury vapor pressure under a high environmental temperature.

As described above, the phosphor layer 20 is formed on the inner surface of the glass tube constituting the bulb 10 as a result of the phosphors being applied thereto. In may cases, a so-called protective film made of such as aluminum oxide, titanium oxide, silica, yttrium oxide, or the like is applied between the bulb 10 and the phosphor layer 20, so as to prevent a decrease in light transmission due to chemical reaction between the glass tube and the mercury.

The following describes operations (turn-on) of the fluorescent lamp 100. First, electrons 47 are supplied from the electron emitting material on the surface of the filaments 32. The electrons 47 which are accelerated in the electric field in the bulb 10 excite mercury as a result of colliding with mercury vapor 45, and ultraviolet rays 46 are emitted from the excited mercury. The emitted ultraviolet rays 46 excite the phosphors included in the phosphor layer 20 in the bulb 10, generating visible light 48. Mercury ions 45' which have reached the cathodes 30 give their energy to the filaments 32, so that further electrons 47 are supplied from the emitter of the surface of each filament 32. This is how the fluorescent lamp operates as a low-pressure discharge lamp.

Note that while the fluorescent lamp 100 shown in FIG. 10 is a straight hot cathode fluorescent lamp, the fluorescent lamp 100 pertaining to the present embodiment is not limited to this, and may be another hot cathode fluorescent lamp (e.g. annular-shaped). The fluorescent lamp 100 of the present embodiment may be a low-pressure discharge lamp of various forms, and for example, may be a compact fluorescent lamp, a hot cathode fluorescent lamp, a cold cathode fluorescent lamp, an electrodeless fluorescent lamp, or a dielectric barrier discharge lamp.

Next, a further explanation is given on the fluorescent lamp 100 of the present embodiment with reference to FIG. 11. FIG. 11 shows a spectral distribution of the fluorescent lamp 100.

In FIG. 11, 11 is an Eu emission peak of a red emitting rare-earth phosphor YOX which contains a rare-earth element as an emission center whose main emission peak is located in the range of 605 [nm] to 625 [nm]; 12 is a Tb emission peak of a green emitting rare-earth phosphor LAP which contains a rare-earth element as an emission center whose main emission peak is located in the range of 540 [nm] to 550 [nm]; 13 is an Eu emission peak of a blue emitting rare-earth phosphor BAM:Mn which contains a rare-earth element as an emission center whose main emission peak is located in the range of 440 [nm] to 460 [nm]; and 14 is an Mn emission peak of a phosphor BAM:Mn which contains an element whose main peak is located in a range of 480 [nm] to 520 [nm] between the emission peak of the blue emitting rare-earth phosphor and the emission peak of the green emitting rare-earth phosphor.

In the BAM:Mn phosphor of the present example, a peak intensity ratio of Eu whose emission peak is located in the range of 440 [nm] to 460 [nm] to Mn whose emission peak is located in the range of 480 [nm] to 520 [nm] is approximately 1.0 to 0.7 (Eu peak : Mn peak = 1.0 : 0.7).

In the present example, the emission peaks 13 and 14 are obtained by one kind of phosphor, i.e. the BAM:Mn phosphor which has the Eu and Mn emission peaks. Each emission peak appear in a narrow band, thereby efficiently concentrating emission in a targeted luminescence band.

In the present example, various color rendering indexes described below are calculated according to calculation procedures specified by JIS Z8726-1990, and a color gamut Ga4 is defined by four color chips R9 to R12, instead of the color chips used for calculating the gamut area Ga.

Duv is a value calculated according to the calculation procedures specified by JIS Z8725-1999. Duv is obtained by multiplying deviation of u and v values from the Planckian Locus having the corresponding color temperature, by 1000, on the CIE 1960 UCS chromaticity coordinates. Duv takes on a negative value when located below the Planckian Locus. Duv indicates a positional relationship with respect to the Planckian Locus, and the correlated color temperature and Duv determine the chromaticity of the light color of the light source.

Correlated color temperature = 12000 [K], Duv = 0,
Ra = 88, Ga = 113, Ga4 = 105,
R9 = 91, R10 = 89, R11 = 80, R12 = 88, R13 = 84,
R14=82,R15=81

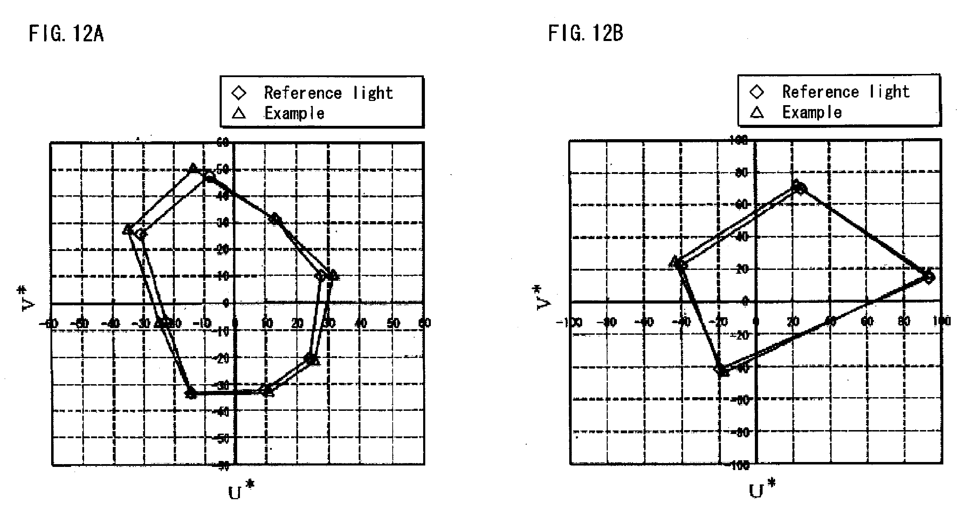

FIGs. 12A and 12B show the 8-color gamut defined by R1 to R8 and the 4-color gamut defined by R9 to R12, respectively, on the U*V* chromaticity coordinates when the respective values as shown above.

The general color rendering index Ra is an average value of respective color deviations between color chips R1 to R8 whose colors are reproduced using reference light with the corresponding correlated color temperature (light for reference), and the color chips R1 to R8 whose colors are reproduced using a light source to be evaluated (example). Even if Ra after averaging indicates the same value, the ways the colors of the color chips deviate differ from one another in detail.

Specifically, when Ra is the same, the more similar in shape a color gamut is to the color gamut pertaining to the color reproduction by the corresponding reference light, the more faithfully the colors are reproduced. In addition, a color gamut larger than the color gamut pertaining to the corresponding reference light has more vivid colors, exhibit a preferable color rendition.

The color rendering indexes R1 to R12 each indicate 100 when they are rendered faithfully to match the color reproduction by the reference light of the corresponding correlated color temperature. Regardless of whether they appear more dull or appear more vivid in a favorable manner, the color rendering indexes R1 to R12 indicate lower values, not exceeding 100.

As is apparent from the above, according to the conventional evaluative values of Ri, a preferable phenomenon such as rendering a high chromaticity color to have appearance of a higher chromaticity only leads to a decrease in the evaluation of the color rendering properties indicated by Ri. Thus, inventions aiming to improve the conventional Ra and Ri differ from the embodiments of the present invention.

Ga and Ga4 are given an evaluative value of 100 when illuminated by the reference light of the corresponding correlated color temperature, and when the Ga and Ga4 become larger than the areas pertaining to the reference light above, Ga and Ga4 indicate a value equal to or higher than 100, working as indexes for whether the colors are rendered more vivid than those rendered with the reference light.

Furthermore, simple evaluation using only conventional Ga yields evaluation dependent on the color chips of medium chroma colors, and accordingly, whether high chroma color chips R9 to R12, which originally present colors preferred to appear vivid, actually appear vivid or not is unclear. As a result, in some cases, Ga is improved but Ga4 is not improved.

This is because the spectral reflectivity of specific spectral ranges of the high chroma chips changes rapidly due to their high chroma, and accordingly, when illuminated by narrow-band illumination light, whether the narrow-band emission spectrum of the illumination light hits the ranges with high spectral reflectivity or not results in a large difference. Consequently, although it is difficult to find a spectral distribution to improve this index, the present invention has found such a spectral distribution at an ultra high color temperature while also improving the melatonin suppression efficiency.

That is to say, when improving the color rendition in the ultra-high color temperature region, conventionally, the red spectrum is reinforced to make up for the insufficient red. The present invention, however, has found out that increasing emission in a blue green region improves appearance of red at an ultra-high color temperature above 7100 [K] or even 10000 [K]; in this case, although R9 falls in terms of numerical value, the evaluative value for the gamut area indicated by Ga4 is maintained high.

Also, in general, Duv is set to a negative value to improve Ra and Ri. However, when Duv is a negative number, luminous efficiency tends to fall due to a decrease of the green emission spectrum which is high in visibility efficiency. On the other hand, the present invention achieves an unconventional effect where Ga and Ga4 are high and the numerical values of Ra and Ri are maintained high even when the luminance efficiency is high with Duv being a positive value. As a result, the melatonin suppression efficiency and the color rendition can be improved at the same time at a correlated color temperature above 7100 [K] or even above 10000 [K] in the ultra-high color temperature region.

In a preferred case of the present invention, distortion of the color reproduction is small, and Ga and Ga4 each attain a value equal to or higher than 100, showing that the decline of the color rendering indexes Ra to R12 result from a preferable vivid color rendition.

Also, in the ultra-high color temperature region, the color gamut pertaining to the reference light is in a shape compressed in the horizontal direction, exhibiting low vividness in the red and green directions. On the other hand, pertaining to the present invention is wider in the horizontal direction with respect to the color gamut pertaining to the reference light, thereby showing an improvement in favorable vividness, with the shape thereof being close to the shape of a color gamut pertaining to the reference light of a correlated color temperature of about 5700 [K] to 7100 [K] in general; and the color chips are evenly distributed on the chromaticity coordinates, showing small distortion. As a result, the present invention realizes natural and favorable appearance of the colors despite the ultra-high color temperature.

As to the melatonin suppression sufficiency of the present invention, the following values are calculated: a value obtained by weighing the spectral power of irradiation spectrum using the melatonin suppression action function $A(\lambda)$ and integrating the resultant value is calculated using a melatonin suppression action function in FIG. 1; and a value obtained by weighing the spectral power of the irradiation spectrum using the CIE standard spectral luminous efficiency $V(\lambda)$ and integrating the resultant value is calculated using the CIE standard spectral luminous efficiency in FIG.. 1.

Based on the spectral distribution shown in FIG. 11 of the present example:

(action function efficiency/visibility efficiency) =
1.25 when (visual pigment-based: the first-type action function G)
1.25 when (direct measurement-based: the second type-action function B)

Furthermore, a case where the following phosphor is included and a case where the following phosphor is not included are compared with each other using fluorescent lamps having the same correlated color temperature and the same Duv: a phosphor containing a rare-earth element whose main peak is located in a range of 480 [nm] to 520 [nm] between the emission peak of the blue emitting rare-earth phosphor and the emission peak of the green emitting rare-earth phosphor. Calculation for realizing and comparing these fluorescent lamps is performed using a difference between not excluding and excluding the emission of the element whose main peak is located in the range of 480 [nm] to 520 [nm], from the lamp spectral distribution, respectively.

In a case where the above-mentioned phosphor is an individual phosphor, the calculation is made by the comparison with a spectral distribution where the corresponding correlated color temperature and Duv are set without the phosphor. In a case where the above-mentioned phosphor is part of a phosphor, the calculation can be made using the phosphor from which the emission center having the main emission peak in the range of 480 [nm] to 520 [nm] is excluded; more simply, the calculation can be made by excluding the spectral distribution of the emission center from the diagram.

Similarly, the difference with the conventional case can be also calculated in another case of the present invention in which the half width from the emission peak of the blue emitting rare-earth phosphor toward a longer wavelength is wide.

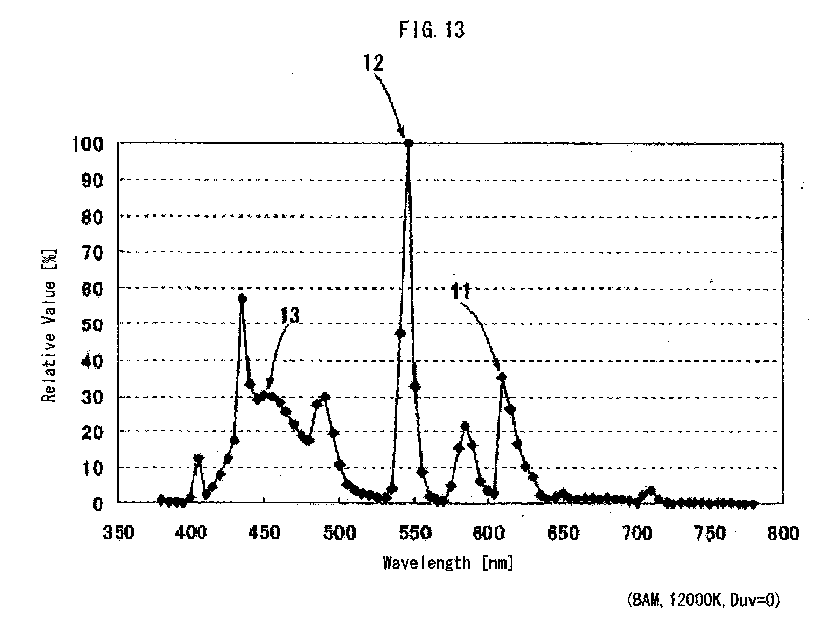

Next, the spectral distributions of BAM, LAP, and YOX used in an conventional narrow-band fluorescent lamp as a target for comparison are shown in FIG. 13.

In FIG. 13, 11 is an Eu emission peak of a red emitting rare-earth phosphor YOX which contains a rare-earth element as an emission center whose main emission peak is located in the range of 605 [nm] to 625 [nm]; 12 is a Tb emission peak of a green emitting rare-earth phosphor LAP which contains a rare-earth element as an emission center whose main emission peak is located in the range of 540 [nm] to 550 [nm]; and 13 is an Eu emission peak of a blue emitting rare-earth phosphor BAM which contains a rare-earth element as an emission center whose main emission peak is located in the range of 440 [nm] to 460 [nm].

Here, the following values are calculated:

Correlated color temperature = 12000 [K], Duv = 0,
Ra = 82, Ga = 96, Ga4 = 91,
R9 = 42, R10 = 46, R11 = 65, R12 = 59, R13 = 93,
R14=71,R15=96.
(action function efficiency/visibility efficiency) =
1.19 when (visual pigment-based: the first-type action function G)
1.19 when (direct measurement-based: the second type-action function B)

Based on the above, realizing and comparing a case where an element whose main peak is located in the range of 480 [nm] to 520 [nm] between the emission peak of the blue emitting rare-earth phosphor and the emission peak of the green emitting rare-earth phosphor is contained and a case where the element is not contained, using a fluorescent lamp having the same correlated color temperature and Duv, the realized fluorescent lamp has the following feature: the ratio of action function efficiency/visibility efficiency and Ra are higher than those in the case where the phosphor having an element whose main peak is located in the range of 480 [nm] to 520 [nm] between the emission peak of the blue emitting rare-earth phosphor and the emission peak of the green emitting rare-earth phosphor is contained. As described above, it is more preferable from the viewpoint of the action function efficiency that the ratio (action function efficiency/visibility efficiency) is 1 or higher.

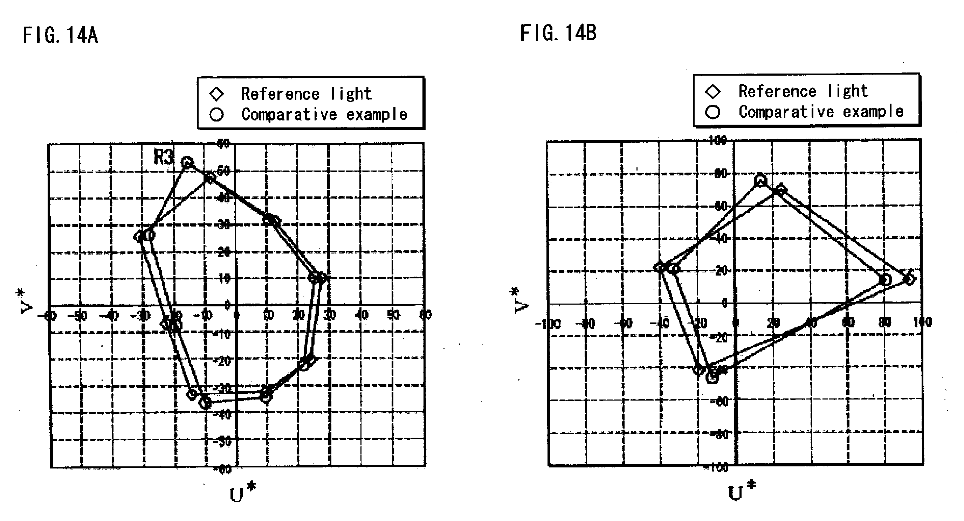

FIGs. 14 show an 8-color gamut defined by R1 to R8 and a 4-color gamut defined by R9 to R12 pertaining to the conventionally structured fluorescent lamp (comparative example) having the spectral distributions in FIG. 13, on the U*V* chromaticity coordinates. These color gamuts show the following features observed when realizing an ultra-high color temperature fluorescent lamp based on a conventional three-wavelength fluorescent lamp: the chroma decreases in the red-green directions, and each color gamut is compressed in the horizontal direction into a vertically long shape, emphasizing the characteristics of the ultra-high color temperature region. Accordingly, it has been found out that an uncomfortable visual environment results from the chroma decrease of the appearance of the red color of the illuminated object in the bluish white color.

Also, when a specific hue stands out, such as when the chroma of the yellow color chip R3 located on the upper left of the chromaticity coordinates of the color gamut defined by R1 to R8 stands out, the balance of the color appearance is significantly distorted in the vertical yellow-blue directions. How the color gamut spreads in this case resembles, in shape, to a color gamut pertaining to reference light of a higher color temperature, intensifying the yellowish and bluish tone of the illuminated object and rendering the reddish and greenish tone dull, as if under an ultra-high color temperature light source having an even higher color temperature.

Yellowish colors shifting toward yellow-greenish colors is a shift evaluated to be less preferable. For example, the skin appears to be yellow green, rendering the skin to appear unhealthy in a high bilirubin state, which is less preferable. The yellowish tone of the melanin pigments also appear yellow greenish, giving unnatural appearance. The present invention is able to improve the above-mentioned tendencies observed for an ultra-high color temperature fluorescent lamp based on a conventional three-wavelength fluorescent lamp, and adjust a balance of color appearances.

Here, attention is focused on the values of the evaluative indexes in FIG. 6 in a case where Duv is varied for each correlated color temperature with use of BAM, LAP, and YOX employed for a conventional narrow-band fluorescent lamp. A table shown in FIG. 6 indicates a typical case pertaining to a conventional narrow-band fluorescent lamp based on a three-wavelength fluorescent lamp, and a table shown in FIG. 7 indicates an example case of the present invention. The intention of improving the action function efficiency in the present invention is to, by appropriately enhancing blue green emission with reference to the values of the evaluative indexes in these figures, achieve higher values for acting power of per unit luminous flux for the correlated color temperatures and Duv than those achieved in the typical case shown in FIG. 6.

Here, the inventors of the present invention has found out that in a typical three-wavelength light source, (action function efficiency/visibility efficiency) can be expressed by a relational expression of Tc (correlated color temperature) and Duv. This is a result of deriving a regression equation according to a simulation result of an action function based on a conventional three-wavelength light source shown in FIG. 6.

That is to say, for an ultra-high color temperature fluorescent lamp based on a conventional three-wavelength light source, the expression of (action function efficiency/visibility efficiency) yields a value exceeding a value expressed by Equation 1 below, when based on the representative second-type action functions Br and Th. This is a result of determining a regression curve for each of the second-type action functions Br and Th for the respective correlated color temperatures and Duv.

[Equation 1]

$$((-0.000000000000000162628 \times Tc^4) + (0.0000000000104466 \times Tc^3) - (0.0000000261804 \times Tc^2) + 0.000322711 \times Tc - 0.379266) - (0.004135401 \times Duv + 0.000094882)$$

Accordingly, increasing emission between the emission peak of the blue emitting rare-earth phosphor and the emission peak of the green emitting rare-earth phosphor, preferably in the range of 480 [nm]-520 [nm], for the respective correlated color temperatures and Duv is another form of disclosure of the expression that the present invention improves (action function efficiency/visibility efficiency). This expressional disclosure is based on the amount of (action function efficiency/ visibility efficiency) for per unit luminous flux.

Furthermore, in a case where the ordinary three-wavelength fluorescent lamp is to be configured such that the value of (action function efficiency/visibility efficiency) thereof does not exceed the calculation result of Equation 1 even if the correlated color temperature Tc (CCT) and Duv, i.e. deviation from the Planckian Locus, change, with variance of the difference between the second-type action functions Br and Th and the value of (action function efficiency/visibility efficiency) obtained from the spectral distributions of the actual fluorescent lamp taken into consideration, a deviation correction value of +0.0392489 can be added in a linear manner to Equation 1.

Furthermore, if an additional correction value equal to or smaller than a regression error of 0.0021 is considered, a regression error correction can be also taken into consideration. Consequently, a higher (action function efficiency/ visibility efficiency) can be estimated for a conventional ordinary three-wavelength fluorescent lamp.

In a case where (action function efficiency/visibility efficiency) exceeds the above-described calculated values, (action function efficiency/visibility efficiency) exceeds that of an ultra-high color temperature fluorescent lamp based on a conventional ordinary three-wavelength light source.

An increase of (action function efficiency/visibility efficiency) according to the present invention can be expressed in an alternative manner in terms of characteristic changes of the emission spectrum. That is to say, in a case where the phosphor layer includes at least a red emitting rare-earth phosphor containing a rare-earth element as an emission center whose emission peak is located in the range of 605 [nm] to 625 [nm], a green emitting rare-earth phosphor containing a rare-earth element as an emission center whose main emission peak is located in the range of 540 [nm] to 550 [nm], and a blue emitting rare-earth phosphor containing a rare-earth element as an emission center whose main emission peak is located in the range of 440 [nm] to 460 [nm], and emission between the emission peak of the green emitting rare-earth phosphor and the emission peak of the blue emitting rare-earth phosphor is enhanced, a spectral intensity ratio of the green emitting rare-earth phosphor and the red emitting rare-earth phosphor each emitting in an even narrower region directly reflects the features thereof. That is, the spectral peak intensity of the red emitting rare-earth phosphor increases as a result of a counter balance of increasing the blue green emission.

In other words, according to the structure of the present invention, when the peak ratio of the green emitting rare-earth phosphor to the red emitting rare-earth phosphor is compared between (a) a case where the emission is enhanced between the main emission peak of the green emitting rare-earth phosphor and the main emission peak of the blue emitting rare-earth phosphor and (b) a case where the emission is not enhanced between the main emission peak of the green emitting rare-earth phosphor and the main emission peak of the blue emitting rare-earth phosphor with the same correlated color temperature and Duv, the peak of the red emitting rare-earth phosphor is likely to become higher in (a).

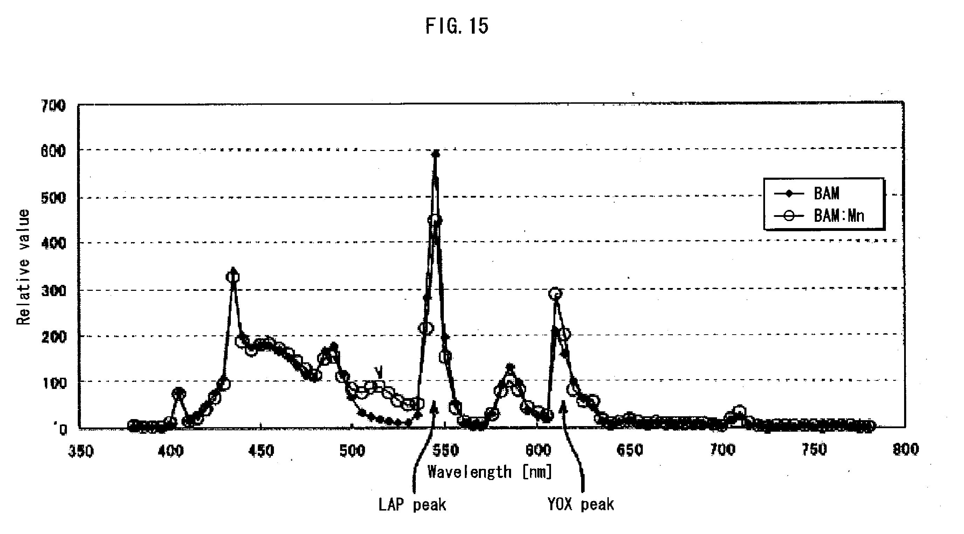

FIG. 15 shows an example of the above-mentioned phenomenon using a case where the correlated color temperature is 12000 [K], and Duv = 0. FIG. 15 compares a case where a narrow-band fluorescent lamp is configured using conventional BAM, LAP, and YOX phosphors and a case where a narrow-band fluorescent lamp is configured using BAM:Mn, LAP, and YOX phosphors of the present invention.

In the present case, the peak intensity ratio of Eu, of the BAM:Mn phosphor, whose main emission peak is located in the range of 440 [nm] to 460 [nm] to Mn, of the BAM:Mn phosphor, whose main emission peak is located in the range of 480 [nm] to 520 [nm] is approximately 1.0 to 0.5 (Eu peak : Mn peak = 1.0 : 0.5). A higher peak of the red emitting rare-earth phosphor results in, among the practical phosphors of the blue emitting rare-earth phosphor (namely BAM), the green emitting rare-earth phosphor (namely LAP), and the red emitting rare-earth phosphor (namely YOX), especially the green emitting rare-earth phosphor (namely Tb emission center) and the red emitting rare-earth phosphor (namely Eu emission center) each emitting light in a narrower band.

The reason for the above is that the emission peak intensity of a fluorescent lamp whose emission band is concentrated on a specific wavelength region reflects more directly the influence of an increase or decrease of spectral components of other wavelength regions as a change in the power of a specific wavelength; and as a result, the peak intensity ratio significantly reflects the influence of enhancing the emission between the main emission peaks of the blue emitting rare-earth phosphor and the green emitting rare-earth phosphor.

FIG. 15 shows that in the present invention, the emission peak intensity of the green emitting rare-earth phosphor decreases, and the emission peak intensity of the red emitting rare-earth phosphorincreases, in a relative manner.

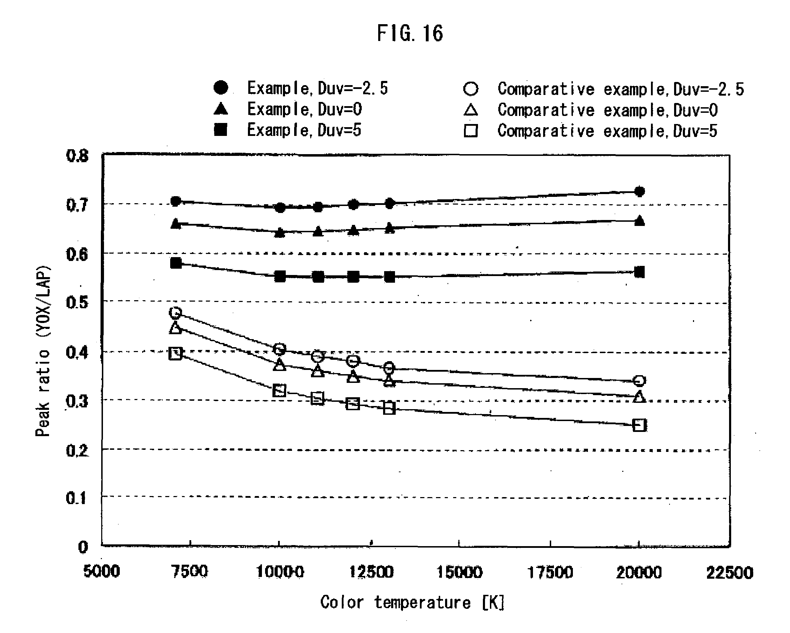

FIG. 16 shows this aspect using a comparison graph of the peak ratios of the green emitting rare-earth phosphor to the red emitting rare-earth phosphor. FIG. 16 shows changes of peak ratios of LAP to YOX for different correlated color temperatures and Duv, comparing conventional BAM and BAM:Mn of the present invention (Eu peak : Mn peak = 1.0 : 0.5).

The vertical axis represents peak ratio obtained by dividing the emission peak intensity of the red emitting rare-earth phosphor by the emission peak intensity of the green emitting rare-earth phosphor; the horizontal axis represents correlated color temperature. As shown in the figure, the enhanced emission between the main emission peaks of the green emitting rare-earth phosphor and the blue emitting rare-earth phosphor is clearly reflected by the peak ratio of the red emitting rare-earth phosphor to the green emitting rare-earth phosphor, exhibiting an increase of the peak intensity of the red emitting rare-earth phosphor.

Described next are features of the emission spectra of a preferred case of the present invention. In a case of not enhancing the emission between the main emission peaks of the blue emitting rare-earth phosphor and the green emitting rare-earth phosphor, that is, when the peak intensity of the green emitting rare-earth phosphor is assumed to be 1.0 according to 5 [nm] spectrometric measurement in an ultra-high color temperature fluorescent lamp based on the conventional three-wavelength fluorescent lamp of FIG. 6, the peak intensity of the red emitting rare-earth phosphor exceeds a value yielded by Equation 2 below which is a function of the correlated color temperature Tc and Duv.

This equation is a result of determining a regression curve based on the peak ratios of the green emitting rare-earth phosphor and the red emitting rare-earth phosphor for the correlated color temperatures and Duv.

[Equation 2]

$$(0.00000000119103 \times Tc2 - (0.0000428646 \times Tc) + 0.682302) + (-0.011546 \times Duv + 0.00962091) - 0.00712759$$

Accordingly, the peak intensity value of the red emitting rare-earth phosphor being higher than the value above for various correlated color temperatures and Duv is another disclosure of the high value of (action function efficiency/ visibility efficiency) of the present invention in terms of the features of the emission spectrum.

The last term of Equation 2, i.e. -0.00712759 is a correction value for the peak intensity of the red emitting rare-earth phosphor of a conventional ordinary three-wavelength fluorescent lamp to not exceed the calculation result of Equation 2, inclusive of deviation.

More strictly, omitting the last term of Equation 2 assures more reliably that the calculation result does not exceed the peak intensity of the red emitting rare-earth phosphor of the conventional ordinary three-wavelength fluorescent lamp. Furthermore, performing a linear subtraction of an additional correction value equal to or less than a regression error 0.0132643 enables an even stricter error correction.

As is apparent from the above, Equation 2 the increase of (action function efficiency/visibility efficiency) in terms of the features of the peak ratios of the green emitting rare-earth phosphor and the red emitting rare-earth phosphor.

Note that in the conventional three-wavelength fluorescent lamp, under the assumption that the peak intensity of the green emitting rare-earth phosphor is 1.0, the peak intensity of the red emitting rare-earth phosphor is as shown in FIG. 16, for correlated color temperatures equal to or higher than 7100 [K] and Duv.

Simply put, as an absolute expression independent of the correlated color temperatures and Duv, it may be preferable that the peak intensity of the red emitting rare-earth phosphor is equal to or higher than 0.5 times the peak intensity of the green emitting rare-earth phosphor, as shown in FIG. 16.

FIG. 16 also shows how the peak intensity of the red emitting rare-earth phosphor changes when the peak intensity ratio of Eu, of the BAM:Mn phosphor, whose main emission peak is located in the range of 440 [nm] to 460 [nm] to Mn, of the BAM:Mn phosphor, whose main emission peak is located in the range of 480 [nm] to 520 [nm] is approximately 1.0 to 0.5 (Eu peak : Mn peak = 1.0 : 0.5). In this case, Equation 3 represents a regression curve determined from the peak ratio values of the green emitting rare-earth phosphor and the red emitting rare-earth phosphor for the correlated color temperatures and Duv.

[Equation 3]

$$(0.000000000363400 \times Tc^2) - (0.00000933917 \times Tc) + 0.695249 + (-0.0186372 \times Duv + 0.0153306) + 0.0393543$$

Accordingly, the emission peak intensity of the red emitting rare-earth phosphor being higher than the value above for the correlated color temperatures and Duv in a more preferable case is another disclosure of an increase of the value (action function efficiency/visibility efficiency) in a preferable case of the present invention.

The last term of Equation 3, i.e., +0.0393543 is a correction value for a regression calculation in which the calculation result, which is the emission peak intensity of the red emitting rare-earth phosphor, exceeds that of the example from which Equation 3 is derived, inclusive of deviation. Omitting this last term leads to determination of the emission peak intensity of the representative red emitting rare-earth phosphor of the example from which Equation 3 is derived. Furthermore, performing a linear subtraction using an additional supplementary value equal to or smaller than the regression error 0.0393543 enables an even stricter error correction.

In general, the emission efficiency of the a red emitting rare-earth phosphor is lower than the emission efficiency of a green emitting rare-earth phosphor. Accordingly, an excessively high emission peak intensity of the red emitting rare-earth phosphor leads to a significant decrease of the emission efficiency of a realized fluorescent lamp. Thus, from the viewpoint of emission efficiency, when the emission peak intensity of the green emitting rare-earth phosphor is assumed to be 1.0, it is preferable that the emission peak intensity of the red emitting rare-earth phosphor is equal to or lower than 1.0, and for an implementation which prioritizes emission efficiency, it is preferable that the emission peak intensity of the red emitting rare-earth phosphor be equal to or higher than that of the conventional case shown in FIG. 16 and equal to or lower than that of the present invention.

From how the peak intensity of the red emitting rare-earth phosphor changes, at various correlated color temperatures and Duv, in FIG. 16, with respect to the peak intensity of the green emitting rare-earth phosphor in a case where the conventional blue emitting rare-earth phosphor, green emitting rare-earth phosphor, and red emitting rare-earth phosphor are used, and also from how the peak intensity of the red emitting rare-earth phosphor of the example of the phosphor containing an element as an emission center whose emission peak is located in the range of 480 [nm] to 520 [nm] (when the Eu peak intensity of BAM:Mn : the Mn peak intentisy = 1 : approximately 0.5), it is apparent that the peak intensity of the red emitting rare-earth phosphor rises. As shown in FIG. 16, the fluorescent peak intensity ratios (red emitting rare-earth phosphor peak intensity/green emitting rare-earth phosphor peak intensity) of the present invention exceed those of the conventional case. FIG. 16 further shows that it is possible for the peak intensity ratios to exceed the fluorescent peak intensity ratios of the present invention shown in FIG. 16 in a more preferable case pertaining to the present invention.

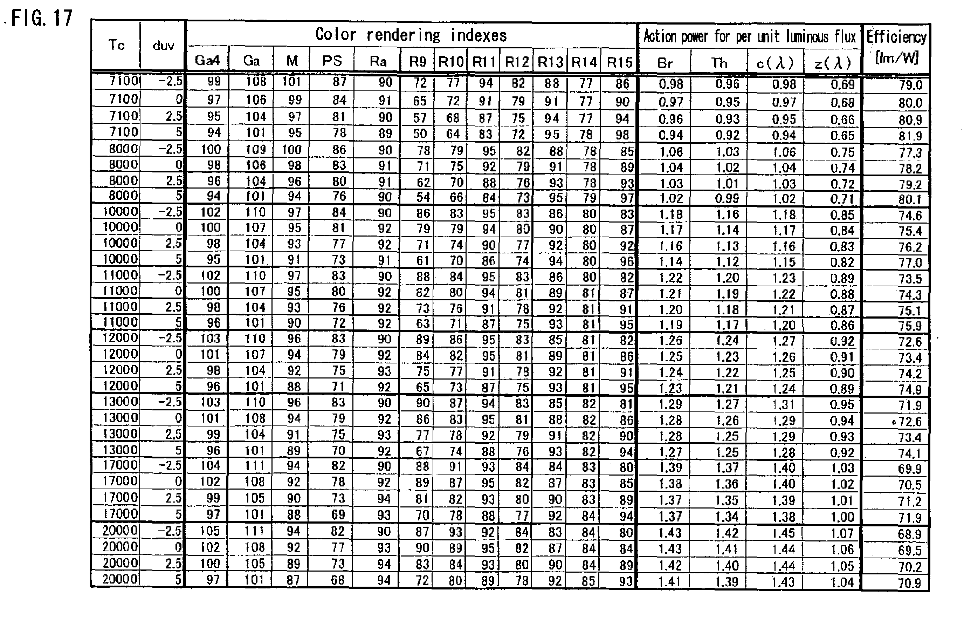

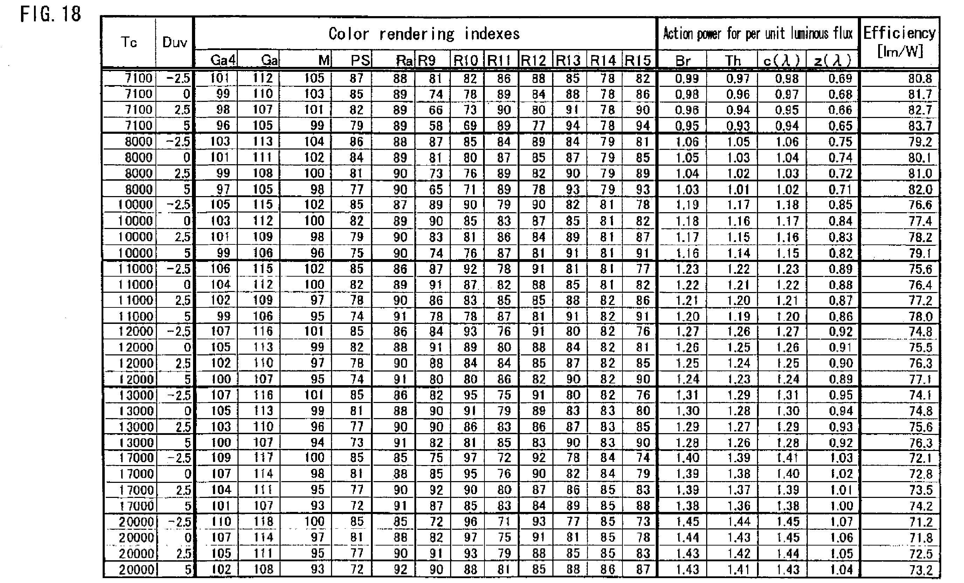

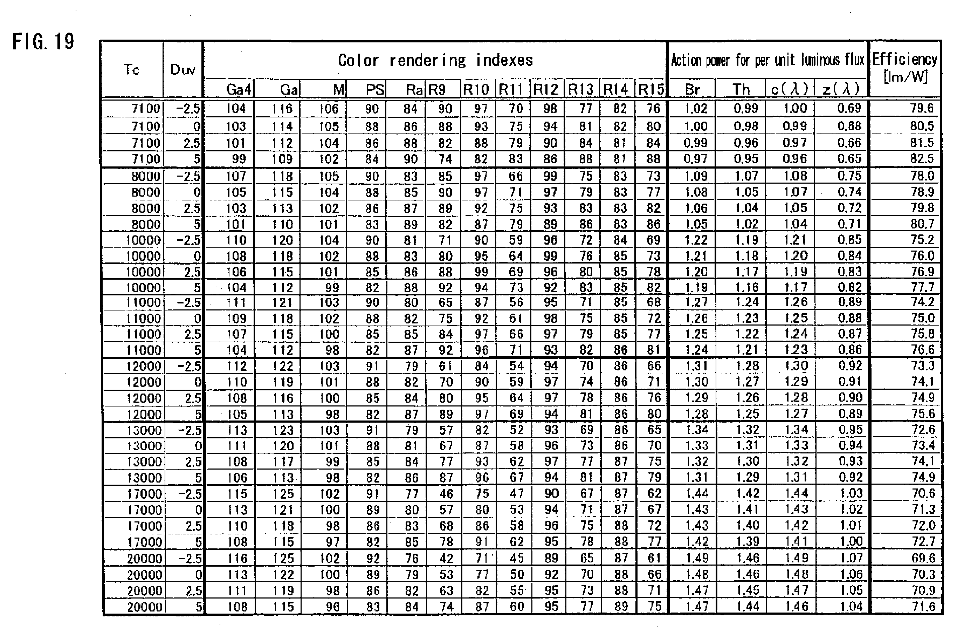

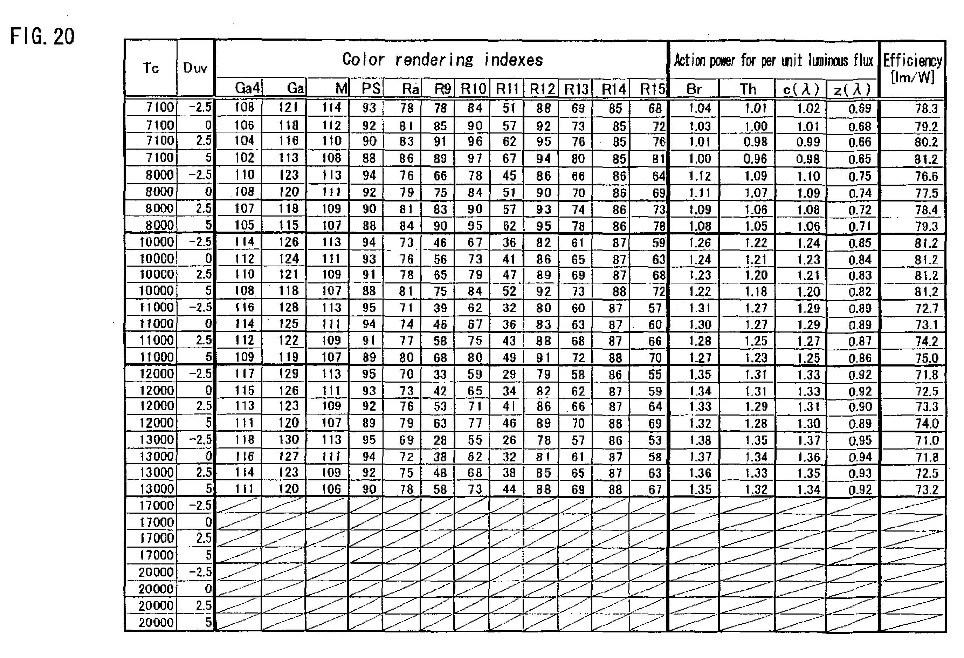

FIGs. 17, 18, 19, and 20 (FIG. 18 being the same as FIG. 7) show values of the rendering indexes when a ratio of the emission intensity of an emission peak 2 whose emission center is located in the range of 480 [nm] to 520 [nm] of the emission spectrum to the emission intensity of an emission peak 1 whose emission center is located in the range of the 440 [nm] to 460 [nm] (emission peak intensity 2/emission peak intensity 1) is approximately 0.5 (FIG. 17: BAM-Mn), approximately 0.7 (FIG. 18:BG-203), approximately 1.0 (FIG. 19:combination of BG-203 and BG-207), and approximately 1.3 (FIG. 20:BG-207), respectively.

As a result of comparing the cases shown in FIGs. 17 to 20 where the emission between the main emission peaks of the blue emitting rare-earth phosphor and the green emitting rare-earth phosphor is enhanced and the case in FIG. 6 where the emission between the main emission peaks of the blue emitting rare-earth phosphor and the green emitting rare-earth phosphor is not enhanced, using fluorescent lamps having the same correlated color temperature and Duv, it is understood that the numerical values of the action functions pertaining to melatonin suppression in the cases shown in FIGs. 17 to 20 are all larger than those in the case shown in FIG. 6.

Also, the values of the special color rendering indexes Ri for evaluating appearance of vivid colors show significant improvements. When (emission peak intensity 2/emission peak intensity 1) is not higher than 1.3, that is, when the peak intensity ratio of Eu and Mn is not higher than 1:1.3, almost all the special color rendering indexes from R9 to R11 achieve a value of 50 or higher. In addition, the special color rendering index R10 for indicating appearance of yellow, which is an opposite color of blue having a large spectral power due to the high color temperature is not lower than 50, exhibiting no large color shift.

The indexes of R9 to R11 improve more when Duv is a positive value, and especially when Duv is a positive value for R9 pertaining to the appearance of red, it is possible to achieve a value no less than 50 for high Duv greater than 5. Accordingly, when intensifying the blue green emission in a narrow band, Duv can be set up to 10 to adjust the present invention to a range of Duv in which Duv of skylight is often included. Additionally, simulations and subjective evaluations have revealed that a numerical decrease of R9 when Ga4 is around 100 includes an effect of favorable color deviation that renders the color more vivid, and is not solely the deterioration in appearance of color.

According to the present invention, simply from the viewpoint of the numerical value of R9, which is a conventional color rendering index, ranges of the correlated color temperatures and Duv can be set such that the value of R9 can be improved to no less that 60, which is considered to be significantly more preferable on a perception level compared to a case where an ultra-high color temperature is realized by a conventional three-wavelength fluorescent lamp, and the value of R9 can be optimally improved to no less than 80, as is the case with Ra. Also, for Ga4 which is closely related to an improvement of R9, 95 or higher, which a conventional three-wavelength fluorescent lamp at an ultra-high color temperature is not likely to realize, can be achieved. Accordingly, unlike conventional cases, the present invention secures high Ga and Ga4, and based on these high Ga and Ga4, exhibits an improvement or a fall of Ra, R9 and the like. In the best embodiment in terms of the color rendition, Ga is no less than 100, and more preferably, Ga4 is also no less than 100.

With Ga and Ga4 taken into consideration, unlike an invention which simply improves Ra, R9 and the like, negative evaluation of color deviation which actually contributes to better color rendition can be appropriately evaluated, thereby realizing more preferable color rendition. When Duv takes on a positive value, these indexes maintain a large value, and Ga, Ga4 and the like are also likely to indicate a value equal to or higher than 100. It should be noted that in order to suppress an excessive greenish color of the light color, it is preferable that Duv be suppressed to 5 or smaller.

It is difficult for a conventional ordinary narrow-band fluorescent lamp to achieve Ga, Ga4 and the like of a value exceeding 100, which pertains to the reference color, when Duv is a positive value. However, according to the present invention, it is possible for Ga to exceed 100, and this tendency is maintained in a high range of Duv as well. Similarly, while it is difficult for the conventional ordinary narrow-band fluorescent lamp to achieve Ga4, defined by the color chips of vivid colors, of a value exceeding 95, and furthermore, 100, the present invention enables Ga4 to achieve a value exceeding 95, or 100, thereby realizing vivid color rendition for a wider range of positive value of Duv.

Here, a large difference between the color rendering properties of the present invention and those of a conventional ordinary narrow-band fluorescent lamp with respect to changes of Duv is the following: according to the conventional ordinary narrow-band fluorescent lamp, when Duv is increased, Ra and Ri monotonously decrease even though the luminance efficiency improves; on the other hand, according to the present invention, Ra and Ri tend to improve with an increase of Duv. This tendency first appears for, among Ri, R9 (red) and R11 (green), and then appears for R10 (yellow) and R12 (blue). Also, R13 to R15 for indicating complexion, appearance of leaves, and the like, are likely to improve when Duv is a positive value.

The higher the rate for enhancing the emission between the main emission peaks of the blue emitting rare-earth phosphor and the green emitting rare-earth phosphor, the more clearly these color rendering characteristics appear, and the higher the correlated color temperature becomes, the more significant these color rendering characteristics become. Furthermore, in a case where the emission between the main emission peaks of the blue emitting rare-earth phosphor and the green emitting rare-earth phosphor is enhanced by a means of adding a phosphor having an emission center whose emission peak appears in the range of 480 [nm] to 520 [nm], these color rendering characteristics exhibit higher values, and in addition, in a case where the half width of the emission center of the added emission peak is located in a narrow-band, preferably at not higher than 50 [nm], these color rendering characteristics appear more significantly.

The following is a further detailed explanation of how the colors are reproduced, using distributions of color gamuts pertaining to the present invention and to the conventional three-wavelength fluorescent lamp, respectively, with reference to FIG. 8. It is apparent from FIG. 8 that the shape of the color gamut pertaining to the present invention produces smaller distortion with respect to the color gamut pertaining to the reference light. While the upper portion and the lower portion of the figure correspond to yellow and blue, respectively, a weak point of the ordinary three-wavelength fluorescent lamp is that yellow is rendered with a color shift to the yellow green region. This shift is expressed in the figure as a projection of an upper part of the color gamut toward the left. In the present invention, however, this shift is significantly improved.

Regarding the color rendition of yellow, a color shift to the yellow red region is more acceptable than a color shift to the yellow green region. Accordingly, this improvement yields an effect of a larger improvement, subjectively, than an improvement of Ri of various color chips for yellow in terms of numerical value. For example, complexion is judged to be preferable when it appears reddish rather than yellow-greenish by the same amount of color shift, since reddish complexion gives an preferable impression of ruddiness. Evaluating the present invention using an index PS used for preferable complexion, instead of evaluating it using values of the indexes R13, R15 and the like which evaluate complexion simply based on the differences from the color rendition by the reference light, shows the significance of the improvement.

As described above, judging from the viewpoints of both the emission efficiency and the color rendition, implementation using the phosphor BAM:Mn is most preferable. In this case, blending the phosphors of the fluorescent lamp by combining a plurality of BAM:Mn and BAM which differ in emission peak intensity ratio of the emission centers Eu and Mn facilitates realization of adjustment of a predetermined emission peak ratio during the fluorescent lamp manufacture. As a result, facilitation of manufacturing and strict color matching can be realized.

When manufacturing, SCA which has narrow-band spectral distributions similar to BAM is sometimes used as a low-cost blue emitting rare-earth phosphor, which contributes to fluorescent lamp manufacturing in terms of cost reduction. In a case where, for example, BAM, LAP, and YOX phosphors are applied in manufacture of an ultra-high color temperature fluorescent lamp, the weight ratio of the applied BAM phosphor increases with respect to the conventional case. For example, when the correlated color temperature is 10000 [K] or higher, the weight ratio of BAM exceeds 25%, and BAM:Mn exceeds 35%.

Furthermore, BAM and BAM:Mn phosphors are often substantially plate-shaped, reflecting the hexagonal crystal structure thereof. Accordingly, when the weight ratio is high, flow of the application liquid easily gets disrupted, causing spotty application in the phosphor application process. Specifically, the shape of the phosphor may be changed to be substantially spherically-shaped in order to reduce spotty application of the phosphor and color unevenness of the emitted color of the lamp arc tube. Additionally, while the phosphors tend to deteriorate at a relatively large rate with time while the lamp is lit, coating the surface of the phosphors enhances the effect of the improvement on color shift as a lamp. The above-mentioned effects are more apparent in an ultra-high color temperature lamp in which the weight ratio of the applied BAM and BAM:Mn phosphors is higher than in an ordinary case.

(Second Embodiment)

The following describes a second embodiment of the present invention. In the second embodiment, a theoretical explanation is given on a relative tendency of the effects caused by the present invention, based on the theoretical spectral distributions in a case where an emission center whose main emission peak is located between the main emission peaks of the blue emitting rare-earth phosphor and the green emitting rare-earth phosphor, preferably in a range of 480 [nm] to 520 [nm].

First, as a theoretical model of a conventional ordinary narrow-band fluorescent lamp, the following structure is theoretically simulated: the structure in which the phosphor layer includes at least a red emitting rare-earth phosphor containing a rare-earth element as an emission center whose emission peak is located in a range of 605 [nm] to 625 [nm], a green emitting rare-earth phosphor containing a rare-earth element as an emission center whose main emission peak is located in a range of 540 [nm] to 550 [nm], and a blue emitting rare-earth phosphor containing a rare-earth element as an emission center whose main emission peak is located in a range of 440 [nm] to 460 [nm].

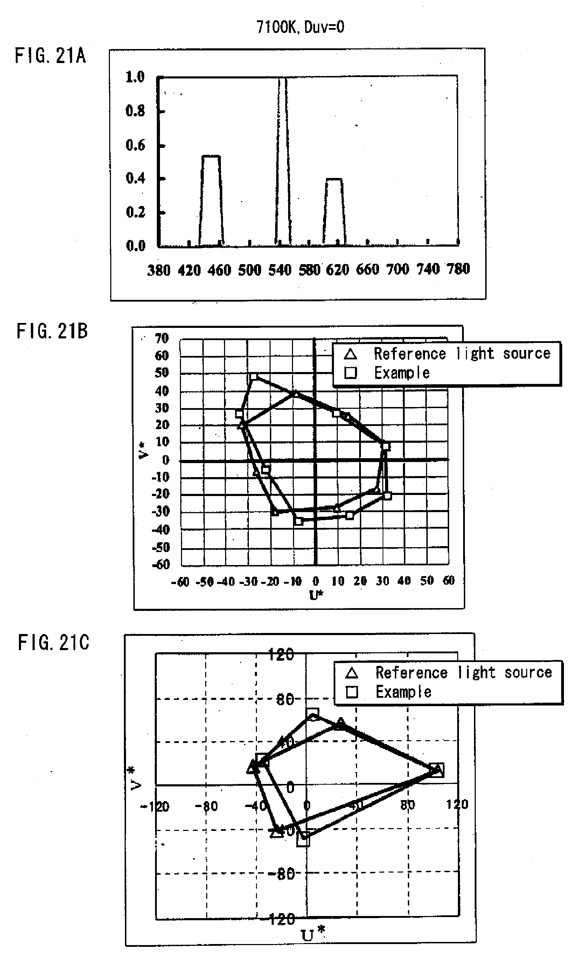

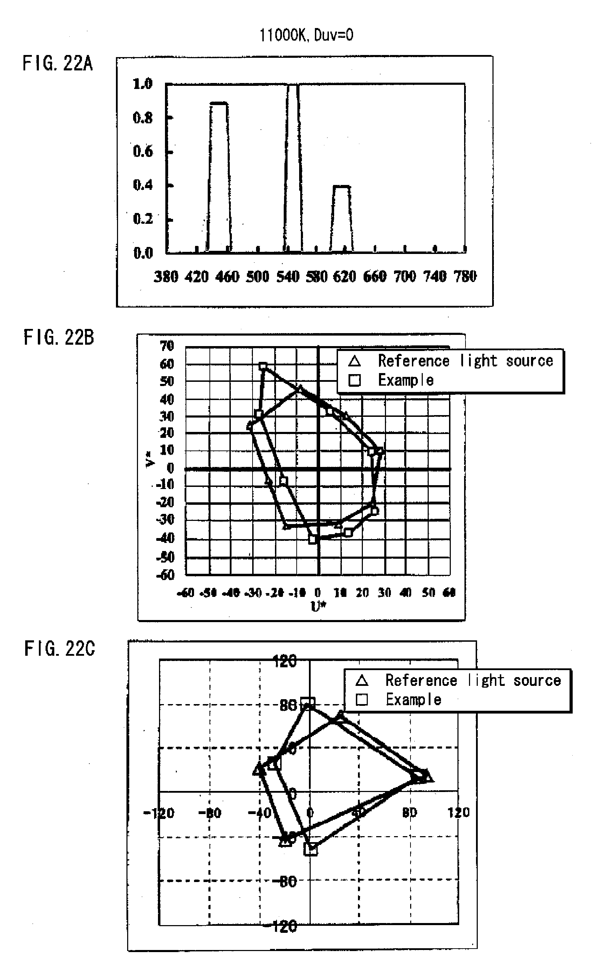

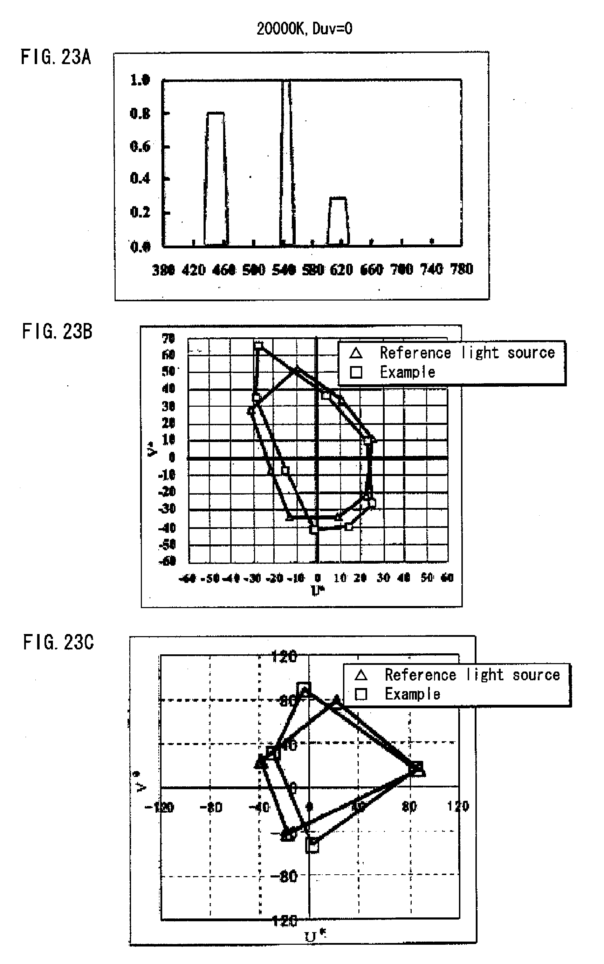

FIGs. 21 to 23 show spectral distributions and color gamuts in cases where ideal phosphors of red and green, and blue which ideally emit only in the above-mentioned emission regions, respectively, are blended. Duv is 0 for all the cases shown in FIGs. 21 to 23. FIGs. 21 show a case with a correlated color temperature of 7100 [K]; FIGs. 22 show a case with a correlated color temperature of 11000 [K]; and FIGs. 23 show a case with a correlated color temperature of 20000 [K].

The theoretical simulation does not include spectrum outside the emission band, such as mercury emission lines and sub-emission of the phosphors. Accordingly, the emission power is concentrated in the narrow band, increasing excitation purity as a result. Consequently, various tendencies appear in a more extreme manner than the examples using actual phosphors.

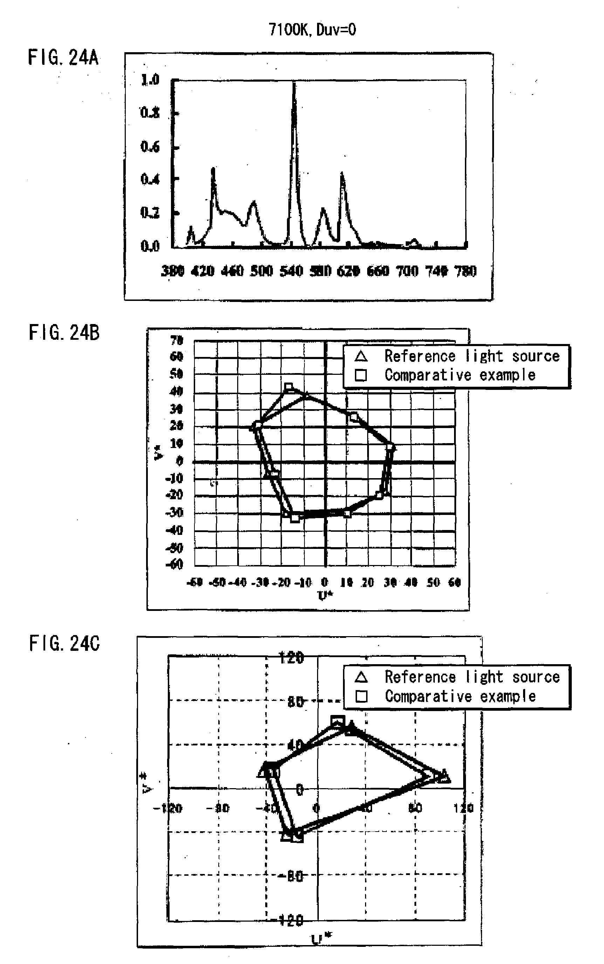

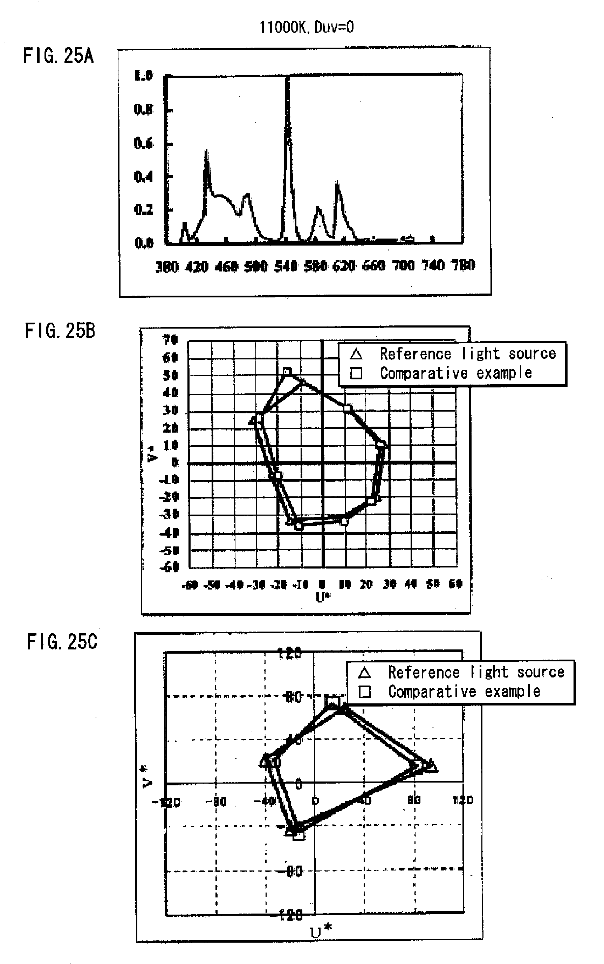

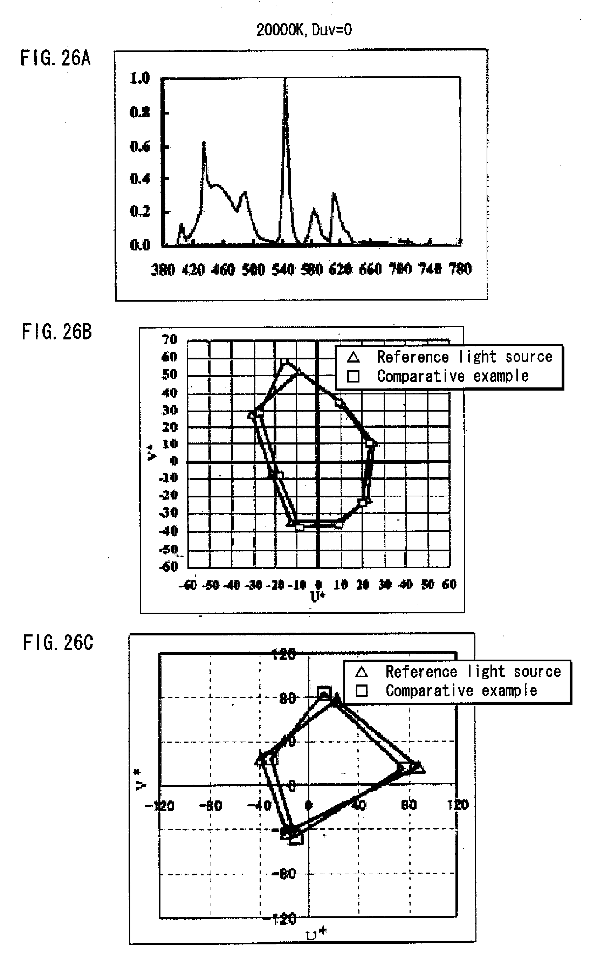

FIGs. 24 to 26 show results of comparative cases in which BAM, LAP, and YOX for use in actual conventional ordinary three-wavelength fluorescent lamps are employed. The correlated color temperatures and Duv for the cases in FIGs. 24 to 26 are the same as those in FIGs. 21 to 23, respectively. In the results of the simulation and the results related to these actual light sources, tendencies such as the following are consistent with each other: prominence of the excitation of the color chip R3; the chroma of R9 being lower than that pertaining to the reference light; the color gamuts being compressed in the red-green directions due to deteriorated chroma of red and green; and the color gamuts being smaller with respect to those pertaining to the reference light.

Similarly, simulations were performed using actual phosphors and theoretical models while varying Duv from -5 to 10 and maintaining the correlated color temperatures. In this case also, the tendencies of the color chips indicated by the simulations and indicated by actual light sources are consistent with each other, that is to say, the color gamuts are compressed in the horizontal direction with an increase of Duv.

Further detailed theoretical simulations were performed on the following case with respect to the above-mentioned results: case where, as is the case with the present invention, the ratio (emission peak intensity 2/emission peak intensity 1) of the emission intensity (emission peak 2) of an emission peak whose emission center is located in the range of the 480 [nm] to 520 [nm] to the emission intensity (emission peak 1) of an emission peak whose emission center is located in the range of 440 [nm] to 460 [nm] of the emission spectrum is in a range of 0.5 to 1.0.

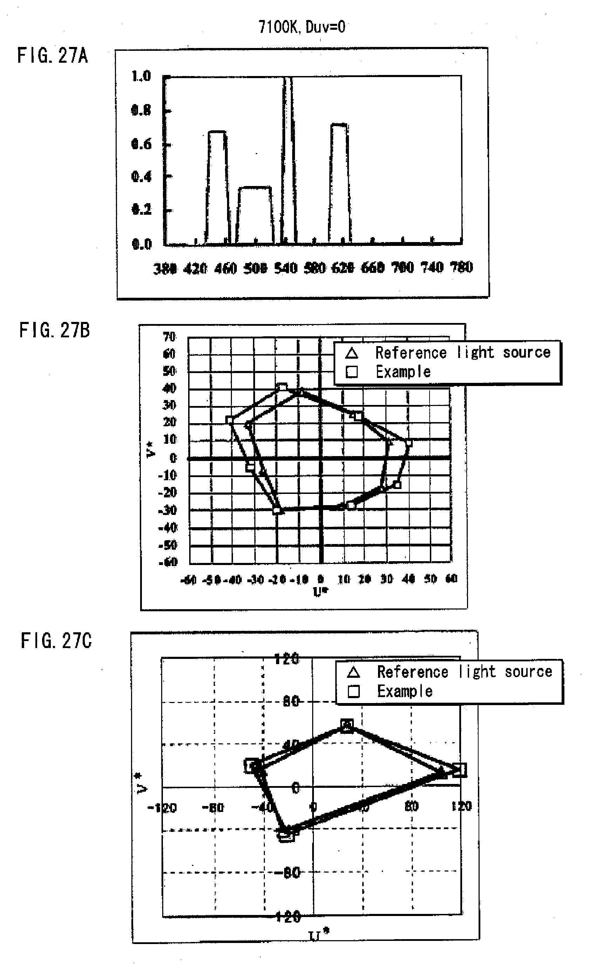

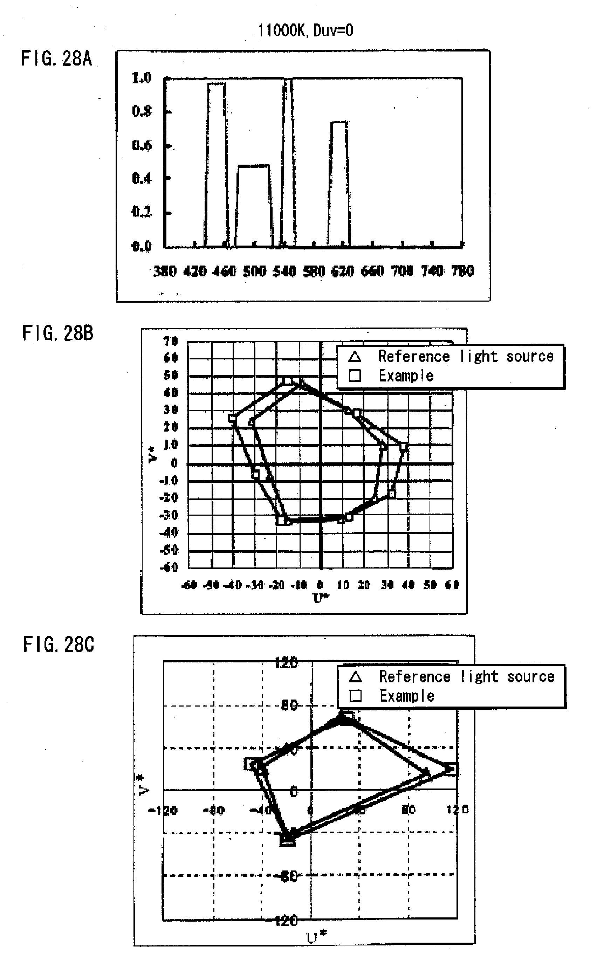

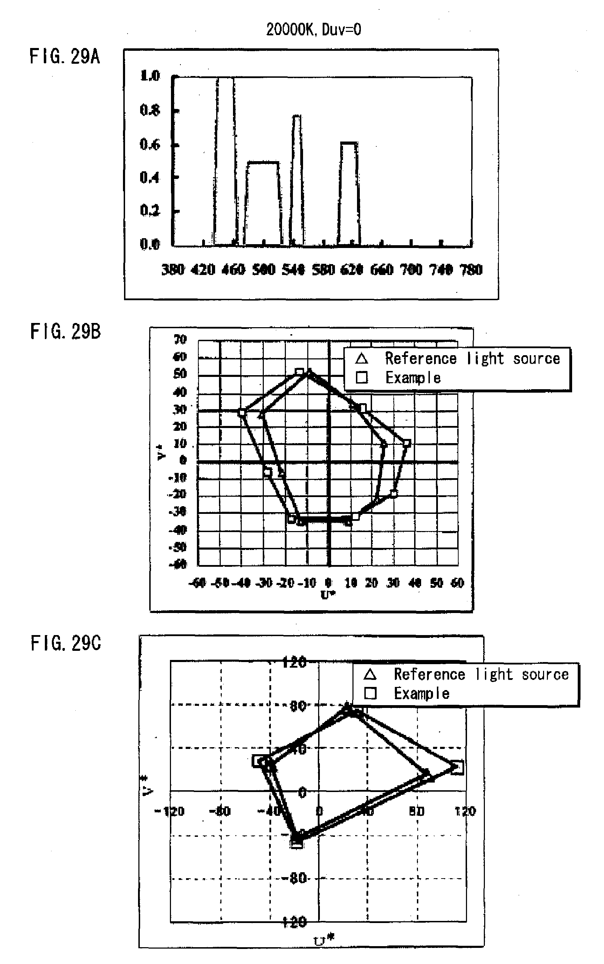

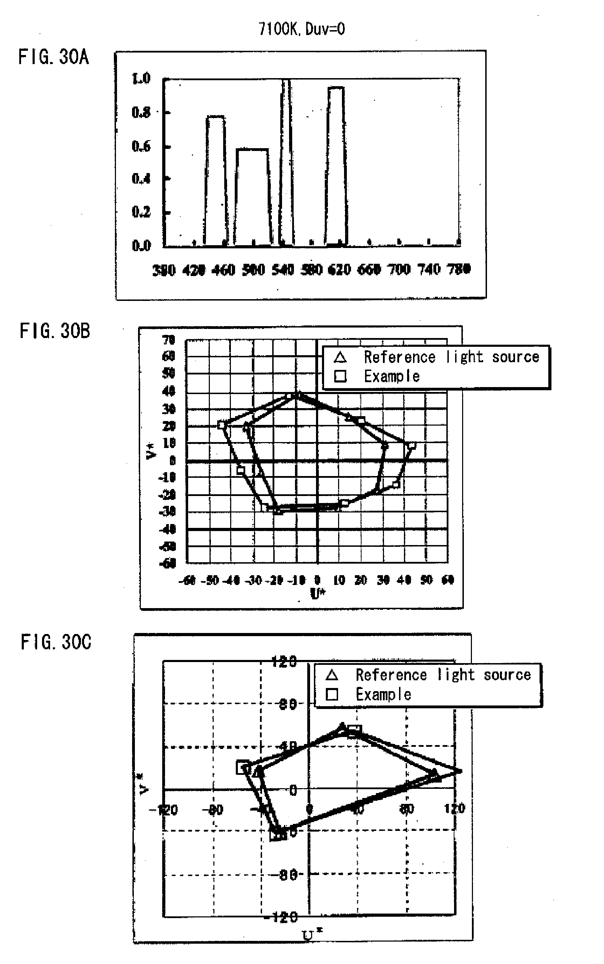

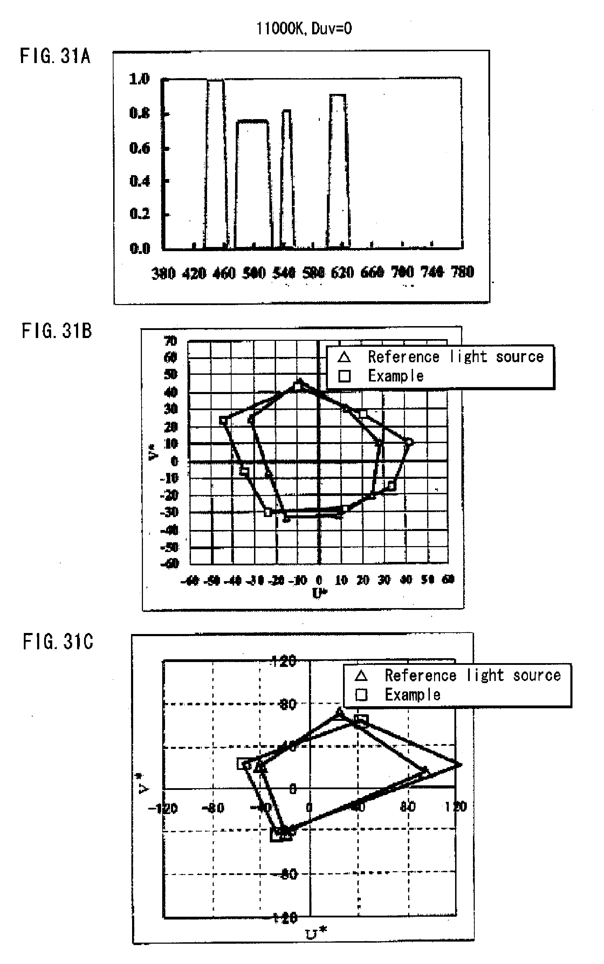

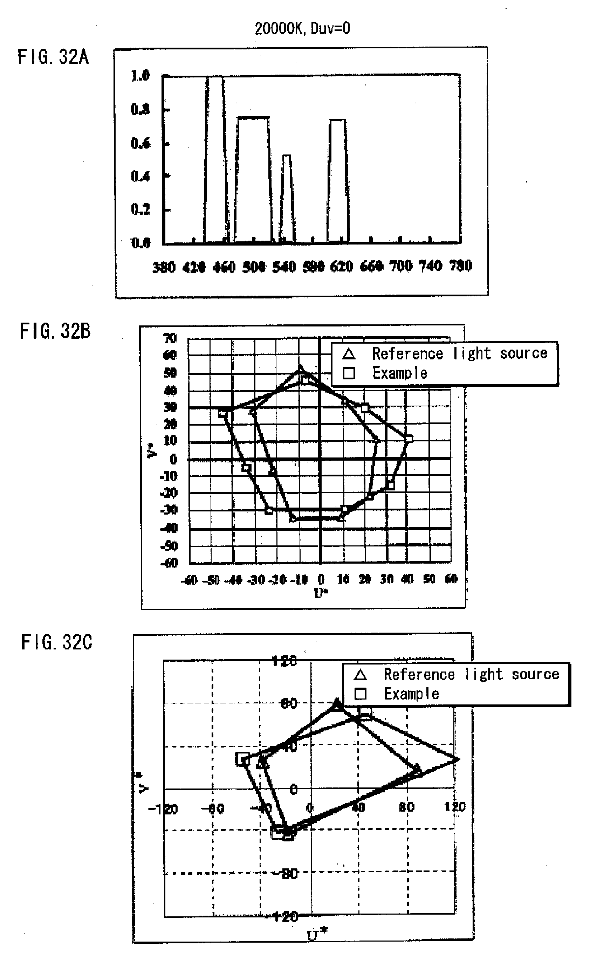

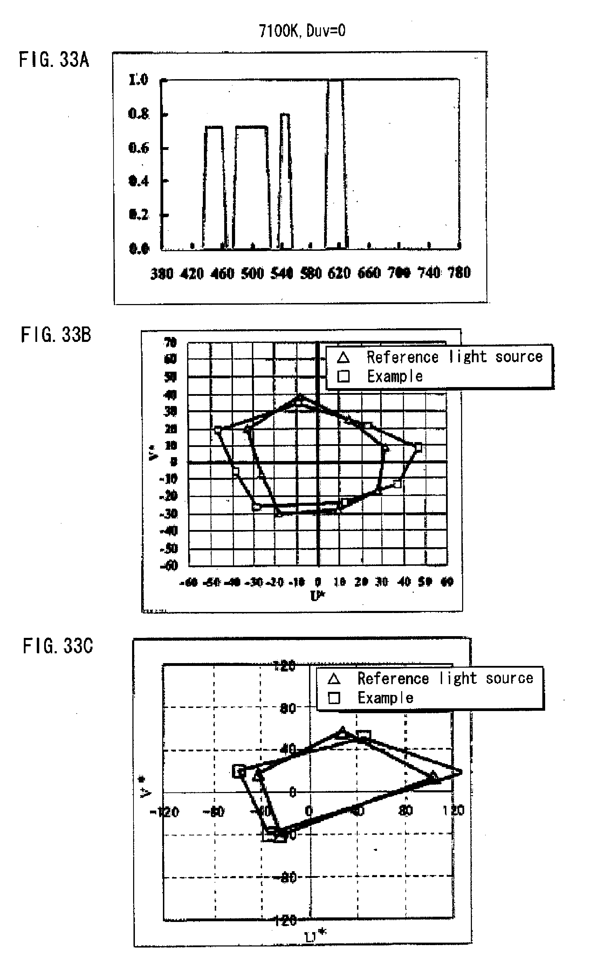

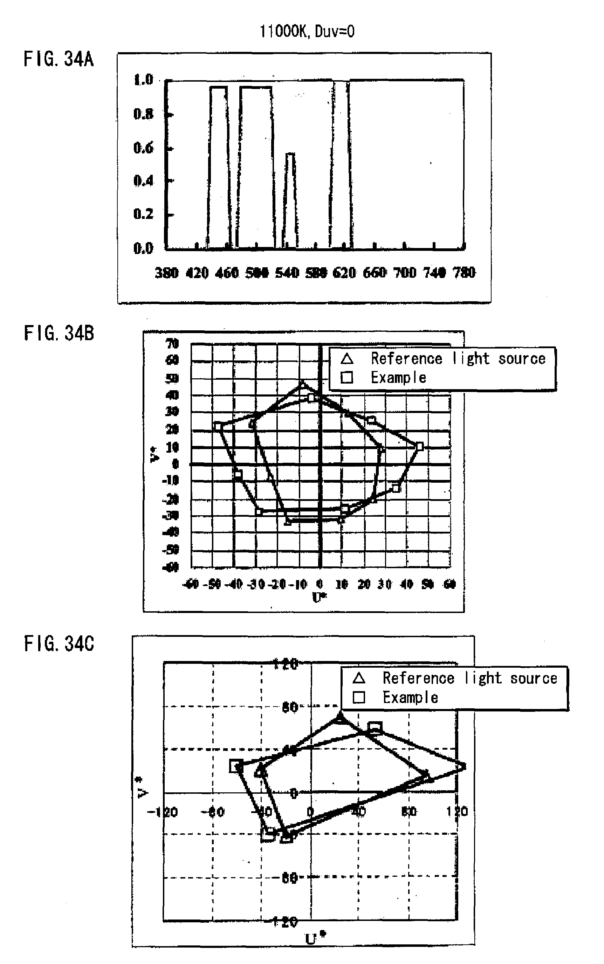

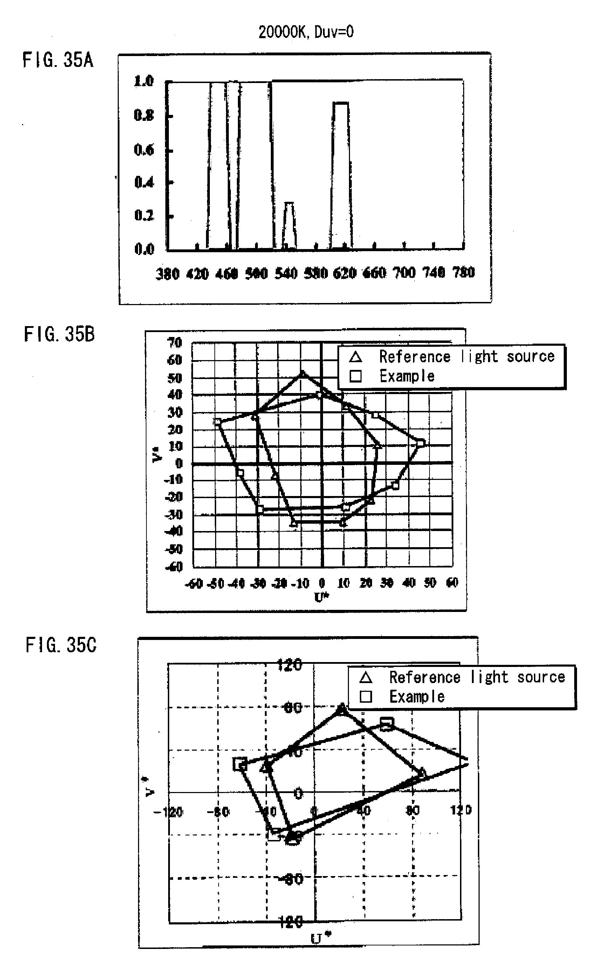

The emission between the main emission peaks of the green emitting rare-earth phosphor and the blue emitting rare-earth phosphor was enhanced under the assumption that the emission only appears in the region to which the emission was added to, as described above. FIGs. 27 to 29 show cases where (emission peak intensity 2/emission peak intensity 1) is 0.5; FIGs. 30 to 32 show cases where (emission peak intensity 2/emission peak intensity 1) is 0.75; and FIGs. 33 to 35 show cases where (emission peak intensity 2/emission peak intensity 1) is 1.0.

In each case, the prominence of the chroma of the color chip R3 for yellow, the chroma of R9 being lower than that pertaining to the reference light, the color gamuts being compressed in the red-green directions due to the deteriorated chroma of red and green, and the color gamuts being smaller with respect to those pertaining to the reference light, which are mentioned above, are significantly improved. Moreover, the color gamuts are clearly larger with respect to those pertaining to the reference light, more balanced in shape, and show tendencies to extend in the horizontal direction as is the case with those pertaining to an ordinary color temperature light color.

The shape of such gamut distributions are closer to that of gamut distributions of the reference light having a lower correlated color temperature. Accordingly, although the light color is a refreshing high color temperature, the illuminated object is rendered to give out a natural impression as if illuminated by a relatively low correlated color temperature light source. It also indicates that the addition of the blue green emission resulted not only in rise of the chroma of R11 for green but also in rise of the chroma of R9 for red. The apparent decrease of R9 and Ra is a result of the color rendition of the present invention enhancing the color rendition of the reference light in a favorable way. The present invention has found out that the light source with the above-mentioned features yield these tendencies.

The addition of a blue green spectrum naturally leads to a significant improvement of (action function efficiency/ visibility efficiency). When (emission peak intensity 2/emission peak intensity 1) is in a range of 0.5 to 1, a relatively large color gamut was able to be secured, compared to a case where the emission was focused in the red, green, and blue regions.

When the emission in the range of 480 [nm] to 520 [nm] is intense and the correlated color temperature is high in an ideal condition with high excitation purity, such as in a theoretical simulation, which does not include mercury emission lines, sub-emission of the phosphors, and extended tail, the green emission in the range of 540 [nm] to 550 [nm] is extremely low, exhibiting excessive spectrum distributions and changes of color gamuts compared to actual lamps. In this case, the color gamut is compressed in the horizontal direction, and the chroma starts decreasing in the blue-yellow directions. Based on the above, more preferably, the addition is 0.75 or smaller, considering that although the color gamut can be secured theoretically, an excessive addition disturbs the balance of the entire color reproduction.

Additionally, in actual lamps, because emissions in emission regions other than the ideal emission regions are superimposed, photostimulation purity in each region decreases. Consequently, the effect of the present invention can be achieved more preferably in cases where each spectrum is 50 [nm] or less, which is the half width desired for a narrow-band light source in general.

Here, changes of shapes of the color gamuts are indicated based on comparison between the result of the example case shown using BAM:Mn, LAP, and YOX in FIG. 12 and the result of the comparative case using BAM, LAP, and YOX shown in FIG. 14. FIG. 8 shows both the example case and the comparative case. In the embodiment shown in FIGs. 12 and 8, BAM:Mn has a ratio of Eu peak intensity of 1 to Mn peak intensity of approximately 0.7. These figures sufficiently reproduce the tendency of the theoretical simulation, that is, as a result of the addition of the blue green region spectrum of Mn, the color gamuts expand in the horizontal direction, i.e., the red-green directions, the color gamuts show little distortion, and are improved to be closer in shape to the color gamuts pertaining to the color reproduction using the reference light of a relatively low correlated color temperature. As is the case with the theoretical simulation, the higher the Mn peak intensity becomes, the further the color gamuts expand in the horizontal direction, i.e., the red-green directions.

Based on the integration of the embodiments 1 and 2, it is more preferable that the ratio (emission peak intensity 2/emission peak intensity 1) of the emission intensity of the emission peak of the emission center located in the range of 480 [nm] to 520 [nm] of the emission spectrum (emission peak intensity 2) to the emission intensity of the emission peak of the emission center located in the range of 440 [nm] to 460 [nm] of the emission spectrum (emission peak intensity 1) be in a range of 0.5 to 0.7, even more preferably, 0.6 or higher. This is considered preferable in the viewpoint of distinguishing the improvement on the characteristics, in order not to cause the emission efficiency of actual phosphors to decline and the difference from the conventional color rendition to be clearly perceived.

According to the theoretical simulation, the color rendering properties significantly improve at around 0.75; at 1.0, however, the emission efficiency of actual phosphors significantly decline. Accordingly, 0.8, which is a value in a range of up to 1.0 and is obtained by rounding 0.75, may be considered to be the practical highest value that can improve the color rendering properties while maintaining the emission efficiency. Thus, in a comprehensive standpoint, a range of 0.6 to 0.8 is a preferable range for implementation. Note that in recent years, implementation with higher priority on emission efficiency is often desired in the viewpoint of energy conservation. In this case, a range of 0.5 to 0.8 is an appropriate range for implementation.

Estimating emission efficiency and color rendering properties for a range of 0 to 0.5 using correlations between the ratio (emission peak intensity 2/emission peak intensity 1) and the emission efficiency shown in FIGs. 6, 7, and 17 to 20 enables implementations with the ratio at 0.4, 0.3, or the like, where the emission efficiency is emphasized. Based on subjective evaluations, the difference in color rendition from cases where the conventional blue green region emission is not enhanced was perceived at 0.4 or higher.

(Third Embodiment)

The following describes a third embodiment of the present invention. In the third embodiment, a theoretical explanation is given on a relative tendency of advantageous effects achieved by the present invention, based on the theoretical spectral distributions in a case where the half width is extended toward a long wavelength from the emission peak of the blue emitting rare-earth phosphor.

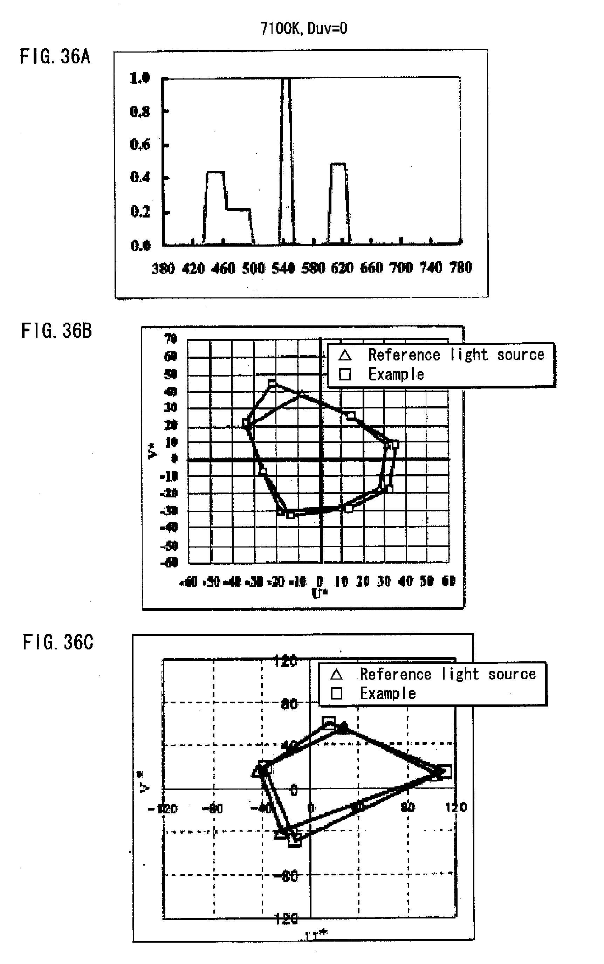

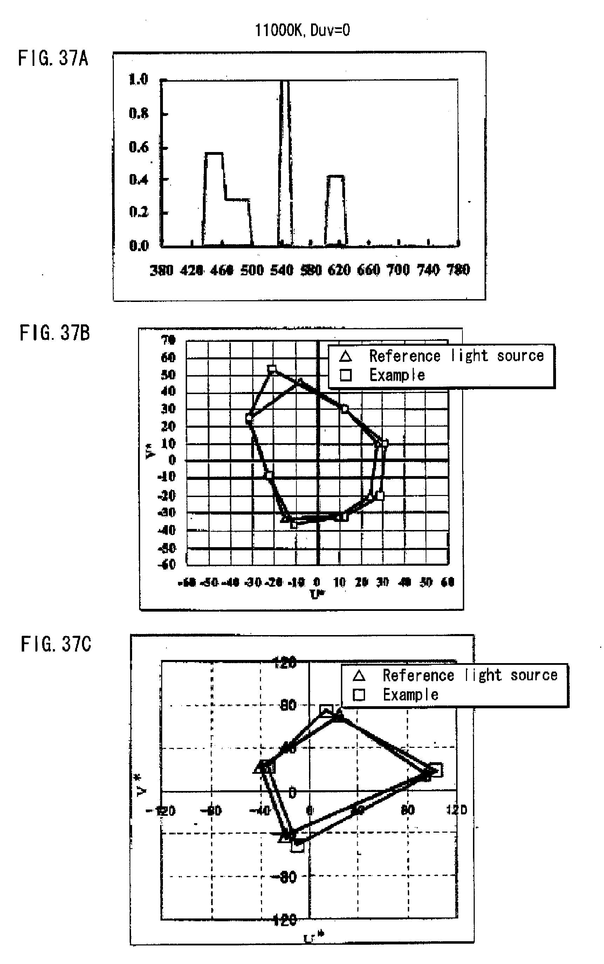

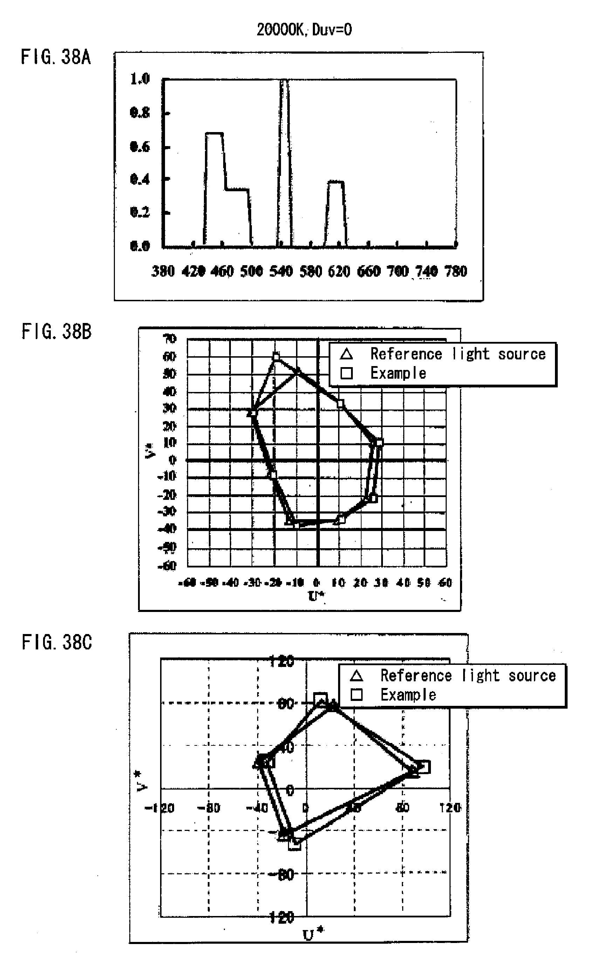

FIGs. 36 to 38 show results of simulations performed under the same conditions as the second embodiment except the following, in order to simulate that a blue phosphor which was used to enhance the emission between the main emission peaks of the green emitting rare-earth phosphor and the blue emitting rare-earth phosphor and which has the main emission peak in a range of 440 [nm] to 460 [nm] is a blue emitting phosphor whose half width from the emission peak toward a longer wavelength is 50 [nm] or greater: the emission intensity of the blue emitting phosphor being 100% in the range of 440 [nm] to 460 [nm]; and the emission intensity of the blue emitting phosphor being 50% in 50 [nm] from 460 [nm] toward a longer wavelength, to 510 [nm].

In this case, the resultant improvement does not indicate as large a shift of the color rendition toward higher chromas as in the second embodiment. Still, relatively sufficient faithful color reproduction is achieved in comparison with the reference light of the corresponding correlated color temperature. It should be noted, however, that the prominence of the chroma of the yellow color chip R3 peculiar to a three-wavelength light source is not improved, and Ga and Ga4 both show relatively small expansion in the horizontal direction.

Because the blue green spectrum is not concentrated in a narrow band, the portion of the color gamut pertaining to green shows relatively small enhancement. while the addition of the blue green spectrum naturally improves the ratio (action function efficiency/visibility efficiency), it is not as obvious as in the second embodiment, since it is only an addition of a gradual and low spectral power in a broad range.

From a viewpoint of adding a broad-band spectral, however, the above-mentioned tendency grows in an actual light source as emission from another spectral band is mixed and the excitation purity begins to decrease. Thus, from the viewpoint of improving numerical value of conventional Ra, Ri and the like to secure relatively sufficient faithful color reduction, in comparison with the reference light of the corresponding correlated color temperature, the above-described case of the present invention achieves an effect.

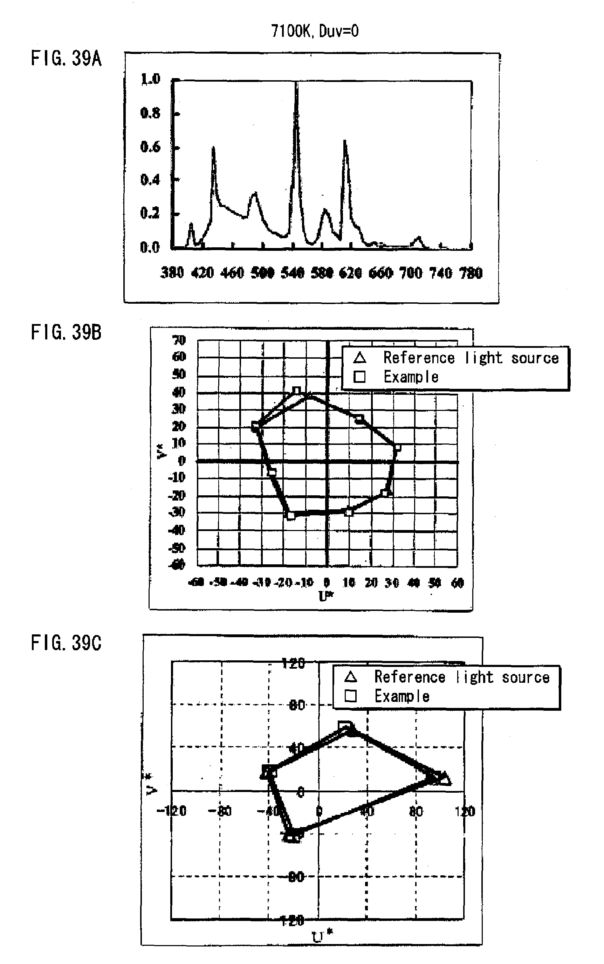

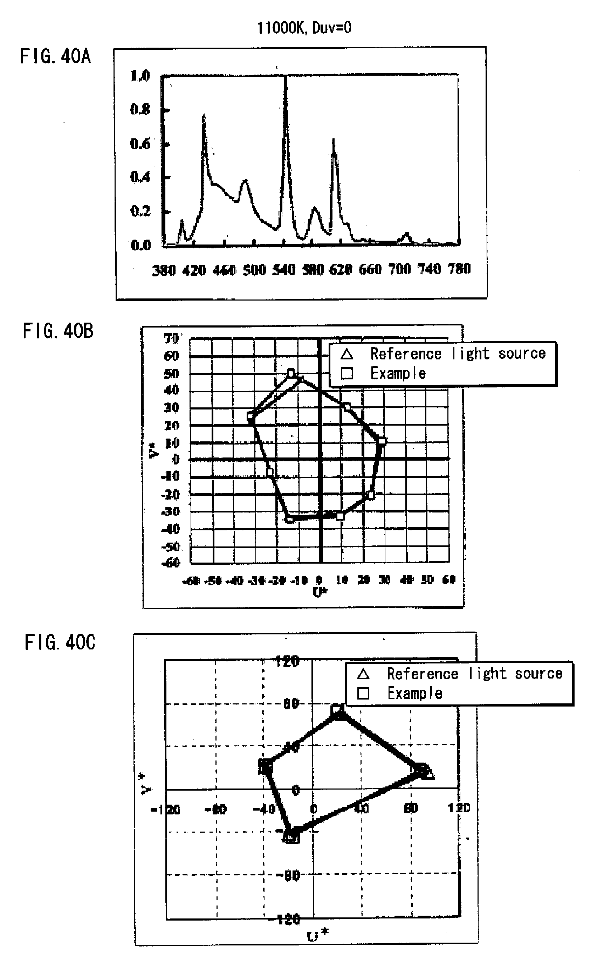

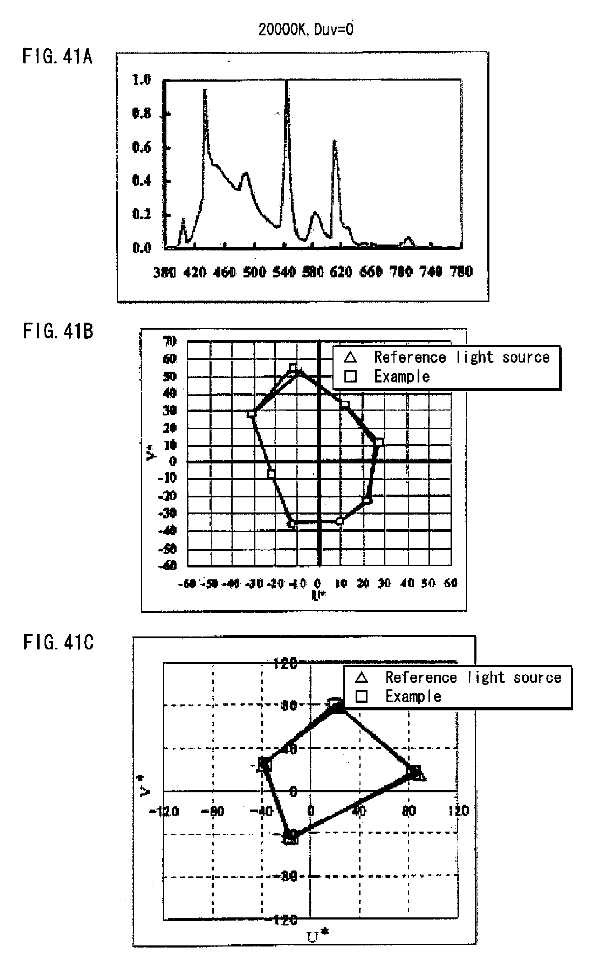

FIGs. 39 to 41 show results of cases using SCA, LAP, and YOX, at the corresponding correlated color temperature and Duv, to be compared with the simulation results shown in FIGs. 36 to 38. SCA is a blue emitting phosphor for use in actual fluorescent lamp, and has a half width of 50 [nm] or greater from the emission peak toward a longer wavelength.

As shown by these figures, the chroma of the high chroma color chips rises less than in the first embodiment in which the blue green spectrum is concentrated in a narrow band. The theoretical simulations sufficiently represent the tendencies of these actual results by reproducing the above-described characteristics.

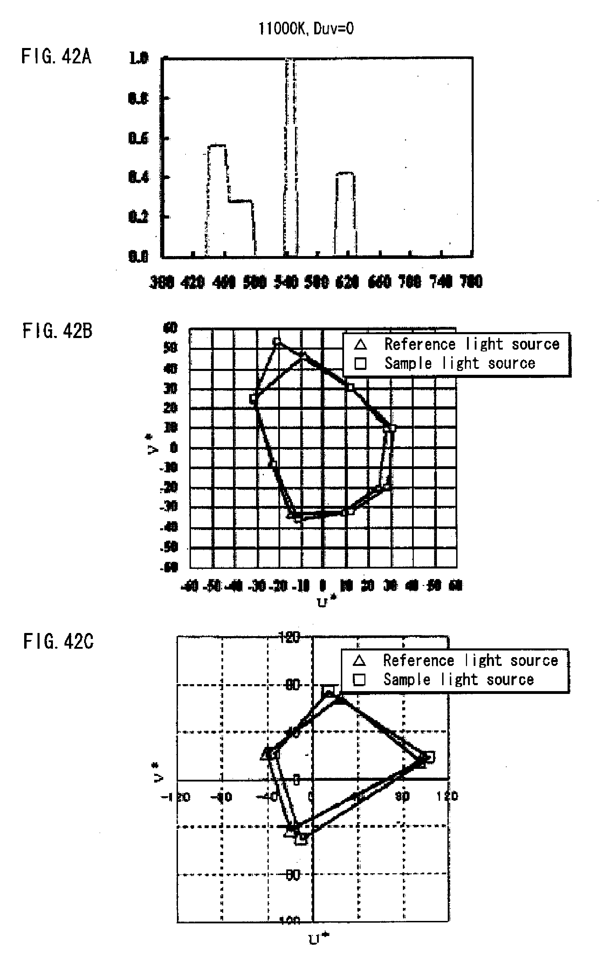

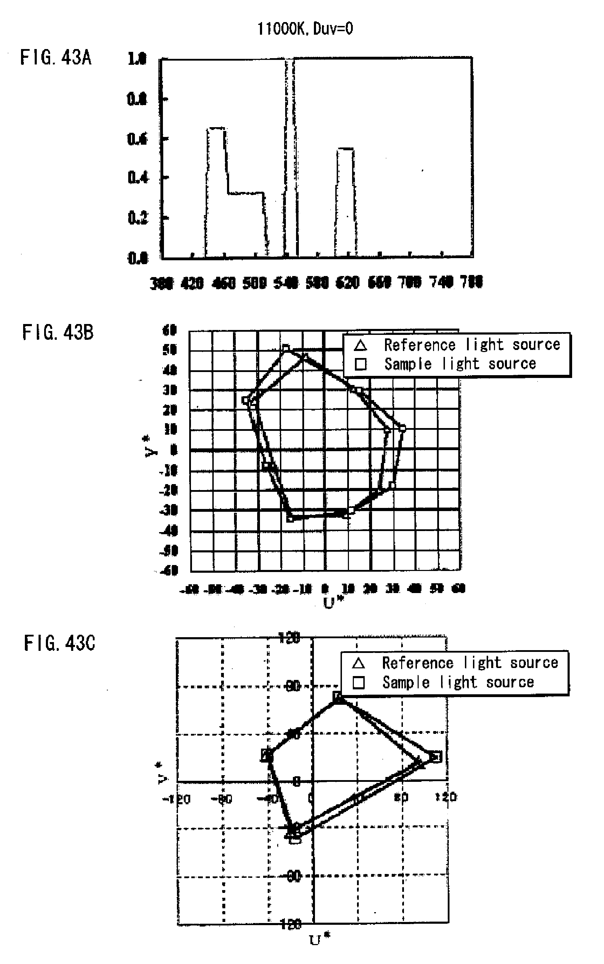

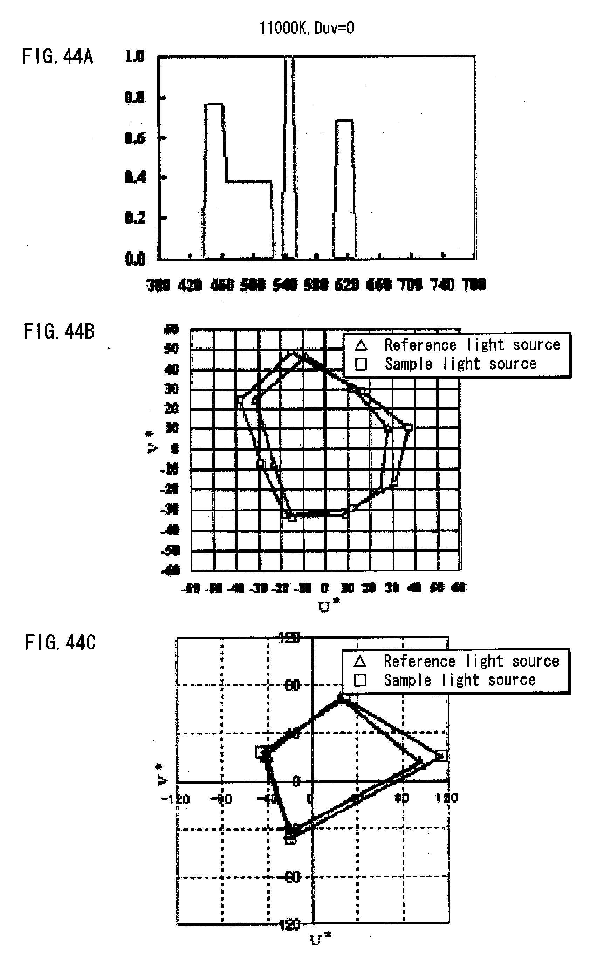

Furthermore, FIGs. 42 to 44 show simulation cases in which the half width from the emission peak toward a longer wavelength is varied, where the correlated color temperature is 11000 [K] and Duv = 0. FIG. 42 shows a case where the emission intensity is 50% in a range of 465 [nm] to 495 [nm]; FIG. 43 shows a case where the emission intensity is 50% in a range of 465 [nm] to 510 [nm], where the half width of 50 [nm] is assumed in a simulative manner; and FIG. 44 shows a case where the emission intensity is 50% in a range of 465 [nm] to 520 [nm].

When the half width is 50 [nm] or smaller, the color gamut is only slightly larger than the color gamut pertaining to the reference light. However, when the half width is 50 [nm] or greater, the horizontal width of the color gamut, i.e., the width in the green-red directions, exceeds the color gamut pertaining to the reference light. It should be noted here, however, that that the portion of the color gamut pertaining to green is not as large as in the case where a phosphor having the emission peak in the range of 480 [nm] to 520 [nm] is mixed.

Additionally, in portions of the color gamut pertaining to the high chroma color chips, a wider half width causes a decrease in the color gamut in the vertical direction, i.e., the yellow-blue directions, and the decrease is especially obvious in the blue.

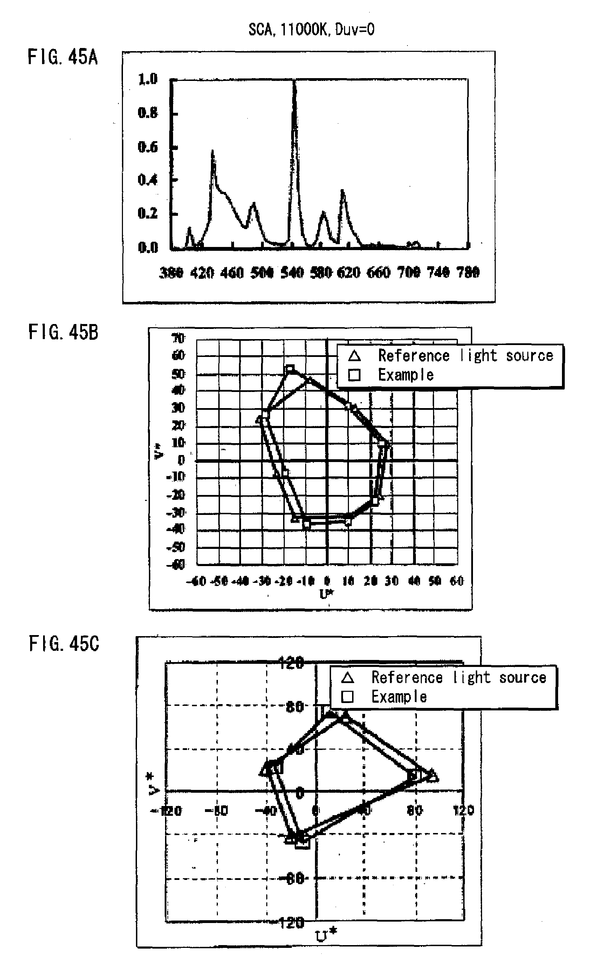

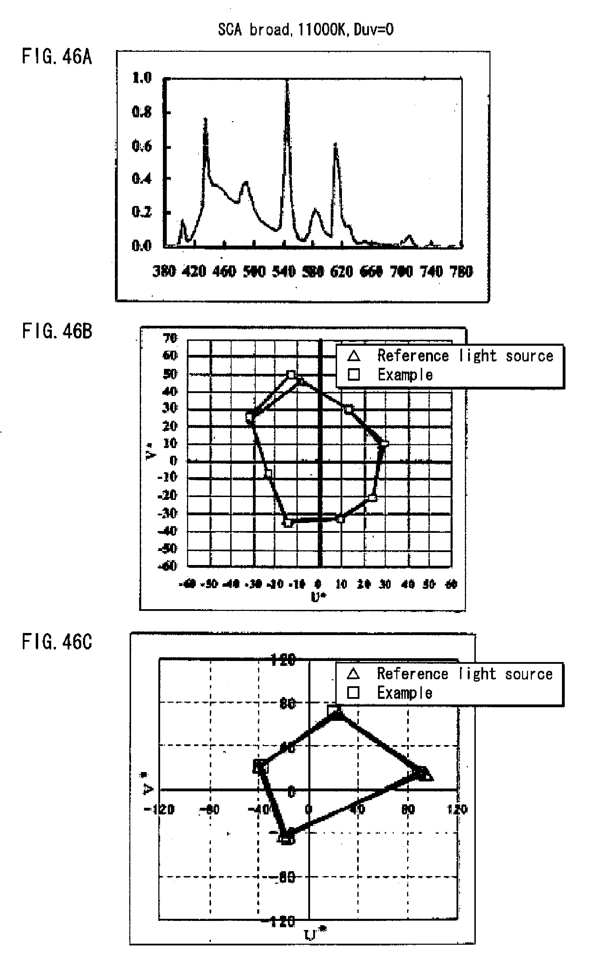

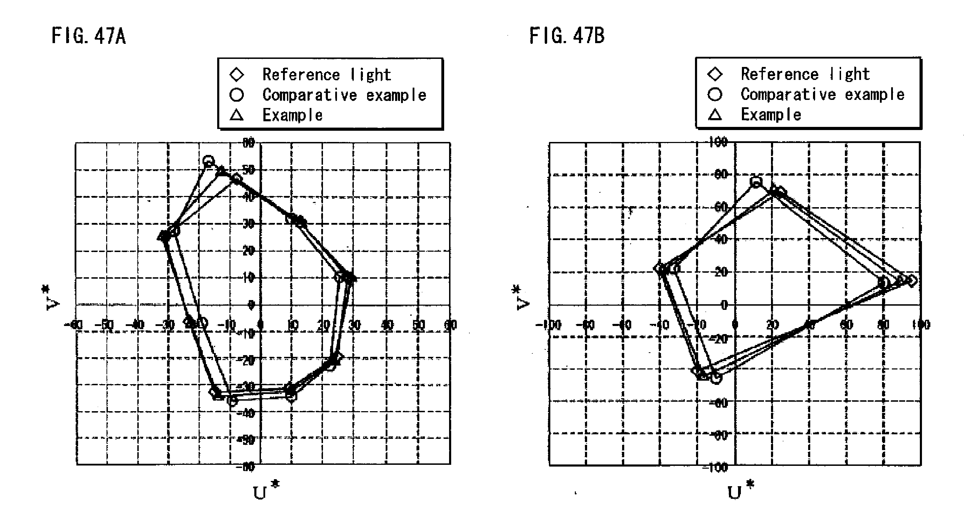

FIG.s 45 and 46 show cases for comparison with each other, where the correlated color temperature is 11000 [K] and Duv = 0 for both figures. FIG. 45 shows a case in which the half width from the emission peak of the SCA phosphor toward a longer wavelength is 50 [nm] or smaller and thus the emission in the range of 460 [nm] to 480[mn] is small ("SCA"); and FIG. 46 shows a case in which the half width from the emission peak of the SCA phosphor toward a longer wavelength is 50 [nm] or greater and thus the emission in the range of 460 [nm] to 480[mn] is not small ("SCA broad"). Note that FIG. 47 shows the comparison of these cases, the comparative case showing the result of SCA and the example case showing the result of SCA broad.

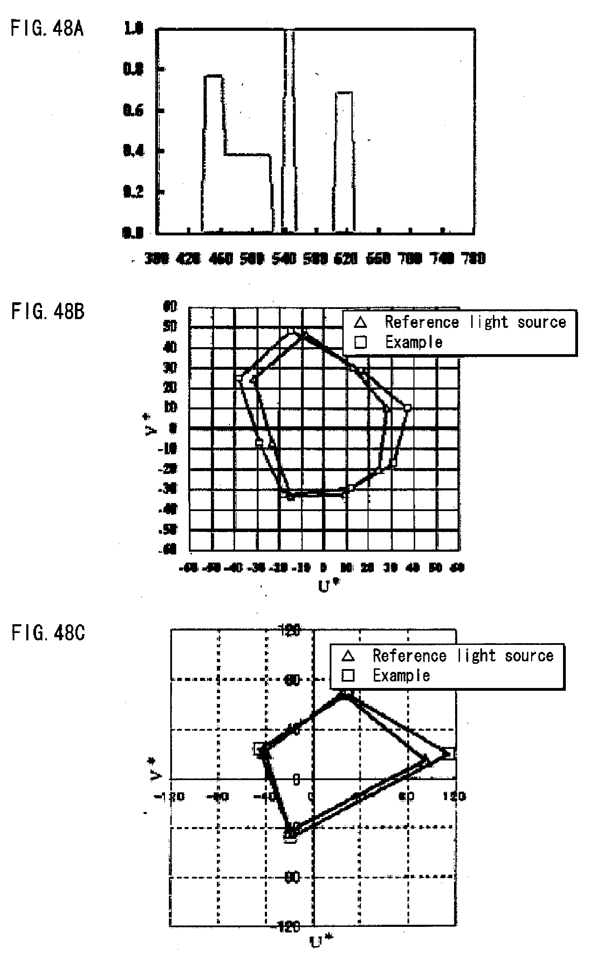

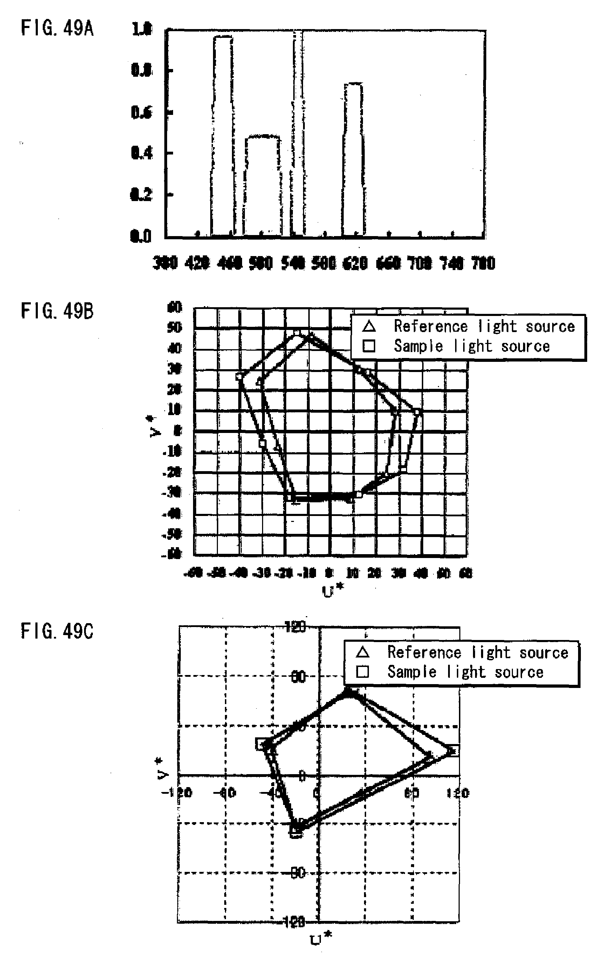

In the case where the emission in the range of 460 [nm] to 480 [mn] increases, the color gamut becomes closer, though unlikely to exceed, in size to the color gamut pertaining to the reference light, reflecting the effect of the increase. However, in the horizontal direction, i.e., the red-green directions of the color gamut, this effect does not achieve an increase as large as in the embodiment of the lamp of the present invention in which the emission is enhanced in a narrow band between the main emission peaks of the green emitting rare-earth phosphor and the blue emitting rare-earth phosphor. This characteristic is especially significant in the color gamut defined by the high chroma color chips. Here, a detailed comparison is made between a case where the emission is enhanced in a narrow band of 480 [nm] to 520 [nm] by means of widening the half width and a case where the emission is enhanced in a narrow band, with reference to FIGs. 48 and 49.

FIG. 48 shows results of a simulation for the case with the wider half width; emission having emission intensity of 50% is successively added in a range of 465 [nm] to 520 [nm]. FIGs. 49 show a simulation for the case where emission with an emission intensity of 50% is added in the narrow band of 480 [nm] to 520 [nm].

Based on FIGs. 48 and 49, it is understood that in the case of enhancing the emission in the narrow band of 480 [nm] to 520 [nm], the color gamuts extend wider in the horizontal direction than in the case of enhancing the emission in the range of 480 [nm] to 520 [nm] by extending the half width. This is because the excitation purity for green increases, and extension of the color gamut defined by the high chroma color chips in the green direction is particularly conspicuous.

In the case of enhancing the emission in the range of 480 [nm] to 520 [nm] by widening the half width, excitation purity of blue and green lowers, and as a result, the color gamut defined by the medium chroma color chips shows a significant decrease in the blue direction. This phenomenon also appears in a case where actual phosphors are used, indicating that the enhancement of the emission causes differences between the results of the examples.

As described above, the addition of a light blue green spectrum in a broad range improves (action function efficiency/visibility efficiency), though not as much as in the first and second embodiments. However, in a practical case using an actual fluorescent lamp, the emission efficiency deteriorates with respect to that in the first and second embodiments due to a significant extended tail of the half width toward a longer wavelength. Thus, according to the present embodiment, it is relatively difficult to maintain the emission efficiency while also improving Ra, Ri and the like at the corresponding correlated color temperature and Duv of an ordinary three-wavelength light source. However, it is an advantageous effect that the conventional Ra, Ri and the like improve in a preferable manner.

(Fourth Embodiment)

The following describes a fourth embodiment of the present invention. In the fourth embodiment, an explanation is given on a fluorescent lamp in which an emission between the main emission peaks of the green emitting rare-earth phosphor and the blue emitting rare-earth phosphor is enhanced by a blue emitting rare-earth phosphor containing an element whose main emission peak is located in the range of 440 [nm] to 460 [nm] and whose half width from the emission peak to a longer wavelength side is 50 [nm] or greater.

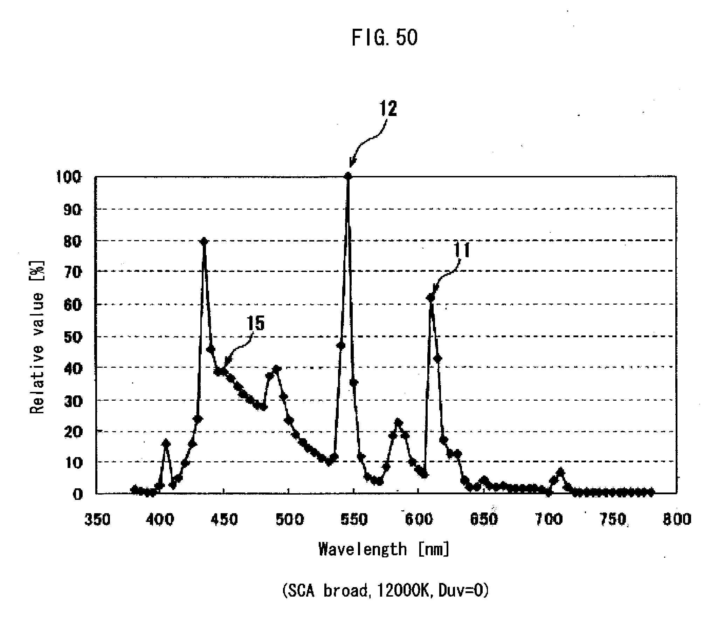

FIG. 50 shows spectral distributions of the fluorescent lamp of the fourth embodiment. In FIG. 50, 11 is an Eu emission peak of a red emitting rare-earth phosphor YOX which contains a rare-earth element as an emission center whose main emission peak is located in a range of 605 [nm] to 625 [nm]; 12 is a Tb emission peak of a green emitting rare-earth phosphor LAP which contains a rare-earth element as an emission center whose main emission peak is located in a range of 540 [nm] to 550 [nm]; 13 is an emission peak of a blue emitting rare-earth phosphor SAC which contains a rare-earth element whose main emission peak is located in a range of 440 [nm] to 460 [nm] and whose half width from the emission peak toward a longer wavelength is 50 [nm] or greater.

Because the half width of the blue emitting rare-earth phosphor from the emission peak thereof toward a longer wavelength is 50 [nm] or greater, an emission between the main emission peaks of the green emitting rare-earth phosphor and the blue emitting rare-earth phosphor is enhanced.

While the SCA phosphor exhibits a spectral emission similar to BAM generally, it is possible to increase the half width from the emission peak toward a longer wavelength by adding an additive such as Sr. In the case where the half width from the emission peak toward a longer wavelength is increased, as in the simulation described above, the chroma rises in the red-green directions compared to a case where no material for increasing the half width is added.

The following are results of calculations using SCA whose half width from the emission peak toward a longer wavelength is increased by 50 [nm] or more:

correlated color temperature = 12000 [K], Duv = 0, Ra = 93, Ga = 104, Ga4 = 97, R9= 73, R10 = 80, R11 = 92, R12 = 84, R13 = 92, R14 = 83, R15 = 91;
(action function efficiency/visibility efficiency) =
1.25 where (visual pigment-based: the first-type action function), or
1.27 where (direct measurement-based: the second type-action function)

As described above, the blue emitting rare-earth phosphor having (action function efficiency/visibility efficiency) of 1 or greater and containing the rare-earth element whose main emission peak is located in the range of 440 [nm] to 460 [nm] between the main emission peaks of the green emitting rare-earth phosphor and the blue emitting rare-earth phosphor can be compared with FIG. 6 of the first embodiment in both of the following two cases: the case where the half width from the emission peak toward a longer wavelength is 50 [nm] or wider; and the case where the half width is narrower than 50 [nm]. The comparison results show that the ratio of action function efficiency/visibility efficiency, Ra, and R9 improve in a wide range.

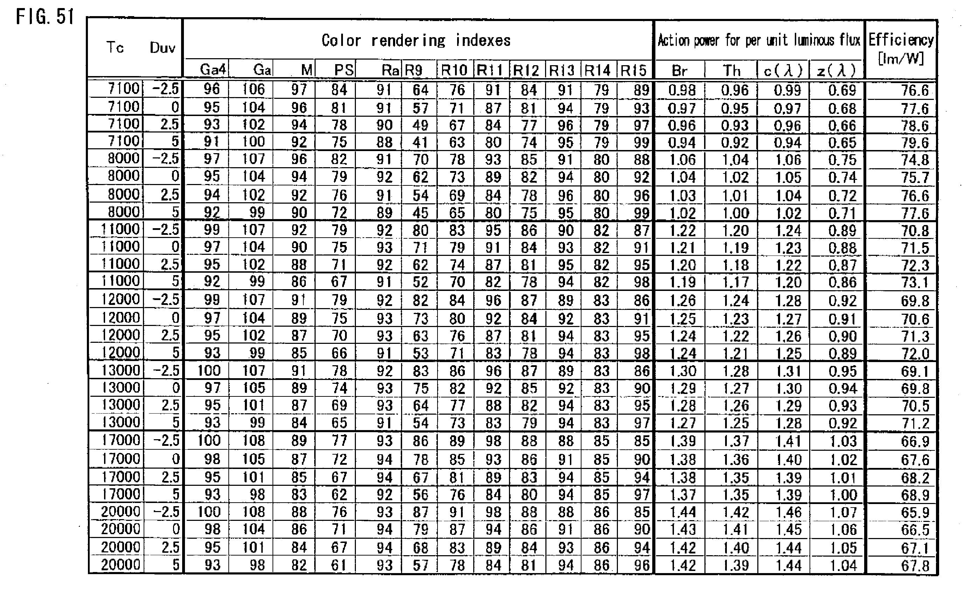

FIG. 51 shows values of various indexes for the present embodiment where the blue emitting rare-earth phosphor SCA whose half width from the emission peak toward a longer wavelength is 50 [nm] or more, LAP, and YOX are used and Duv is varied for each correlated color temperature.

Here, as in the second embodiment, varying Duv does not cause a large difference with respect to the conventional case; and as in a case of using BAM, LAP, and YOX which are used in a conventional ordinary narrow-band fluorescent lamp and increasing Duv, the emission efficiency improves but Ra, R9 to R12 and the like decrease as Duv increases. Also, when Duv is a positive value, it is unlikely that Ga and Ga4 exceeds 100, which is the value corresponding to the reference light, and it is especially unlikely for Ga4.

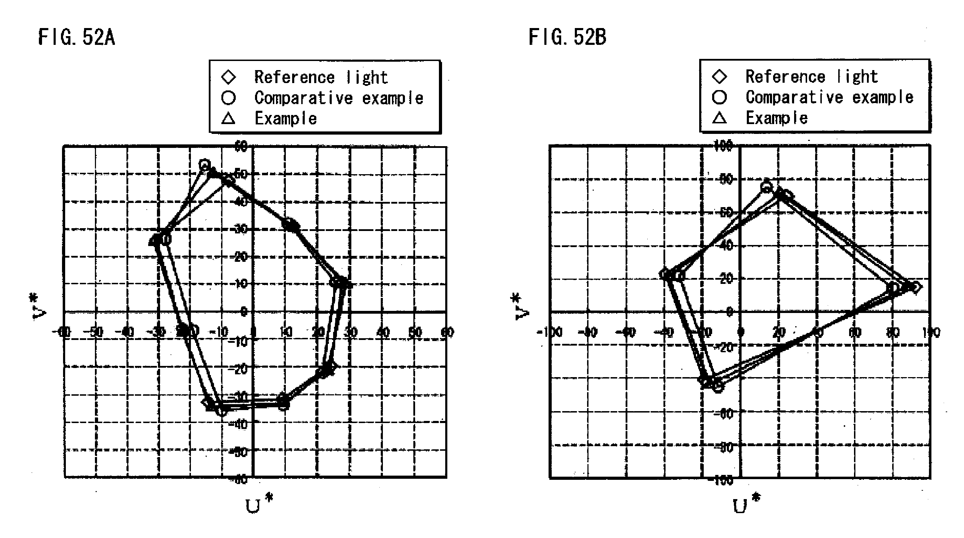

However, it is possible to improve Ra, R9, and the like in terms of numerical value by enhancing the emission in this region, and an advantageous effect can be achieved in the standpoint of securing comparatively sufficient faithful color reproduction in comparison with the reference light of the corresponding correlated color temperature. FIG. 52 shows these tendencies in further detail, using a case where the correlated color temperature is 12000 [K] and Duv = 0.

In the figure, the comparative example corresponds to SCA, and the example corresponds to SCA broad.

The distribution tendencies of the color gamut are consistent with the simulation of the third embodiment. The color gamut extends in the red-green directions, and more specifically, the extension is more significant in the red direction. It should be noted, however, that the extension in the green direction is smaller than the case where the phosphor whose emission peak is located in the range of 480 [nm] to 520 [nm] is mixed.

Additionally, it is also understood by comparing FIGs. 47 and 52 that SCA whose half width from the emission peak toward a longer wavelength is 50 [nm] or smaller and BAM exhibits substantially the same behaviors in terms of color rendition characteristics.

(Fifth Embodiment)

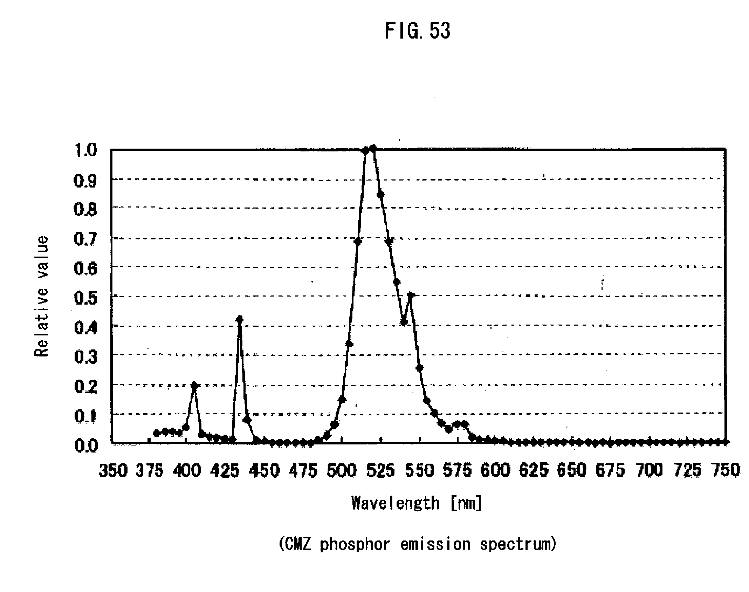

FIG. 53 shows spectral distributions of a CMZ phosphor used in a fifth embodiment of the present invention. The present embodiment uses the following combination of phosphors: a red emitting rare-earth phosphor YOX containing a rare-earth element as an emission center whose main emission peak is located in a range of 605 [nm] to 625 [nm]; a green emitting rare-earth phosphor LAP containing a rare-earth element as an emission center whose main emission peak is located in a range of 540 [nm] to 550 [nm]; a blue emitting rare-earth phosphor BAM containing a rare-earth element as an emission center whose main emission peak is located in a range of 440 [nm] to 460 [nm]; and in addition, the phosphor CMZ containing an element whose main emission peak is located in a range of 480 [nm] to 520 [nm] between the main emission peaks of the blue emitting rare-earth phosphor and the green emitting rare-earth phosphor.

The phosphor used in the present embodiment is a phosphor whose emission peak location and half width differ from those of the Mn emission peak of the BAM:Mn phorphor. The emission peak of the phosphor of the present embodiment has the half width of 50 [nm] or smaller and belongs to a narrow band emission, and accordingly, the emission can be concentrated in a targeted emission band efficiently.

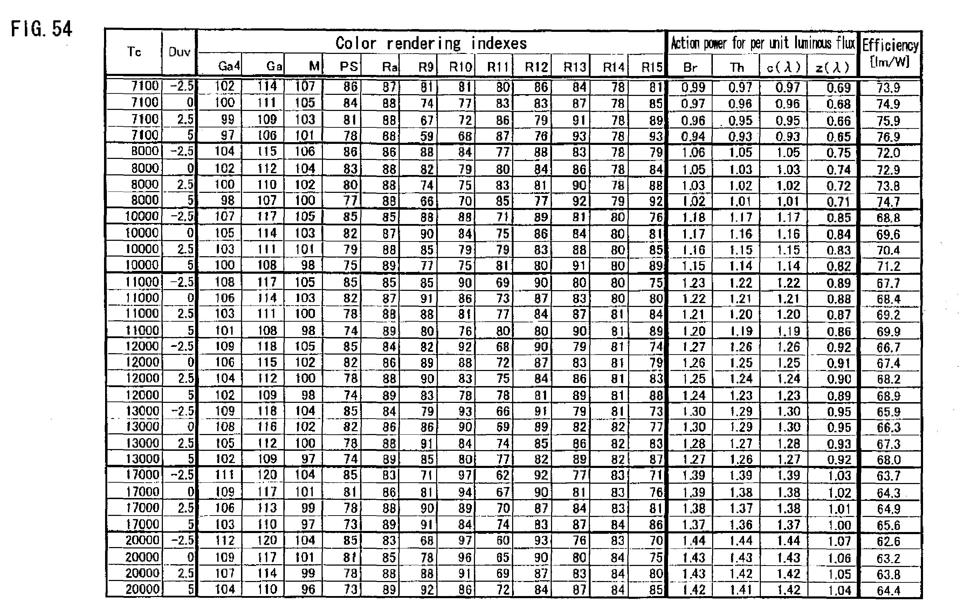

FIG. 54 shows various evaluation values for the present embodiment with Duv varied for each correlated color temperature. In the case shown in FIG. 54, the ratio (emission peak intensity 2/emission peak intensity 1) of the emission intensity of the emission peak 2 of the emission center located in the range of 480 [nm] to 520 [nm] to the emission intensity of the emission peak 1 of the emission center located in the range of 440 [nm] to 460 [nm] is approximately 0.75.

Comparing the evaluation values with those in FIG. 6 of the first embodiment reveals improvements in a broad range of the (action function efficiency/visibility efficiency), Ra, and R9. Also, although the emission peak and the half width are different, the above-described tendencies resemble those in the case of the first embodiment of the present invention, in which BAM:Mn is used and the Mn peak ratio is approximately 0.7 as shown in FIG. 7. Note that since emission efficiency varies in actual phosphors, use of the CMZ phosphor of the present embodiment leads to deterioration of the emission efficiency as a lamp with respect to the implementation using BAM:Mn.

From the viewpoint of suppressing deterioration of the emission efficiency, in addition to the viewpoint of improving color rendering properties, it is preferable in general that the emission intensity of the emission peak 2 of each phosphor is low. Specifically, it is preferable that the ratio (emission peak intensity 2/emission peak intensity 1) of the emission intensity of the emission peak 2 of the emission center located in the range of 480 [nm] to 520 [nm] to the emission intensity of the emission peak 1 of the emission center located in the range of 440 [nm] to 460 [nm] is approximately 0.75 or lower, which is a midpoint of 0.5 and 1.0.

Furthermore, the following applies not only to the present embodiment but also to a three-wavelength fluorescent lamp in general: when a relative comparison is made between the emission efficiency of daylight white N having the correlated color temperature of about 5000 [K] and the emission efficiency of daylight D having the correlated color temperature in the range of 6500 [K] to 7000 [K], with the emission efficiency of the daylight white being 100, the emission efficiency of daylight D is around 95; accordingly, it is assumed that the emission efficiency of the light color having a one-level higher color temperature is preferably around 90.

From this viewpoint, it is preferable that the correlated color temperature be 13000 [K ] or lower and Duv be 0 or higher, or exceed 0.

From the viewpoint of the color rendering properties, it is possible to reproduce preferable appearance of red with R9 of not less than 60, despite Ga4 being nearly 100, when the following criteria are satisfied: the ratio (emission peak intensity 2/emission peak intensity 1) of the emission intensity of the emission peak 2 of the emission center in the range of 480 [nm] to 520 [nm] to the emission intensity of the emission peak 1 of the emission center in the range of 440 [nm] to 460 [nm] is 0.5 or higher; the correlated color temperature is 10000 [K] or higher; and Duv is in a range up to 5, where the light color is not likely to be perceived as greenish. Additionally, it is more preferable that the half width of the emission center added in the range of 480 [nm] to 520 [nm] be 50 [nm] or narrower.

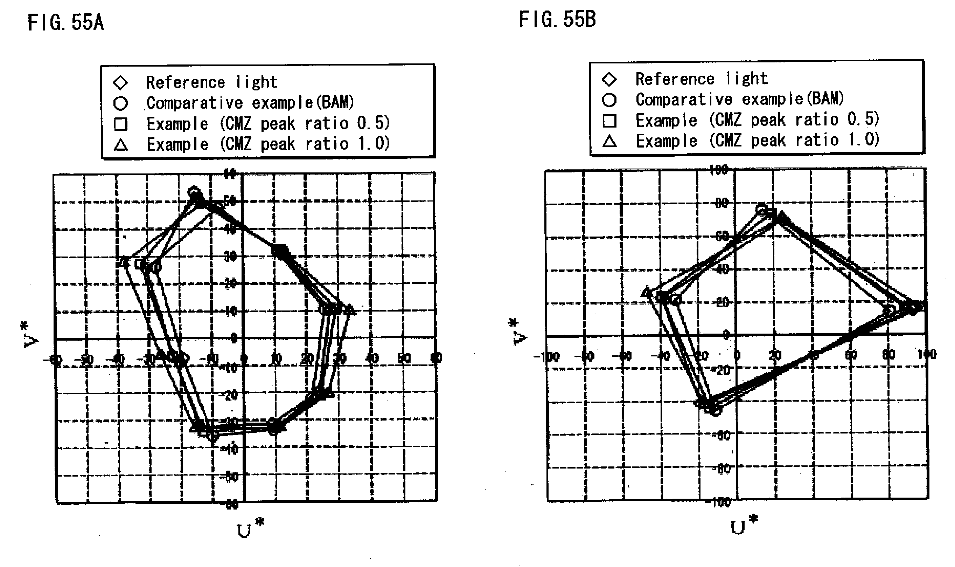

FIG. 55 shows the color gamuts of the present embodiment in more detail, using a case where the correlated color temperature is 12000 [K] and Duv = 0. The distribution tendencies of the color gamut are consistent with the simulation of the third embodiment. The color gamut extends in the red-green directions, and more specifically, the extension is more significant in the red direction. It should be noted, however, that strictly speaking, although located in the range of 480 [nm] to 520 [nm], the emission peak is further toward a longer wavelength compared to the case where the BAM:Mn phosphor is mixed, and accordingly, the extension in the green direction is small. Yet, the tendencies correspond with those of the other embodiments, indicating that the advantageous effects are broadly maintained within the scope of the present invention.

(Sixth Embodiment)

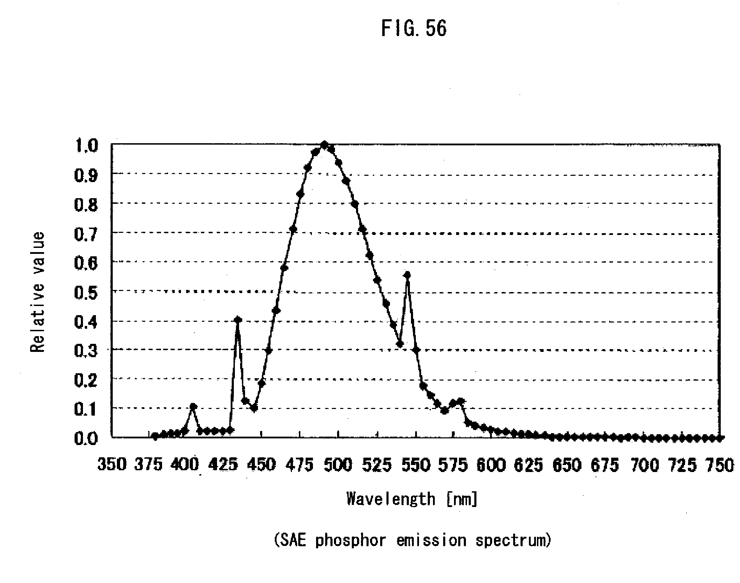

FIG. 56 shows spectral distributions of an SAE phosphor used in a sixth embodiment of the present invention. The present embodiment uses the following combination of phosphors: the red emitting rare-earth phosphor YOX containing a rare-earth element as an emission center whose main emission peak is located in the range of 605 [nm] to 625 [nm]; the green emitting rare-earth phosphor LAP containing a rare-earth element as an emission center whose main emission peak is located in the range of 540 [nm] to 550 [nm]; the blue emitting rare-earth phosphor BAM containing a rare-earth element as an emission center whose main emission peak is located in the range of 440 [nm] to 460 [nm]; and in addition, the phosphor SAE containing an element whose main emission peak is located in the range of 480 [nm] to 520 [nm] between the main emission peaks of the blue emitting rare-earth phosphor and the green emitting rare-earth phosphor.

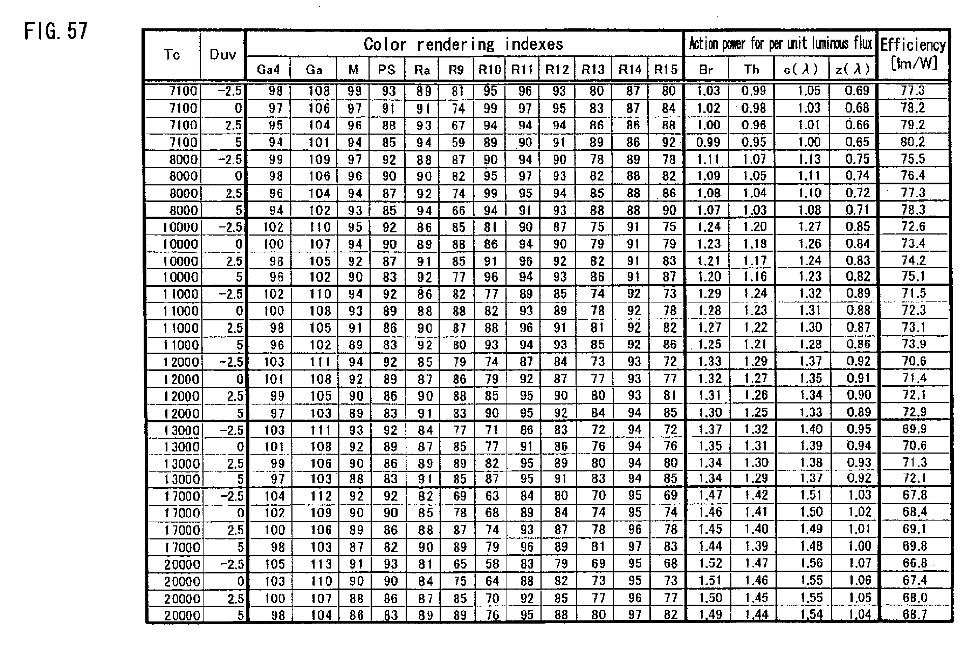

The phosphor used in the present embodiment is a phosphor whose emission peak location and half width differ from those of the Mn emission peak of the BAM:Mn phosphor. The emission peak of the phosphor of the present embodiment has a half width of 50 [nm] or greater. FIG. 57 shows various evaluation values for the present embodiment with Duv varied for each correlated color temperature.

In the case shown in FIG. 57, the ratio (emission peak intensity 2/emission peak intensity 1) of the emission intensity of the emission peak 2 of the emission center in the range of 480 [nm] to 520 [nm] to the emission intensity of the emission peak 1 of the emission center in the range of 440 [nm] to 460 [nm] is approximately 0.75.

Comparing the evaluation values with those in FIG. 6 of the first embodiment reveals improvements in a broad range of the (action function efficiency/visibility efficiency), Ra, and R9. Also, in the case where the emission peak and the half width exceed 50 [nm], the above-described tendencies resemble those in the case of the first embodiment of the present invention, in which BAM:Mn is used and the Mn peak ratio is approximately 0.7 as shown in FIG. 7.

However, Ga4 exceeds 100 in a smaller range, and Ra is less likely to exceed 90. In addition, deterioration of the emission efficiency becomes greater than the case where the emission is concentrated in a narrow band, showing an aspect between the case using SCA in the fourth embodiment and the case using BAM:Mn of the first embodiment.

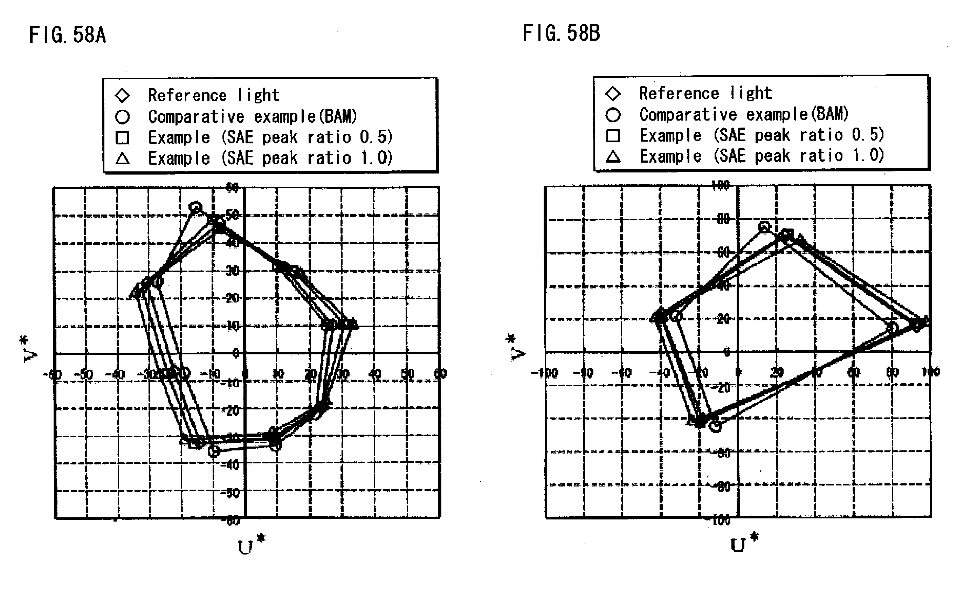

FIG. 58 shows the color gamuts of the present embodiment in more detail, using a case where the correlated color temperature is 12000 [K] and Duv = 0. The distribution tendencies of the color gamut are consistent with the simulation of the third embodiment. The color gamut extends in the red-green directions, and more specifically, the extension is more significant in the red direction. It should be noted, however, that more specifically, the extension in the green direction is comparatively smaller than a case where the phosphor whose emission peak is located in the range of 480 [nm] to 520 [nm].

More specifically, the present embodiment shows tendencies closer to a case where emission is enhanced in a broad band between the main emission peaks of the blue emitting rare-earth phosphor and the green emitting rare-earth phosphor, with the gamuts compressed in the vertical direction and the chroma of blue declining. This results from low excitation purity of the blue spectrum and declining color separation, This is because the spectrum added to the emission center having the emission peak in the range of 480 [nm] to 520 [nm] in the blue green region is broad, and accordingly, a valley-shaped spectrum is unlikely to occur between the spectrum pertaining to the blue emission center and the spectrum pertaining to the blue green emission center.

Yet, the tendencies correspond with those of the simulations of the embodiments in which a phosphor containing an element whose main emission peak is located in the range of 480 [nm] to 520 [nm] between the main emission peaks of the blue emitting rare-earth phosphor and the green emitting rare-earth phosphor. To be strict, the addition in the narrow band and the addition in the broad band cause differences, but the effects are broadly maintained in the scope of the present invention.

(Seventh Embodiment)

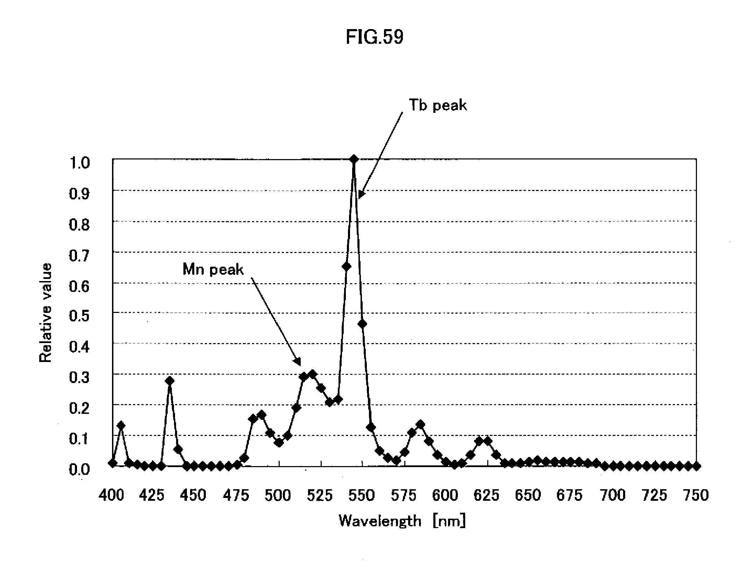

FIG. 59 shows spectral distributions of a CAT:Mn phosphor used in a seventh embodiment of the present invention. The present embodiment uses the following combination of phosphors: the red emitting rare-earth phosphor YOX containing a rare-earth element as an emission center whose main emission peak is located in the range of 605 [nm] to 625 [nm]; the blue emitting rare-earth phosphor BAM containing a rare-earth element as an emission center whose main emission peak is located in the range of 440 [nm] to 460 [nm]; the green emitting rare-earth phosphor Tb containing a rare-earth element as an emission center whose main emission peak is located in the range of 540 [nm] to 550 [nm]; and the phosphor CAT:Mn containing an element Mn as an emission center whose main emission peak is located in the range of 480 [nm] to 520 [nm].

As in the case of using the BAM:Mn phosphor, the present embodiment includes a narrow-band emission of 50 [nm] or greater in the range of 480 [nm] to 520 [nm] between the main emission peaks of the blue emitting rare-earth phosphor and the green emitting rare-earth element, and accordingly, the emission can be concentrated in a targeted emission band efficiently.

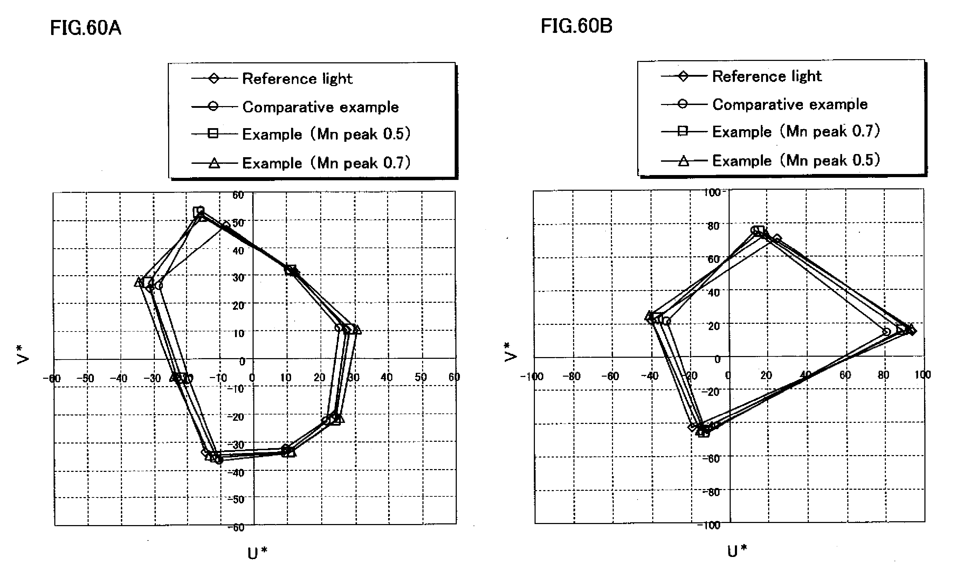

The present embodiment has a means to suppress melatonin secretion while realizing preferable color rendering properties, as in the other embodiments. FIG. 60 shows these tendencies of the present embodiment in detail, using cases where the correlated color temperature is 12000 [K] and Duv = 0. The distribution tendencies of the color gamuts are consistent with the simulations of the third embodiment; the gamuts extend in the red-green directions.

Note that in the cases shown in the figure, the ratio (Mn peak/Eu peak) of the emission peak of Mn, which is the emission center of CAT:Mn, to the emission peak of Eu, which is the emission center included in BAM, is 0.5 and 0.7, respectively within the spectral distributions of the entire lamp.

In the present embodiment, the phosphor CAT:Mn which contains Tb and Mn both as the emission centers causes the emission from the emission center Mn having the main emission peak in the range of 480 [nm] to 520 [nm], thereby realizing the same effects as the first embodiment.

(Eighth Embodiment)

The present embodiment explains the present invention as a lighting apparatus. The present embodiment is realized not by a single fluorescent lamp but by a lighting apparatus that blends lighting from fluorescent lamps that each include combinations of the phosphors of the present invention.

The advantageous effects of the present invention as the lighting apparatus are achieved by mixing light of the following lamps: a fluorescent lamp having a red emitting rare-earth phosphor containing a rare-earth element as an emission center whose emission peak is located in the range of 605 [nm] to 625 [nm]; a fluorescent lamp having a green emitting rare-earth phosphor containing a rare-earth element as an emission center whose emission peak is located in the range of 540 [nm] to 550 [nm]; a fluorescent lamp having a blue emitting rare-earth phosphor containing a rare-earth element as an emission center whose emission peak is located in the range of 440 [nm] to 460 [nm]; and a fluorescent lamp having a phosphor containing an element as an emission center whose emission peak is located in the range of 480 [nm] to 520 [nm] between the main emission peaks of the blue emitting rare-earth phosphor and the green emitting rare-earth phosphor. In the structure mentioned above, GB is added to a conventional RGB light mixing lighting apparatus.

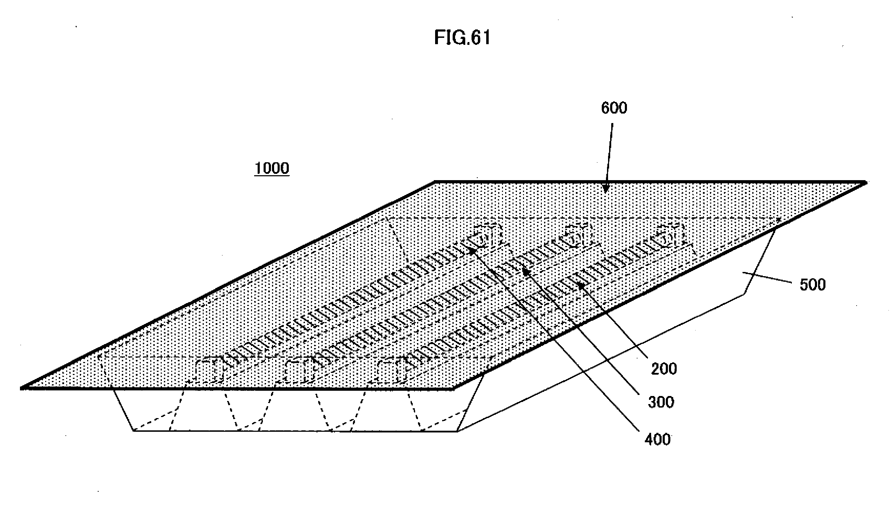

Alternatively, the lighting apparatus of the present invention can be structured using three kinds of lamps. FIG. 61 schematically shows a lighting apparatus 1000 of the embodiment pertaining to the present invention. The lighting apparatus 1000 of the present embodiment includes a device 500 accommodating three kinds of lamps 200, 300, and 400 whose light colors are different from one another, and a diffusion transmission plate 600. The lighting apparatus 1000 emits white light by simultaneously turns on the lamps 200, 300, and 400 in the device 500 and mixing the three colors using the diffusion transmission plate 600. The fluorescent lamp 200 having a relatively low color temperature is constituted from at least a red emitting rare-earth phosphor containing a rare-earth element as an emission center whose emission peak is located in the range of 605 [nm] to 625 [nm] and a green emitting rare-earth phosphor containing a rare-earth element as an emission center whose emission peak is located in the range of 540 [nm] to 550 [nm]. The fluorescent lamp 300 having a relatively low color temperature is constituted from at least a blue emitting rare-earth phosphor containing a rare-earth element as an emission center whose emission peak is located in the range of 440 [nm] to 460 [nm] and a green emitting rare-earth phosphor containing a rare-earth element as an emission center whose emission peak is located in the range of 540 [nm] to 550 [nm]. The fluorescent lamp 400 is constituted from at least a phosphor containing an element as an emission center whose main emission peak is located in the range of 480 [nm] to 520 [nm] between the main emission peaks of the blue emitting rare-earth phosphor and the green emitting rare-earth phosphor.

The advantageous effects of the present invention as a lighting apparatus are also achieved by the mixing lighting structured as above.

As a further alternative, mixing light using the following lamps can also achieve the advantageous effects of the present invention as a lighting apparatus: a fluorescent lamp including at least a red emitting rare-earth phosphor containing a rare-earth element as an emission center whose emission peak is located in the range of 605 [nm] to 625 [nm], a green emitting rare-earth phosphor containing a rare-earth element as an emission center whose emission peak is located in the range of 540 [nm] to 550 [nm], and a blue emitting rare-earth phosphor containing a rare-earth element as an emission center whose emission peak is located in the range of 440 [nm] to 460 [nm]; and a fluorescent lamp including at least an element as an emission enter whose main emission peak is located in the range of 480 [nm] to 520 [nm] between the main emission peaks of the blue emitting rare-earth phosphor and the green emitting rare-earth phosphor.

It should be noted that in the present embodiment, various lamps can be combined, and modulating each lamp enables varying color and adjusting the melatonin suppression effect.

In the present invention, the BAM phosphor is Eu-activated barium magnesium aluminate.
A typical composition is $BaMgAl_{10}O_{17}$:Eu.
An alternative composition is $BaMg_2Al_{16}O_{27}$:Eu, $BaMg_3Al_{14}O_{25}$:Eu, or $(Ba, Sr)MgAl_{10}O_{17}$:Eu.
A further alternative composition is any of the above compositions with part thereof replaced (typically, Sr, Eu or the like).

In the present invention, the BAM:Mn phosphor is Eu, Mn-coactivated barium magnesium aluminate.
A typical composition is $BaMgAl_{10}O_{17}$:Eu,Mn.
An alternative composition is $BaMg_2Al_{16}O_{27}$:Eu,Mn, $BaMg_3Al_{14}O_{25}$:Eu,Mn, or $(Ba, Sr)MgAl_{10}O_{17}$:Eu,Mn.
A further alternative composition is any of the above compositions with part thereof replaced (typically, Sr, Eu or the like).

In the present invention, the SCA phosphor is Eu-activated barium calcium strontium magnesium chlorapatite.
A typical composition is $Sr_{10}(PO_4)_6Cl_2$:Eu.
An alternative composition is $(Sr,Ca)_{10}(PO_4)_6Cl_2$:Eu, $(Sr,Ca)_{10}(PO_4)_6Cl_2 \cdot nB_2O_3$:Eu, $(Ba,Ca,Mg)_{10}(PO_4)_6Cl_2$:Eu, or $(Sr, Ba,Ca)_{10}(PO_4)_6Cl_2$:Eu.
A further alternative composition is any of the above compositions with part thereof replaced (typically, Ba).

In the present invention, the SAE phosphor is Eu-activated strontium aluminate.
A typical composition is $Sr_4Al_{15}O_{25}$:Eu.
An alternative composition is $4SrO \cdot 7Al_2O_3$:Eu.
A further alternative is any of the composition above with part thereof replaced.

In the present invention, the BCA phosphor is Eu-activated barium calcium magnesium chlorapatite.
A typical composition is $(Ba,Ca)_5(PO_4)_3Cl$:Eu.
An alternative is the composition above with part thereof replaced (typically, Sr, Mg or the like).
In $(Ba,Ca,X)_5(PO_4)_3Cl$:$Eu^{2+}$, X can be described as including (i) one of Sr and Mg, (ii) both of Sr and Mg, or (iii) neither of Sr and Mg.

In the present invention, the CMZ phosphor is Mn-activated cerium magnesium zinc aluminate.
A typical composition is $Ce(Mg,Zn)Al_{11}O_{19}$:Mn.
An alternative is the composition above with part thereof replaced.

In the present invention, the LAP phosphor is Ce, Tb-coactivated lanthan phosphate.
A typical composition is $LaPO_4$:Ce, Tb.
An alternative is the composition above with part thereof replaced (typically, $SiO_2$).

In the present invention, the CAT phosphor is Tb-activated cerium magnesium aluminate.
A typical composition is $CeMgAl_{11}O_{19}$:Tb.
An alternative is the composition above with part thereof replaced.

In the present invention, the CBT phosphor is Ce, Tb-coactivated gadolinium magnesium borate.
A typical composition is $GdMgB_5O_{10}$:Ce,Tb.
An alternative is the composition above with part thereof replaced.

In the present invention, the CAT:Mn phosphor is Tb, Mn-coactivated cerium magnesium aluminate.
A typical composition is $CeMgAl_{11}O_{19}$:Tb,Mn.
An alternative is the composition above with part thereof replaced.

In the present invention, the YOX phosphor is Eu-activated yttrium oxide.
A typical composition is $Y_2O_3$:Eu.
An alternative is the composition above with part thereof replaced.

In the present invention, the YOS phosphor is Eu-activated yttrium oxysulfide.
A typical composition is $Y_2O_2S$:Eu.
An alternative is the composition above with part thereof replaced.

In the present invention, the YVO phosphor is Eu-activated yttrium vanadate.
A typical composition is $YVO_4$:Eu.
An alternative composition is $Y(P,V)O_4$:Eu (Eu-activated yttrium phosphate vanadate).
A further alternative composition is any of the above compositions with part thereof replaced (typically, Bi).

In each of the embodiments above, the spectral distributions of a specific fluorescent lamp are indicated.

However, unless particularly specified, expressions pertaining to structural components such as "have" do not exclude components or steps other than those indicated in the present Description including Claims. An optional addition of a component such as a protective layer or amalgam, or an embodiment other than the hot cathode fluorescent lamp, such as a cold cathode fluorescent lamp, a dielectric barrier fluorescent lamp or the like would be easily inferred by one skilled in the art. Also, a component expressed by use of a singular form does not exclude, from Claims, an equivalent effect of the component in a plurality.

In the definition of various measurements, the characteristics of ordinary fluorescent lamps are indicated in rated lighting at 25 [°C] after 100-hour aging. The present invention can be measured in conformity of the above. For the correlated color temperature and Duv, it may be more realistic to use typical values during a stable practical period of the lamp life. Additionally, because spectral values of various color rendering calculations are given in 5 [nm] increments, the spectral ranges of the present invention may have errors of 5 [nm] or smaller.

Some of the embodiments in the present Description include structures recited in different dependent claims, and as understood from this fact, one skilled in the art would easily infer to use combinations of these structures. Although the present invention has been described by way of Description, various changes and modifications may be made to the fluorescent lamp and the lighting apparatus using the fluorescent lamp without departing from the scope of the present invention, and the changes and the modifications are construed as being included in the scope of claims.

**[Industrial Applicability]**

The fluorescent lamp and lighting apparatus of the present invention have a light color in a high color temperature region, and provide photostimulation related to melatonin secretion and suppression of living bodies while improving, as illumination light, the color rendition of the illuminated objects. Thus, the fluorescent lamp and lighting apparatus of the present invention are not only effective as general lighting such as school lighting, office lighting, hospital lighting, or stand light but also effective as illumination light sources of phototherapeutic apparatus.

Additionally, the characterstics of the light color and color rendition of the present invention enable the white point of images to have similar characteristics to those of an ordinary display monitor. Accordingly, use of the present invention for illuminating signs, advertisements and the like will highlight whiteness, and thus allows the illuminated objects to appear vivid, and further, as a backlight, allow transparent light colors to appear vivid as well.

Moreover, indicating a light color and color reproducibility which are close to clear-sky light, the present invention is also suitable for use as quasi-skylight, and for use that requires high color rendition. In addition, being capable of highlighting whiteness and rendering colors to appear vivid, the present invention is also effective for store lighting.

**Claims**

1.  A fluorescent lamp having a light color with a correlated color temperature higher than 10000 [K] and lower than 17000 [K] and Duv in a range of -2.5 to 5, inclusive, the fluorescent lamp comprising:

    an arc tube; and
    a phosphor layer formed on an inner surface of the arc tube, wherein
    the phosphor layer includes at least:

    a red emitting rare-earth phosphor containing a rare-earth element as a light emission center with a main emission peak located in a range of 605 [nm] to 625 [nm], inclusive;
    a green emitting rare-earth phosphor containing a rare-earth element as a light emission center with a main emission peak located in a range of 540 [nm] to 550 [nm], inclusive; and
    a blue emitting rare-earth phosphor containing a rare-earth element as a light emission center with a main emission peak located in a range of 440 [nm] to 460 [nm], inclusive,

    an action function efficiency of melatonin suppression for per unit luminous flux is higher than 1.0, and
    a general color rendering index Ra is 80 or higher.

2.  A fluorescent lamp having a light color with a correlated color temperature higher than 7100 [K] and Duv in a range of -2.5 to 5, inclusive, the fluorescent lamp comprising:

    an arc tube; and
    a phosphor layer formed on an inner surface of the arc tube, wherein
    the phosphor layer includes at least:

a red emitting rare-earth phosphor containing a rare-earth element as a light emission center with a main emission peak located in a range of 605 [nm] to 625 [nm], inclusive;
a green emitting rare-earth phosphor containing a rare-earth element as a light emission center with a main emission peak located in a range of 540 [nm] to 550 [nm], inclusive;
a blue emitting rare-earth phosphor containing a rare-earth element as a light emission center with a main emission peak located in a range of 440 [nm] to 460 [nm] inclusive; and
a phosphor containing an element as a light emission center with a main emission peak located in a range of 480 [nm] to 520 [nm], inclusive, between the main emission peak of the blue emitting rare-earth phosphor and the main emission peak of the green emitting rare-earth phosphor, wherein

the phosphor containing the element as the light emission center with the main emission peak located in the range of 480 [nm] to 520 [nm], inclusive, photostimulates melanopsin by enhancing emission between the main emission peak of the blue emitting rare-earth phosphor and the main emission peak of the green emitting rare-earth phosphor, thereby increasing a melatonin suppression efficiency,
a general color rendering index Ra is 80 or higher, and
a special color rendering index R9 is 50 or higher.

3. A fluorescent lamp having a light color with a correlated color temperature higher than 7100 [K] and Duv in a range of -2.5 to 5, inclusive, the fluorescent lamp comprising:

an arc tube; and
a phosphor layer formed on an inner surface of the arc tube, wherein
the phosphor layer includes at least:

a red emitting rare-earth phosphor containing a rare-earth element as a light emission center with a main emission peak located in a range of 605 [nm] to 625 [nm], inclusive;
a green emitting rare-earth phosphor containing a rare-earth element as a light emission center with a main emission peak located in a range of 540 [nm] to 550 [nm], inclusive; and
a blue emitting rare-earth phosphor containing a rare-earth element as a light emission center with a main emission peak located in a range of 440 [nm] to 460 [nm] inclusive, wherein

the blue emitting rare-earth phosphor containing the rare-earth element as the light emission center with the main emission peak located in the range of 480 [nm] to 520 [nm], inclusive, has a half width of 50 [nm] or greater from an emission peak toward a longer wavelength and photostimulates melanopsin by enhancing emission between the main emission peak of the blue emitting rare-earth phosphor and the main emission peak of the green emitting rare-earth phosphor, thereby increasing a melatonin suppression efficiency,
a general color rendering index Ra is 80 or higher, and
a special color rendering index R9 is 50 or higher.

4. The fluorescent lamp according to any of Claims 2 and 3, wherein
the correlated color temperature is in a range of 10000 [K] to 20000 [K], inclusive.

5. The fluorescent lamp according to any of Claims 1 to 3, wherein
the correlated color temperature is in a range of 11000 [K] to 13000 [K], inclusive.

6. The fluorescent lamp according to any of Claims 1 to 3, wherein
Duv is greater than 0.

7. The fluorescent lamp of Claim 3, wherein
at least one of the phosphors is composed of SCA.

8. The fluorescent lamp of Claim 2, wherein
the blue emitting rare-earth phosphor containing the rare-earth element as the light emission center with the main emission peak located in the range of 440 [nm] to 460 [nm], inclusive, is composed of one or more of BAM:Mn, BAM and SCA, and
the blue emitting rare-earth phosphor containing the rare-earth element as the light emission center with the main emission peak located in the range of 480 [nm] to 520 [nm], inclusive, is composed of one or more of BAM:Mn, BAM, SAE, CMZ, and BCA.

9. The fluorescent lamp of Claim 2, wherein
the blue emitting rare-earth phosphor containing the rare-earth element as the light emission center with the main emission peak located in the range of 440 [nm] to 460 [nm], inclusive, and the phosphor containing the element as the light emission center with the main emission peak located in the range of 480 [nm] to 520 [nm], inclusive, are each composed of a BAM:Mn phosphor.

10. The fluorescent lamp of Claim 2, wherein
the phosphor containing the element as the light emission center with the main emission peak located in the range of 480 [nm] to 520 [nm], inclusive, and the green emitting rare-earth phosphor containing the rare-earth element as the light emission center with the main emission peak located in the range of 540 [nm] to 550 [nm], inclusive, are each composed of a CAT:Mn phosphor.

11. The fluorescent lamp of Claim 2, wherein
a ratio of an emission intensity of the main emission peak located in the range of 480 [nm] to 520 [nm] to an emission intensity of the main emission peak located in the range of 440 [nm] to 460 [nm] is in a range of 0.5 to 1.0, inclusive.

12. The fluorescent lamp according to any of Claims 1 to 3, wherein
the red emitting rare-earth phosphor containing the rare-earth element as the light emission center with the main emission peak located in the range of 605 [nm] to 625 [nm], inclusive, is composed of one or a plurality of YOX, YVO, and YOS,
the green emitting rare-earth phosphor containing the rare-earth element as the light emission center with the main emission peak located in the range of 540 [nm] to 550 [nm], inclusive, is composed of one or a plurality of LAP, CAT, CAT:Mn, and CBT,
the phosphor containing the element as the light emission center with the main emission peak located in the range of 480 [nm] to 520 [nm], inclusive, is composed of one or a plurality of BAM:Mn, SAE, CMZ, BCA, CAT:Mn, and the blue emitting rare-earth phosphor containing the rare-earth element as the light emission center with the main emission peak located in the range of 440 [nm] to 460 [nm], inclusive, is composed of one or a plurality of BAM:Mn, BAM, and SCA.

13. A lighting apparatus using the fluorescent lamp according to any of Claims 1 to 3.

14. A lighting apparatus which forms a spectral distribution of the fluorescent lamp according to any of Claims 1 to 3 by blending light of a plurality of fluorescent lamps that each have one of phosphor layers, each of the phosphor layers being made of one of phosphors that have mutually different colors.

FIG. 1

EP 2 281 606 A1

FIG. 2

## FIG. 3

◆ Narrow-band three-wavelength fluorescent lamp
■ Broad-band fluorescent lamp (general type)
▲ Broad-band fluorescent lamp (high color rendition type)
● CIE reconstituted daylight

◇ Narrow-band three-wavelength fluorescent lamp
△ Narrow-band three-wavelength, blue green enhanced
   (blue:blue green peak ratio = 1:0.7) fluorescent lamp
□ Narrow-band three-wavelength, blue green enhanced
   (blue:blue green peak ratio = 1:1.3) fluorescent lamp

EP 2 281 606 A1

## FIG. 4

◆ Narrow-band three-wavelength fluorescent lamp
■ Broad-band fluorescent lamp (general type)
▲ Broad-band fluorescent lamp (high color rendition type)
● CIE reconstituted daylight

◇ Narrow-band three-wavelength fluorescent lamp
△ Narrow-band three-wavelength, blue green enhanced (blue:blue green peak ratio = 1:0.7) fluorescent lamp
□ Narrow-band three-wavelength, blue green enhanced (blue:blue green peak ratio = 1:1.3) fluorescent lamp

FIG. 5

◆ Broad-band (halogen lamp, high color rendition)
■ Black body radiation
▲ CIE reconstituted daylight

◇ Narrow-band three-wavelength fluorescent lamp
○ Narrow-band three-wavelength, blue green enhanced
  (blue:blue green peak ratio = 1:0.7) fluorescent lamp
△ Narrow-band three-wavelength, blue green enhanced
  (blue:blue green peak ratio = 1:1.3) fluorescent lamp

EP 2 281 606 A1

FIG. 6

| Tc | Duv | Color rendering indexes | | | | | | | | | | | | Action power for per unit luminous flux | | | | Efficiency [lm/W] |
|---|---|---|---|---|---|---|---|---|---|---|---|---|---|---|---|---|---|---|
| | | Ga4 | Ga | M | PS | Ra | R9 | R10 | R11 | R12 | R13 | R14 | R15 | Br | Th | c(λ) | z(λ) | |
| 7100 | -2.5 | 93 | 101 | 93 | 78 | 84 | 44 | 50 | 70 | 62 | 97 | 70 | 97 | 0.95 | 0.94 | 0.95 | 0.69 | 81.7 |
| 7100 | 0 | 91 | 99 | 92 | 76 | 83 | 37 | 47 | 68 | 60 | 95 | 71 | 98 | 0.93 | 0.92 | 0.93 | 0.68 | 82.6 |
| 7100 | 2.5 | 90 | 97 | 90 | 72 | 81 | 30 | 44 | 64 | 58 | 92 | 71 | 95 | 0.92 | 0.91 | 0.92 | 0.66 | 83.6 |
| 7100 | 5 | 88 | 95 | 88 | 69 | 79 | 22 | 41 | 61 | 55 | 89 | 71 | 91 | 0.90 | 0.89 | 0.90 | 0.65 | 84.6 |
| 8000 | -2.5 | 93 | 101 | 91 | 76 | 85 | 48 | 50 | 70 | 63 | 97 | 70 | 97 | 1.01 | 1.00 | 1.01 | 0.75 | 80.2 |
| 8000 | 0 | 91 | 99 | 90 | 72 | 83 | 40 | 47 | 67 | 60 | 94 | 71 | 98 | 1.00 | 0.99 | 1.00 | 0.74 | 81.1 |
| 8000 | 2.5 | 89 | 96 | 88 | 68 | 81 | 31 | 43 | 63 | 58 | 91 | 71 | 94 | 0.99 | 0.98 | 0.99 | 0.72 | 82.0 |
| 8000 | 5 | 88 | 94 | 86 | 65 | 79 | 22 | 40 | 60 | 55 | 88 | 71 | 90 | 0.97 | 0.96 | 0.98 | 0.71 | 82.9 |
| 10000 | -2.5 | 93 | 100 | 88 | 71 | 84 | 51 | 49 | 69 | 62 | 97 | 71 | 97 | 1.12 | 1.12 | 1.13 | 0.85 | 77.6 |
| 10000 | 0 | 91 | 97 | 86 | 67 | 82 | 42 | 46 | 66 | 60 | 93 | 71 | 97 | 1.11 | 1.11 | 1.12 | 0.84 | 78.5 |
| 10000 | 2.5 | 89 | 95 | 84 | 63 | 81 | 32 | 43 | 62 | 57 | 90 | 71 | 93 | 1.10 | 1.10 | 1.11 | 0.83 | 79.3 |
| 10000 | 5 | 87 | 92 | 81 | 58 | 78 | 22 | 39 | 58 | 55 | 86 | 72 | 89 | 1.09 | 1.09 | 1.10 | 0.82 | 80.1 |
| 11000 | -2.5 | 93 | 100 | 86 | 69 | 84 | 52 | 49 | 69 | 62 | 96 | 71 | 97 | 1.16 | 1.16 | 1.18 | 0.89 | 76.7 |
| 11000 | 0 | 91 | 97 | 84 | 65 | 82 | 42 | 46 | 65 | 59 | 93 | 71 | 96 | 1.16 | 1.15 | 1.17 | 0.88 | 77.5 |
| 11000 | 2.5 | 89 | 94 | 82 | 61 | 80 | 32 | 42 | 62 | 57 | 89 | 72 | 92 | 1.15 | 1.14 | 1.16 | 0.87 | 78.3 |
| 11000 | 5 | 86 | 91 | 80 | 56 | 78 | 22 | 39 | 58 | 55 | 86 | 72 | 88 | 1.14 | 1.13 | 1.15 | 0.86 | 79.1 |
| 12000 | -2.5 | 93 | 99 | 85 | 68 | 84 | 53 | 49 | 68 | 62 | 96 | 71 | 96 | 1.20 | 1.20 | 1.22 | 0.92 | 75.9 |
| 12000 | 0 | 91 | 96 | 83 | 64 | 82 | 42 | 46 | 65 | 59 | 93 | 71 | 96 | 1.19 | 1.19 | 1.21 | 0.91 | 76.6 |
| 12000 | 2.5 | 88 | 94 | 81 | 59 | 80 | 32 | 42 | 61 | 57 | 89 | 72 | 92 | 1.18 | 1.18 | 1.20 | 0.90 | 77.4 |
| 12000 | 5 | 86 | 91 | 79 | 54 | 78 | 21 | 39 | 57 | 55 | 85 | 72 | 87 | 1.18 | 1.17 | 1.19 | 0.89 | 78.2 |
| 12000 | 10 | 81 | 85 | 74 | 44 | 74 | -1 | 32 | 50 | 50 | 78 | 73 | 78 | 1.16 | 1.15 | 1.17 | 0.87 | 79.8 |
| 13000 | -2.5 | 93 | 99 | 85 | 67 | 84 | 53 | 49 | 68 | 61 | 96 | 71 | 96 | 1.23 | 1.23 | 1.25 | 0.95 | 75.2 |
| 13000 | 0 | 91 | 96 | 83 | 63 | 82 | 43 | 46 | 65 | 59 | 93 | 71 | 95 | 1.23 | 1.24 | 1.25 | 0.95 | 75.9 |
| 13000 | 2.5 | 88 | 93 | 80 | 57 | 80 | 31 | 42 | 61 | 57 | 89 | 72 | 91 | 1.22 | 1.21 | 1.23 | 0.93 | 76.7 |
| 13000 | 5 | 86 | 90 | 78 | 52 | 78 | 20 | 39 | 57 | 55 | 85 | 72 | 87 | 1.21 | 1.20 | 1.22 | 0.92 | 77.5 |
| 17000 | -2.5 | 93 | 98 | 82 | 64 | 84 | 53 | 50 | 67 | 61 | 96 | 71 | 96 | 1.32 | 1.32 | 1.34 | 1.03 | 73.3 |
| 17000 | 0 | 90 | 95 | 80 | 59 | 82 | 42 | 46 | 63 | 59 | 92 | 72 | 94 | 1.31 | 1.31 | 1.33 | 1.02 | 74.0 |
| 17000 | 2.5 | 87 | 92 | 77 | 54 | 80 | 30 | 43 | 59 | 57 | 88 | 72 | 90 | 1.31 | 1.31 | 1.33 | 1.01 | 74.7 |
| 17000 | 5 | 85 | 89 | 75 | 48 | 78 | 18 | 39 | 55 | 55 | 84 | 72 | 85 | 1.30 | 1.30 | 1.32 | 1.00 | 75.4 |
| 20000 | -2.5 | 92 | 98 | 81 | 63 | 84 | 53 | 50 | 66 | 62 | 95 | 72 | 95 | 1.36 | 1.37 | 1.39 | 1.07 | 72.3 |
| 20000 | 0 | 90 | 94 | 79 | 57 | 82 | 41 | 46 | 63 | 60 | 91 | 72 | 93 | 1.36 | 1.36 | 1.38 | 1.06 | 73.0 |
| 20000 | 2.5 | 87 | 91 | 76 | 52 | 80 | 29 | 43 | 59 | 58 | 88 | 72 | 89 | 1.35 | 1.35 | 1.37 | 1.05 | 73.7 |
| 20000 | 5 | 84 | 88 | 74 | 46 | 78 | 17 | 39 | 55 | 55 | 84 | 73 | 85 | 1.34 | 1.34 | 1.36 | 1.04 | 74.4 |

EP 2 281 606 A1

FIG. 7

| Tc | Duv | Color rendering indexes | | | | | | | | | | | | Action power for per unit luminous flux | | | | Efficiency [lm/W] |
|---|---|---|---|---|---|---|---|---|---|---|---|---|---|---|---|---|---|---|
| | | Ga4 | Ga | M | PS | Ra | R9 | R10 | R11 | R12 | R13 | R14 | R15 | Br | Th | c($\lambda$) | z($\lambda$) | |
| 7100 | -2.5 | 101 | 112 | 105 | 87 | 88 | 81 | 82 | 86 | 88 | 85 | 78 | 82 | 0.99 | 0.97 | 0.98 | 0.69 | 80.8 |
| 7100 | 0 | 99 | 110 | 103 | 85 | 89 | 74 | 78 | 89 | 84 | 88 | 78 | 86 | 0.98 | 0.96 | 0.97 | 0.68 | 81.7 |
| 7100 | 2.5 | 98 | 107 | 101 | 82 | 89 | 66 | 73 | 90 | 80 | 91 | 78 | 90 | 0.96 | 0.94 | 0.95 | 0.66 | 82.7 |
| 7100 | 5 | 96 | 105 | 99 | 79 | 89 | 58 | 69 | 89 | 77 | 94 | 78 | 94 | 0.95 | 0.93 | 0.94 | 0.65 | 83.7 |
| 8000 | -2.5 | 103 | 113 | 104 | 86 | 88 | 87 | 85 | 84 | 89 | 84 | 79 | 81 | 1.06 | 1.05 | 1.06 | 0.75 | 79.2 |
| 8000 | 0 | 101 | 111 | 102 | 84 | 89 | 81 | 80 | 87 | 85 | 87 | 79 | 85 | 1.05 | 1.03 | 1.04 | 0.74 | 80.1 |
| 8000 | 2.5 | 99 | 108 | 100 | 81 | 90 | 73 | 76 | 89 | 82 | 90 | 79 | 89 | 1.04 | 1.02 | 1.03 | 0.72 | 81.0 |
| 8000 | 5 | 97 | 105 | 98 | 77 | 90 | 65 | 71 | 89 | 78 | 93 | 79 | 93 | 1.03 | 1.01 | 1.02 | 0.71 | 82.0 |
| 10000 | -2.5 | 105 | 115 | 102 | 85 | 87 | 89 | 90 | 79 | 90 | 82 | 81 | 78 | 1.19 | 1.17 | 1.18 | 0.85 | 76.6 |
| 10000 | 0 | 103 | 112 | 100 | 82 | 89 | 90 | 85 | 83 | 87 | 85 | 81 | 82 | 1.18 | 1.16 | 1.17 | 0.84 | 77.4 |
| 10000 | 2.5 | 101 | 109 | 98 | 79 | 90 | 83 | 81 | 86 | 84 | 89 | 81 | 87 | 1.17 | 1.15 | 1.16 | 0.83 | 78.2 |
| 10000 | 5 | 99 | 106 | 96 | 75 | 90 | 74 | 76 | 87 | 81 | 91 | 81 | 91 | 1.16 | 1.14 | 1.15 | 0.82 | 79.1 |
| 11000 | -2.5 | 106 | 115 | 102 | 85 | 86 | 87 | 92 | 78 | 91 | 81 | 81 | 77 | 1.23 | 1.22 | 1.23 | 0.89 | 75.6 |
| 11000 | 0 | 104 | 112 | 100 | 82 | 89 | 91 | 87 | 82 | 88 | 85 | 81 | 82 | 1.22 | 1.21 | 1.22 | 0.88 | 76.4 |
| 11000 | 2.5 | 102 | 109 | 97 | 78 | 90 | 86 | 83 | 85 | 85 | 88 | 82 | 86 | 1.21 | 1.20 | 1.21 | 0.87 | 77.2 |
| 11000 | 5 | 99 | 106 | 95 | 74 | 91 | 78 | 78 | 87 | 81 | 91 | 82 | 91 | 1.20 | 1.19 | 1.20 | 0.86 | 78.0 |
| 12000 | -2.5 | 107 | 116 | 101 | 85 | 86 | 84 | 93 | 76 | 91 | 80 | 82 | 76 | 1.27 | 1.26 | 1.27 | 0.92 | 74.8 |
| 12000 | 0 | 105 | 113 | 99 | 82 | 88 | 91 | 89 | 80 | 88 | 84 | 82 | 81 | 1.26 | 1.25 | 1.26 | 0.91 | 75.5 |
| 12000 | 2.5 | 102 | 110 | 97 | 78 | 90 | 88 | 84 | 84 | 85 | 87 | 82 | 85 | 1.25 | 1.24 | 1.25 | 0.90 | 76.3 |
| 12000 | 5 | 100 | 107 | 95 | 74 | 91 | 80 | 80 | 86 | 82 | 90 | 82 | 90 | 1.24 | 1.23 | 1.24 | 0.89 | 77.1 |
| 13000 | -2.5 | 107 | 116 | 101 | 85 | 86 | 82 | 95 | 75 | 91 | 80 | 82 | 76 | 1.31 | 1.29 | 1.31 | 0.95 | 74.1 |
| 13000 | 0 | 105 | 113 | 99 | 81 | 88 | 90 | 91 | 79 | 89 | 83 | 83 | 80 | 1.30 | 1.28 | 1.30 | 0.94 | 74.8 |
| 13000 | 2.5 | 103 | 110 | 96 | 77 | 90 | 90 | 86 | 83 | 86 | 87 | 83 | 85 | 1.29 | 1.27 | 1.29 | 0.93 | 75.6 |
| 13000 | 5 | 100 | 107 | 94 | 73 | 91 | 82 | 81 | 85 | 83 | 90 | 83 | 90 | 1.28 | 1.26 | 1.28 | 0.92 | 76.3 |
| 17000 | -2.5 | 109 | 117 | 100 | 85 | 85 | 75 | 97 | 72 | 92 | 78 | 84 | 74 | 1.40 | 1.39 | 1.41 | 1.03 | 72.1 |
| 17000 | 0 | 107 | 114 | 98 | 81 | 88 | 85 | 95 | 76 | 90 | 82 | 84 | 79 | 1.39 | 1.38 | 1.40 | 1.02 | 72.8 |
| 17000 | 2.5 | 104 | 111 | 95 | 77 | 90 | 92 | 90 | 80 | 87 | 86 | 85 | 83 | 1.39 | 1.37 | 1.39 | 1.01 | 73.5 |
| 17000 | 5 | 101 | 107 | 93 | 72 | 91 | 87 | 85 | 83 | 84 | 89 | 85 | 88 | 1.38 | 1.36 | 1.38 | 1.00 | 74.2 |
| 20000 | -2.5 | 110 | 118 | 100 | 85 | 85 | 72 | 96 | 71 | 93 | 77 | 85 | 73 | 1.45 | 1.44 | 1.45 | 1.07 | 71.2 |
| 20000 | 0 | 107 | 114 | 97 | 81 | 88 | 82 | 97 | 75 | 91 | 81 | 85 | 78 | 1.44 | 1.43 | 1.45 | 1.06 | 71.8 |
| 20000 | 2.5 | 105 | 111 | 95 | 77 | 90 | 91 | 93 | 79 | 88 | 85 | 85 | 83 | 1.43 | 1.42 | 1.44 | 1.05 | 72.5 |
| 20000 | 5 | 102 | 108 | 93 | 72 | 92 | 90 | 88 | 81 | 85 | 88 | 86 | 87 | 1.43 | 1.41 | 1.43 | 1.04 | 73.2 |

FIG. 8A

| | |
|---|---|
| ◇ | Reference light |
| ○ | Comparative example |
| △ | Example |

V*

U*

FIG. 8B

| | |
|---|---|
| ◇ | Reference light |
| ○ | Comparative example |
| △ | Example |

V*

U*

EP 2 281 606 A1

FIG. 9

FIG. 10A

FIG. 10B

FIG. 11

EP 2 281 606 A1

FIG. 12B

FIG. 12A

FIG. 13

(BAM, 12000K, Duv=0)

EP 2 281 606 A1

FIG. 14B

FIG. 14A

FIG. 15

EP 2 281 606 A1

# FIG. 16

● Example, Duv=-2.5     ○ Comparative example, Duv=-2.5
▲ Example, Duv=0        △ Comparative example, Duv=0
■ Example, Duv=5        □ Comparative example, Duv=5

EP 2 281 606 A1

FIG. 17

| Tc | duv | Color rendering indexes | | | | | | | | | | | | Action power for per unit luminous flux | | | | Efficiency [lm/W] |
|---|---|---|---|---|---|---|---|---|---|---|---|---|---|---|---|---|---|---|
| | | Ga4 | Ga | M | PS | Ra | R9 | R10 | R11 | R12 | R13 | R14 | R15 | Br | Th | c(λ) | z(λ) | |
| 7100 | -2.5 | 99 | 108 | 101 | 87 | 90 | 72 | 77 | 94 | 82 | 88 | 77 | 86 | 0.98 | 0.96 | 0.98 | 0.69 | 79.0 |
| 7100 | 0 | 97 | 106 | 99 | 84 | 91 | 65 | 72 | 91 | 79 | 91 | 77 | 90 | 0.97 | 0.95 | 0.97 | 0.68 | 80.0 |
| 7100 | 2.5 | 95 | 104 | 97 | 81 | 90 | 57 | 68 | 87 | 75 | 94 | 77 | 94 | 0.96 | 0.93 | 0.95 | 0.66 | 80.9 |
| 7100 | 5 | 94 | 101 | 95 | 78 | 89 | 50 | 64 | 83 | 72 | 95 | 78 | 98 | 0.94 | 0.92 | 0.94 | 0.65 | 81.9 |
| 8000 | -2.5 | 100 | 109 | 100 | 86 | 90 | 78 | 79 | 95 | 82 | 88 | 78 | 85 | 1.06 | 1.03 | 1.06 | 0.75 | 77.3 |
| 8000 | 0 | 98 | 106 | 98 | 83 | 91 | 71 | 75 | 92 | 79 | 91 | 78 | 89 | 1.04 | 1.02 | 1.04 | 0.74 | 78.2 |
| 8000 | 2.5 | 96 | 104 | 96 | 80 | 91 | 62 | 70 | 88 | 76 | 93 | 78 | 93 | 1.03 | 1.01 | 1.03 | 0.72 | 79.2 |
| 8000 | 5 | 94 | 101 | 94 | 76 | 90 | 54 | 66 | 84 | 73 | 95 | 79 | 97 | 1.02 | 0.99 | 1.02 | 0.71 | 80.1 |
| 10000 | -2.5 | 102 | 110 | 97 | 84 | 90 | 86 | 83 | 95 | 83 | 86 | 80 | 83 | 1.18 | 1.16 | 1.18 | 0.85 | 74.6 |
| 10000 | 0 | 100 | 107 | 95 | 81 | 92 | 79 | 79 | 94 | 80 | 90 | 80 | 87 | 1.17 | 1.14 | 1.17 | 0.84 | 75.4 |
| 10000 | 2.5 | 98 | 104 | 93 | 77 | 92 | 71 | 74 | 90 | 77 | 92 | 80 | 92 | 1.16 | 1.13 | 1.16 | 0.83 | 76.2 |
| 10000 | 5 | 95 | 101 | 91 | 73 | 91 | 61 | 70 | 86 | 74 | 94 | 80 | 96 | 1.14 | 1.12 | 1.15 | 0.82 | 77.0 |
| 11000 | -2.5 | 102 | 110 | 97 | 83 | 90 | 88 | 84 | 95 | 83 | 86 | 80 | 82 | 1.22 | 1.20 | 1.23 | 0.89 | 73.5 |
| 11000 | 0 | 100 | 107 | 95 | 80 | 92 | 82 | 80 | 94 | 81 | 89 | 81 | 87 | 1.21 | 1.19 | 1.22 | 0.88 | 74.3 |
| 11000 | 2.5 | 98 | 104 | 93 | 76 | 92 | 73 | 76 | 91 | 78 | 92 | 81 | 91 | 1.20 | 1.18 | 1.21 | 0.87 | 75.1 |
| 11000 | 5 | 96 | 101 | 90 | 72 | 92 | 63 | 71 | 87 | 75 | 93 | 81 | 95 | 1.19 | 1.17 | 1.20 | 0.86 | 75.9 |
| 12000 | -2.5 | 103 | 110 | 96 | 83 | 90 | 89 | 86 | 95 | 83 | 85 | 81 | 82 | 1.26 | 1.24 | 1.27 | 0.92 | 72.6 |
| 12000 | 0 | 101 | 107 | 94 | 79 | 92 | 84 | 82 | 95 | 81 | 89 | 81 | 86 | 1.25 | 1.23 | 1.26 | 0.91 | 73.4 |
| 12000 | 2.5 | 98 | 104 | 92 | 75 | 93 | 75 | 77 | 91 | 78 | 92 | 81 | 91 | 1.24 | 1.22 | 1.25 | 0.90 | 74.2 |
| 12000 | 5 | 96 | 101 | 88 | 71 | 92 | 65 | 73 | 87 | 75 | 93 | 81 | 95 | 1.23 | 1.21 | 1.24 | 0.89 | 74.9 |
| 13000 | -2.5 | 103 | 110 | 96 | 83 | 90 | 90 | 87 | 94 | 83 | 85 | 82 | 81 | 1.29 | 1.27 | 1.31 | 0.95 | 71.9 |
| 13000 | 0 | 101 | 108 | 94 | 79 | 92 | 86 | 83 | 95 | 81 | 88 | 82 | 86 | 1.28 | 1.26 | 1.29 | 0.94 | 72.6 |
| 13000 | 2.5 | 99 | 104 | 91 | 75 | 93 | 77 | 78 | 92 | 79 | 91 | 82 | 90 | 1.28 | 1.25 | 1.29 | 0.93 | 73.4 |
| 13000 | 5 | 96 | 101 | 89 | 70 | 92 | 67 | 74 | 88 | 76 | 93 | 82 | 94 | 1.27 | 1.25 | 1.28 | 0.92 | 74.1 |
| 17000 | -2.5 | 104 | 111 | 94 | 82 | 90 | 88 | 91 | 93 | 84 | 84 | 83 | 80 | 1.39 | 1.37 | 1.40 | 1.03 | 69.9 |
| 17000 | 0 | 102 | 108 | 92 | 78 | 92 | 89 | 87 | 95 | 82 | 87 | 83 | 85 | 1.38 | 1.36 | 1.40 | 1.02 | 70.5 |
| 17000 | 2.5 | 99 | 105 | 90 | 73 | 94 | 81 | 82 | 93 | 80 | 90 | 83 | 89 | 1.37 | 1.35 | 1.39 | 1.01 | 71.2 |
| 17000 | 5 | 97 | 101 | 88 | 69 | 93 | 70 | 78 | 88 | 77 | 92 | 84 | 94 | 1.37 | 1.34 | 1.38 | 1.00 | 71.9 |
| 20000 | -2.5 | 105 | 111 | 94 | 82 | 90 | 87 | 93 | 92 | 84 | 83 | 84 | 80 | 1.43 | 1.42 | 1.45 | 1.07 | 68.9 |
| 20000 | 0 | 102 | 108 | 92 | 77 | 93 | 90 | 89 | 95 | 82 | 87 | 84 | 84 | 1.43 | 1.41 | 1.44 | 1.06 | 69.5 |
| 20000 | 2.5 | 100 | 105 | 89 | 73 | 94 | 83 | 84 | 93 | 80 | 90 | 84 | 89 | 1.42 | 1.40 | 1.44 | 1.05 | 70.2 |
| 20000 | 5 | 97 | 101 | 87 | 68 | 94 | 72 | 80 | 89 | 78 | 92 | 85 | 93 | 1.41 | 1.39 | 1.43 | 1.04 | 70.9 |

EP 2 281 606 A1

FIG. 18

| Tc | Duv | Color rendering indexes | | | | | | | | | | | | Action power for per unit luminous flux | | | | Efficiency [lm/W] |
|---|---|---|---|---|---|---|---|---|---|---|---|---|---|---|---|---|---|---|
| | | Ga4 | Ga | M | PS | Ra | R9 | R10 | R11 | R12 | R13 | R14 | R15 | Br | Th | c($\lambda$) | z($\lambda$) | |
| 7100 | −2.5 | 101 | 112 | 105 | 87 | 88 | 81 | 82 | 86 | 88 | 85 | 78 | 82 | 0.99 | 0.97 | 0.98 | 0.69 | 80.8 |
| 7100 | 0 | 99 | 110 | 103 | 85 | 89 | 74 | 78 | 89 | 84 | 88 | 78 | 86 | 0.98 | 0.96 | 0.97 | 0.68 | 81.7 |
| 7100 | 2.5 | 98 | 107 | 101 | 82 | 89 | 66 | 73 | 90 | 80 | 91 | 78 | 90 | 0.96 | 0.94 | 0.95 | 0.66 | 82.7 |
| 7100 | 5 | 96 | 105 | 99 | 79 | 89 | 58 | 69 | 89 | 77 | 94 | 78 | 94 | 0.95 | 0.93 | 0.94 | 0.65 | 83.7 |
| 8000 | −2.5 | 103 | 113 | 104 | 86 | 88 | 87 | 85 | 84 | 89 | 84 | 79 | 81 | 1.06 | 1.05 | 1.06 | 0.75 | 79.2 |
| 8000 | 0 | 101 | 111 | 102 | 84 | 89 | 81 | 80 | 87 | 85 | 87 | 79 | 85 | 1.05 | 1.03 | 1.04 | 0.74 | 80.1 |
| 8000 | 2.5 | 99 | 108 | 100 | 81 | 90 | 73 | 76 | 89 | 82 | 90 | 79 | 89 | 1.04 | 1.02 | 1.03 | 0.72 | 81.0 |
| 8000 | 5 | 97 | 105 | 98 | 77 | 90 | 65 | 71 | 89 | 78 | 93 | 79 | 93 | 1.03 | 1.01 | 1.02 | 0.71 | 82.0 |
| 10000 | −2.5 | 105 | 115 | 102 | 85 | 87 | 89 | 90 | 79 | 90 | 82 | 81 | 78 | 1.19 | 1.17 | 1.18 | 0.85 | 76.6 |
| 10000 | 0 | 103 | 112 | 100 | 82 | 89 | 90 | 85 | 83 | 87 | 85 | 81 | 82 | 1.18 | 1.16 | 1.17 | 0.84 | 77.4 |
| 10000 | 2.5 | 101 | 109 | 98 | 79 | 90 | 83 | 81 | 86 | 84 | 89 | 81 | 87 | 1.17 | 1.15 | 1.16 | 0.83 | 78.2 |
| 10000 | 5 | 99 | 106 | 96 | 75 | 90 | 74 | 76 | 87 | 81 | 91 | 81 | 91 | 1.16 | 1.14 | 1.15 | 0.82 | 79.1 |
| 11000 | −2.5 | 106 | 115 | 102 | 85 | 86 | 87 | 92 | 78 | 91 | 81 | 81 | 77 | 1.23 | 1.22 | 1.23 | 0.89 | 75.6 |
| 11000 | 0 | 104 | 112 | 100 | 82 | 89 | 91 | 87 | 82 | 88 | 85 | 81 | 82 | 1.22 | 1.21 | 1.22 | 0.88 | 76.4 |
| 11000 | 2.5 | 102 | 109 | 97 | 78 | 90 | 86 | 83 | 85 | 85 | 88 | 82 | 86 | 1.21 | 1.20 | 1.21 | 0.87 | 77.2 |
| 11000 | 5 | 99 | 106 | 95 | 74 | 91 | 78 | 78 | 87 | 81 | 91 | 82 | 91 | 1.20 | 1.19 | 1.20 | 0.86 | 78.0 |
| 12000 | −2.5 | 107 | 116 | 101 | 85 | 86 | 84 | 93 | 76 | 91 | 80 | 82 | 76 | 1.27 | 1.26 | 1.27 | 0.92 | 74.8 |
| 12000 | 0 | 105 | 113 | 99 | 82 | 88 | 91 | 89 | 80 | 88 | 84 | 82 | 81 | 1.26 | 1.25 | 1.26 | 0.91 | 75.5 |
| 12000 | 2.5 | 102 | 110 | 97 | 78 | 90 | 88 | 84 | 84 | 85 | 87 | 82 | 85 | 1.25 | 1.24 | 1.25 | 0.90 | 76.3 |
| 12000 | 5 | 100 | 107 | 95 | 74 | 91 | 80 | 80 | 86 | 82 | 90 | 82 | 90 | 1.24 | 1.23 | 1.24 | 0.89 | 77.1 |
| 13000 | −2.5 | 107 | 116 | 101 | 85 | 86 | 82 | 95 | 75 | 91 | 80 | 82 | 76 | 1.31 | 1.29 | 1.31 | 0.95 | 74.1 |
| 13000 | 0 | 105 | 113 | 99 | 81 | 88 | 90 | 91 | 79 | 89 | 83 | 83 | 80 | 1.30 | 1.28 | 1.30 | 0.94 | 74.8 |
| 13000 | 2.5 | 103 | 110 | 96 | 77 | 90 | 90 | 86 | 83 | 86 | 87 | 83 | 85 | 1.29 | 1.27 | 1.29 | 0.93 | 75.6 |
| 13000 | 5 | 100 | 107 | 94 | 73 | 91 | 82 | 81 | 85 | 83 | 90 | 83 | 90 | 1.28 | 1.26 | 1.28 | 0.92 | 76.3 |
| 17000 | −2.5 | 109 | 117 | 100 | 85 | 85 | 75 | 97 | 72 | 92 | 78 | 84 | 74 | 1.40 | 1.39 | 1.41 | 1.03 | 72.1 |
| 17000 | 0 | 107 | 114 | 98 | 81 | 88 | 85 | 95 | 76 | 90 | 82 | 84 | 79 | 1.39 | 1.38 | 1.40 | 1.02 | 72.8 |
| 17000 | 2.5 | 104 | 111 | 95 | 77 | 90 | 92 | 90 | 80 | 87 | 86 | 85 | 83 | 1.39 | 1.37 | 1.39 | 1.01 | 73.5 |
| 17000 | 5 | 101 | 107 | 93 | 72 | 91 | 87 | 85 | 83 | 84 | 89 | 85 | 88 | 1.38 | 1.36 | 1.38 | 1.00 | 74.2 |
| 20000 | −2.5 | 110 | 118 | 100 | 85 | 85 | 72 | 96 | 71 | 93 | 77 | 85 | 73 | 1.45 | 1.44 | 1.45 | 1.07 | 71.2 |
| 20000 | 0 | 107 | 114 | 97 | 81 | 88 | 82 | 97 | 75 | 91 | 81 | 85 | 78 | 1.44 | 1.43 | 1.45 | 1.06 | 71.8 |
| 20000 | 2.5 | 105 | 111 | 95 | 77 | 90 | 91 | 93 | 79 | 88 | 85 | 85 | 83 | 1.43 | 1.42 | 1.44 | 1.05 | 72.5 |
| 20000 | 5 | 102 | 108 | 93 | 72 | 92 | 90 | 88 | 81 | 85 | 88 | 86 | 87 | 1.43 | 1.41 | 1.43 | 1.04 | 73.2 |

FIG. 19

| Tc | Duv | Color rendering indexes | | | | | | | | | | | | | Action power for per unit luminous flux | | | | Efficiency [lm/W] |
|---|---|---|---|---|---|---|---|---|---|---|---|---|---|---|---|---|---|---|---|
| | | Ga4 | Ga | M | PS | Ra | R9 | R10 | R11 | R12 | R13 | R14 | R15 | Br | Th | $c(\lambda)$ | $z(\lambda)$ | |
| 7100 | -2.5 | 104 | 116 | 106 | 90 | 84 | 90 | 97 | 70 | 98 | 77 | 82 | 76 | 1.02 | 0.99 | 1.00 | 0.69 | 79.6 |
| 7100 | 0 | 103 | 114 | 105 | 88 | 86 | 88 | 93 | 75 | 94 | 81 | 82 | 80 | 1.00 | 0.98 | 0.99 | 0.68 | 80.5 |
| 7100 | 2.5 | 101 | 112 | 104 | 86 | 88 | 82 | 88 | 79 | 90 | 84 | 81 | 84 | 0.99 | 0.96 | 0.97 | 0.66 | 81.5 |
| 7100 | 5 | 99 | 109 | 102 | 84 | 90 | 74 | 82 | 83 | 86 | 88 | 81 | 88 | 0.97 | 0.95 | 0.96 | 0.65 | 82.5 |
| 8000 | -2.5 | 107 | 118 | 105 | 90 | 83 | 85 | 97 | 66 | 99 | 75 | 83 | 73 | 1.09 | 1.07 | 1.08 | 0.75 | 78.0 |
| 8000 | 0 | 105 | 115 | 104 | 88 | 85 | 90 | 97 | 71 | 97 | 79 | 83 | 77 | 1.08 | 1.05 | 1.07 | 0.74 | 78.9 |
| 8000 | 2.5 | 103 | 113 | 102 | 86 | 87 | 89 | 92 | 75 | 93 | 83 | 83 | 82 | 1.06 | 1.04 | 1.05 | 0.72 | 79.8 |
| 8000 | 5 | 101 | 110 | 101 | 83 | 89 | 82 | 87 | 79 | 89 | 86 | 83 | 86 | 1.05 | 1.02 | 1.04 | 0.71 | 80.7 |
| 10000 | -2.5 | 110 | 120 | 104 | 90 | 81 | 71 | 90 | 59 | 96 | 72 | 84 | 69 | 1.22 | 1.19 | 1.21 | 0.85 | 75.2 |
| 10000 | 0 | 108 | 118 | 102 | 88 | 83 | 80 | 95 | 64 | 99 | 76 | 85 | 73 | 1.21 | 1.18 | 1.20 | 0.84 | 76.0 |
| 10000 | 2.5 | 106 | 115 | 101 | 85 | 86 | 88 | 99 | 69 | 96 | 80 | 85 | 78 | 1.20 | 1.17 | 1.19 | 0.83 | 76.9 |
| 10000 | 5 | 104 | 112 | 99 | 82 | 88 | 92 | 94 | 73 | 92 | 83 | 85 | 82 | 1.19 | 1.16 | 1.17 | 0.82 | 77.7 |
| 11000 | -2.5 | 111 | 121 | 103 | 90 | 80 | 65 | 87 | 56 | 95 | 71 | 85 | 68 | 1.27 | 1.24 | 1.26 | 0.89 | 74.2 |
| 11000 | 0 | 109 | 118 | 102 | 88 | 82 | 75 | 92 | 61 | 98 | 75 | 85 | 72 | 1.26 | 1.23 | 1.25 | 0.88 | 75.0 |
| 11000 | 2.5 | 107 | 115 | 100 | 85 | 85 | 84 | 97 | 66 | 97 | 79 | 85 | 77 | 1.25 | 1.22 | 1.24 | 0.87 | 75.8 |
| 11000 | 5 | 104 | 112 | 98 | 82 | 87 | 92 | 96 | 71 | 93 | 82 | 86 | 81 | 1.24 | 1.21 | 1.23 | 0.86 | 76.6 |
| 12000 | -2.5 | 112 | 122 | 103 | 91 | 79 | 61 | 84 | 54 | 94 | 70 | 86 | 66 | 1.31 | 1.28 | 1.30 | 0.92 | 73.3 |
| 12000 | 0 | 110 | 119 | 101 | 88 | 82 | 70 | 90 | 59 | 97 | 74 | 86 | 71 | 1.30 | 1.27 | 1.29 | 0.91 | 74.1 |
| 12000 | 2.5 | 108 | 116 | 100 | 85 | 84 | 80 | 95 | 64 | 97 | 78 | 86 | 76 | 1.29 | 1.26 | 1.28 | 0.90 | 74.9 |
| 12000 | 5 | 105 | 113 | 98 | 82 | 87 | 89 | 97 | 69 | 94 | 81 | 86 | 80 | 1.28 | 1.25 | 1.27 | 0.89 | 75.6 |
| 13000 | -2.5 | 113 | 123 | 103 | 91 | 79 | 57 | 82 | 52 | 93 | 69 | 86 | 65 | 1.34 | 1.32 | 1.34 | 0.95 | 72.6 |
| 13000 | 0 | 111 | 120 | 101 | 88 | 81 | 67 | 87 | 58 | 96 | 73 | 86 | 70 | 1.33 | 1.31 | 1.33 | 0.94 | 73.4 |
| 13000 | 2.5 | 108 | 117 | 99 | 85 | 84 | 77 | 93 | 62 | 97 | 77 | 87 | 75 | 1.32 | 1.30 | 1.32 | 0.93 | 74.1 |
| 13000 | 5 | 106 | 113 | 98 | 82 | 86 | 87 | 96 | 67 | 94 | 81 | 87 | 79 | 1.31 | 1.29 | 1.31 | 0.92 | 74.9 |
| 17000 | -2.5 | 115 | 125 | 102 | 91 | 77 | 46 | 75 | 47 | 90 | 67 | 87 | 62 | 1.44 | 1.42 | 1.44 | 1.03 | 70.6 |
| 17000 | 0 | 113 | 121 | 100 | 89 | 80 | 57 | 80 | 53 | 94 | 71 | 87 | 67 | 1.43 | 1.41 | 1.43 | 1.02 | 71.3 |
| 17000 | 2.5 | 110 | 118 | 98 | 86 | 83 | 68 | 86 | 58 | 96 | 75 | 88 | 72 | 1.43 | 1.40 | 1.42 | 1.01 | 72.0 |
| 17000 | 5 | 108 | 115 | 97 | 82 | 85 | 78 | 91 | 62 | 95 | 78 | 88 | 77 | 1.42 | 1.39 | 1.41 | 1.00 | 72.7 |
| 20000 | -2.5 | 116 | 125 | 102 | 92 | 76 | 42 | 71 | 45 | 89 | 65 | 87 | 61 | 1.49 | 1.46 | 1.49 | 1.07 | 69.6 |
| 20000 | 0 | 113 | 122 | 100 | 89 | 79 | 53 | 77 | 50 | 92 | 70 | 88 | 66 | 1.48 | 1.46 | 1.48 | 1.06 | 70.3 |
| 20000 | 2.5 | 111 | 119 | 98 | 86 | 82 | 63 | 82 | 55 | 95 | 73 | 88 | 71 | 1.47 | 1.45 | 1.47 | 1.05 | 70.9 |
| 20000 | 5 | 108 | 115 | 96 | 83 | 84 | 74 | 87 | 60 | 95 | 77 | 89 | 75 | 1.47 | 1.44 | 1.46 | 1.04 | 71.6 |

FIG. 20

| Tc | Duv | Color rendering indexes | | | | | | | | | | | | Action power for per unit luminous flux | | | | Efficiency [lm/W] |
|---|---|---|---|---|---|---|---|---|---|---|---|---|---|---|---|---|---|---|
| | | Ga4 | Ga | M | PS | Ra | R9 | R10 | R11 | R12 | R13 | R14 | R15 | Br | Th | c(λ) | z(λ) | |
| 7100 | -2.5 | 108 | 121 | 114 | 93 | 78 | 78 | 84 | 51 | 88 | 69 | 85 | 68 | 1.04 | 1.01 | 1.02 | 0.69 | 78.3 |
| 7100 | 0 | 106 | 118 | 112 | 92 | 81 | 85 | 90 | 57 | 92 | 73 | 85 | 72 | 1.03 | 1.00 | 1.01 | 0.68 | 79.2 |
| 7100 | 2.5 | 104 | 116 | 110 | 90 | 83 | 91 | 96 | 62 | 95 | 76 | 85 | 76 | 1.01 | 0.98 | 0.99 | 0.66 | 80.2 |
| 7100 | 5 | 102 | 113 | 108 | 88 | 86 | 89 | 97 | 67 | 94 | 80 | 85 | 81 | 1.00 | 0.96 | 0.98 | 0.65 | 81.2 |
| 8000 | -2.5 | 110 | 123 | 113 | 94 | 76 | 66 | 78 | 45 | 86 | 66 | 86 | 64 | 1.12 | 1.09 | 1.10 | 0.75 | 76.6 |
| 8000 | 0 | 108 | 120 | 111 | 92 | 79 | 75 | 84 | 51 | 90 | 70 | 86 | 69 | 1.11 | 1.07 | 1.09 | 0.74 | 77.5 |
| 8000 | 2.5 | 107 | 118 | 109 | 90 | 81 | 83 | 90 | 57 | 93 | 74 | 86 | 73 | 1.09 | 1.06 | 1.08 | 0.72 | 78.4 |
| 8000 | 5 | 105 | 115 | 107 | 88 | 84 | 90 | 95 | 62 | 95 | 78 | 86 | 78 | 1.08 | 1.05 | 1.06 | 0.71 | 79.3 |
| 10000 | -2.5 | 114 | 126 | 113 | 94 | 73 | 46 | 67 | 36 | 82 | 61 | 87 | 59 | 1.26 | 1.22 | 1.24 | 0.85 | 81.2 |
| 10000 | 0 | 112 | 124 | 111 | 93 | 76 | 56 | 73 | 41 | 86 | 65 | 87 | 63 | 1.24 | 1.21 | 1.23 | 0.84 | 81.2 |
| 10000 | 2.5 | 110 | 121 | 109 | 91 | 78 | 65 | 79 | 47 | 89 | 69 | 87 | 68 | 1.23 | 1.20 | 1.21 | 0.83 | 81.2 |
| 10000 | 5 | 108 | 118 | 107 | 88 | 81 | 75 | 84 | 52 | 92 | 73 | 88 | 72 | 1.22 | 1.18 | 1.20 | 0.82 | 81.2 |
| 11000 | -2.5 | 116 | 128 | 113 | 95 | 71 | 39 | 62 | 32 | 80 | 60 | 87 | 57 | 1.31 | 1.27 | 1.29 | 0.89 | 72.7 |
| 11000 | 0 | 114 | 125 | 111 | 94 | 74 | 46 | 67 | 36 | 83 | 63 | 87 | 60 | 1.30 | 1.27 | 1.29 | 0.89 | 73.1 |
| 11000 | 2.5 | 112 | 122 | 109 | 91 | 77 | 58 | 75 | 43 | 88 | 68 | 87 | 66 | 1.28 | 1.25 | 1.27 | 0.87 | 74.2 |
| 11000 | 5 | 109 | 119 | 107 | 89 | 80 | 68 | 80 | 49 | 91 | 72 | 88 | 70 | 1.27 | 1.23 | 1.25 | 0.86 | 75.0 |
| 12000 | -2.5 | 117 | 129 | 113 | 95 | 70 | 33 | 59 | 29 | 79 | 58 | 86 | 55 | 1.35 | 1.31 | 1.33 | 0.92 | 71.8 |
| 12000 | 0 | 115 | 126 | 111 | 93 | 73 | 42 | 65 | 34 | 82 | 62 | 87 | 59 | 1.34 | 1.31 | 1.33 | 0.92 | 72.5 |
| 12000 | 2.5 | 113 | 123 | 109 | 92 | 76 | 53 | 71 | 41 | 86 | 66 | 87 | 64 | 1.33 | 1.29 | 1.31 | 0.90 | 73.3 |
| 12000 | 5 | 111 | 120 | 107 | 89 | 79 | 63 | 77 | 46 | 89 | 70 | 88 | 69 | 1.32 | 1.28 | 1.30 | 0.89 | 74.0 |
| 13000 | -2.5 | 118 | 130 | 113 | 95 | 69 | 28 | 55 | 26 | 78 | 57 | 86 | 53 | 1.38 | 1.35 | 1.37 | 0.95 | 71.0 |
| 13000 | 0 | 116 | 127 | 111 | 94 | 72 | 38 | 62 | 32 | 81 | 61 | 87 | 58 | 1.37 | 1.34 | 1.36 | 0.94 | 71.8 |
| 13000 | 2.5 | 114 | 123 | 109 | 92 | 75 | 48 | 68 | 38 | 85 | 65 | 87 | 63 | 1.36 | 1.33 | 1.35 | 0.93 | 72.5 |
| 13000 | 5 | 111 | 120 | 106 | 90 | 78 | 58 | 73 | 44 | 88 | 69 | 88 | 67 | 1.35 | 1.32 | 1.34 | 0.92 | 73.2 |
| 17000 | -2.5 | | | | | | | | | | | | | | | | | |
| 17000 | 0 | | | | | | | | | | | | | | | | | |
| 17000 | 2.5 | | | | | | | | | | | | | | | | | |
| 17000 | 5 | | | | | | | | | | | | | | | | | |
| 20000 | -2.5 | | | | | | | | | | | | | | | | | |
| 20000 | 0 | | | | | | | | | | | | | | | | | |
| 20000 | 2.5 | | | | | | | | | | | | | | | | | |
| 20000 | 5 | | | | | | | | | | | | | | | | | |

7100K, Duv=0

**FIG. 21A**

**FIG. 21B**

**FIG. 21C**

11000K, Duv=0

FIG. 22A

FIG. 22B

FIG. 22C

20000K, Duv=0

FIG. 23A

FIG. 23B

FIG. 23C

7100K, Duv=0

FIG. 24A

FIG. 24B

△ Reference light source
□ Comparative example

FIG. 24C

△ Reference light source
□ Comparative example

11000K, Duv=0

FIG. 25A

FIG. 25B

FIG. 25C

20000K, Duv=0

FIG. 26A

FIG. 26B

△ Reference light source
□ Comparative example

FIG. 26C

△ Reference light source
□ Comparative example

7100K, Duv=0

FIG. 27A

FIG. 27B

FIG. 27C

11000K, Duv=0

FIG. 28A

FIG. 28B

FIG. 28C

20000K, Duv=0

FIG. 29A

FIG. 29B

FIG. 29C

7100K, Duv=0

FIG. 30A

FIG. 30B

FIG. 30C

11000K, Duv=0

FIG. 31A

FIG. 31B

FIG. 31C

20000K, Duv=0

FIG. 32A

FIG. 32B

FIG. 32C

7100K, Duv=0

FIG. 33A

FIG. 33B

FIG. 33C

11000K, Duv=0

FIG. 34A

FIG. 34B

FIG. 34C

20000K, Duv=0

FIG. 35A

FIG. 35B

FIG. 35C

7100K, Duv=0

FIG. 36A

FIG. 36B

FIG. 36C

11000K, Duv=0

FIG. 37A

FIG. 37B

Reference light source △
Example □

FIG. 37C

Reference light source △
Example □

20000K, Duv=0

FIG. 38A

FIG. 38B

FIG. 38C

7100K, Duv=0

FIG. 39A

FIG. 39B

FIG. 39C

11000K, Duv=0

FIG. 40A

FIG. 40B

FIG. 40C

20000K, Duv=0

FIG. 41A

FIG. 41B

FIG. 41C

11000K, Duv=0

FIG. 42A

FIG. 42B

FIG. 42C

11000K, Duv=0

FIG. 43A

FIG. 43B

△ Reference light source
□ Sample light source

FIG. 43C

△ Reference light source
□ Sample light source

11000K, Duv=0

FIG. 44A

FIG. 44B

FIG. 44C

SCA, 11000K, Duv=0

FIG. 45A

FIG. 45B

FIG. 45C

FIG. 46A

SCA broad, 11000K, Duv=0

FIG. 46B

FIG. 46C

FIG. 47B

FIG. 47A

FIG. 48A

FIG. 48B

FIG. 48C

EP 2 281 606 A1

# FIG. 50

(SCA broad, 12000K, Duv=0)

FIG. 51

| Tc | Duv | Color rendering indexes | | | | | | | | | | | | Action power for per unit luminous flux | | | | Efficiency [lm/W] |
|---|---|---|---|---|---|---|---|---|---|---|---|---|---|---|---|---|---|---|
| | | Ga4 | Ga | M | PS | Ra | R9 | R10 | R11 | R12 | R13 | R14 | R15 | Br | Th | c(λ) | z(λ) | |
| 7100 | -2.5 | 96 | 106 | 97 | 84 | 91 | 64 | 76 | 91 | 84 | 91 | 79 | 89 | 0.98 | 0.96 | 0.99 | 0.69 | 76.6 |
| 7100 | 0 | 95 | 104 | 96 | 81 | 91 | 57 | 71 | 87 | 81 | 94 | 79 | 93 | 0.97 | 0.95 | 0.97 | 0.68 | 77.6 |
| 7100 | 2.5 | 93 | 102 | 94 | 78 | 90 | 49 | 67 | 84 | 77 | 96 | 79 | 97 | 0.96 | 0.93 | 0.96 | 0.66 | 78.6 |
| 7100 | 5 | 91 | 100 | 92 | 75 | 88 | 41 | 63 | 80 | 74 | 95 | 79 | 99 | 0.94 | 0.92 | 0.94 | 0.65 | 79.6 |
| 8000 | -2.5 | 97 | 107 | 96 | 82 | 91 | 70 | 78 | 93 | 85 | 91 | 80 | 88 | 1.06 | 1.04 | 1.06 | 0.75 | 74.8 |
| 8000 | 0 | 95 | 104 | 94 | 79 | 92 | 62 | 73 | 89 | 82 | 94 | 80 | 92 | 1.04 | 1.02 | 1.05 | 0.74 | 75.7 |
| 8000 | 2.5 | 94 | 102 | 92 | 76 | 91 | 54 | 69 | 84 | 78 | 96 | 80 | 96 | 1.03 | 1.01 | 1.04 | 0.72 | 76.6 |
| 8000 | 5 | 92 | 99 | 90 | 72 | 89 | 45 | 65 | 80 | 75 | 95 | 80 | 99 | 1.02 | 1.00 | 1.02 | 0.71 | 77.6 |
| 11000 | -2.5 | 99 | 107 | 92 | 79 | 92 | 80 | 83 | 95 | 86 | 90 | 82 | 87 | 1.22 | 1.20 | 1.24 | 0.89 | 70.8 |
| 11000 | 0 | 97 | 104 | 90 | 75 | 93 | 71 | 79 | 91 | 84 | 93 | 82 | 91 | 1.21 | 1.19 | 1.23 | 0.88 | 71.5 |
| 11000 | 2.5 | 95 | 102 | 88 | 71 | 92 | 62 | 74 | 87 | 81 | 95 | 82 | 95 | 1.20 | 1.18 | 1.22 | 0.87 | 72.3 |
| 11000 | 5 | 92 | 99 | 86 | 67 | 91 | 52 | 70 | 82 | 78 | 94 | 82 | 98 | 1.19 | 1.17 | 1.20 | 0.86 | 73.1 |
| 12000 | -2.5 | 99 | 107 | 91 | 79 | 92 | 82 | 84 | 96 | 87 | 89 | 83 | 86 | 1.26 | 1.24 | 1.28 | 0.92 | 69.8 |
| 12000 | 0 | 97 | 104 | 89 | 75 | 93 | 73 | 80 | 92 | 84 | 92 | 83 | 91 | 1.25 | 1.23 | 1.27 | 0.91 | 70.6 |
| 12000 | 2.5 | 95 | 102 | 87 | 70 | 93 | 63 | 76 | 87 | 81 | 94 | 83 | 95 | 1.24 | 1.22 | 1.26 | 0.90 | 71.3 |
| 12000 | 5 | 93 | 99 | 85 | 66 | 91 | 53 | 71 | 83 | 78 | 94 | 83 | 98 | 1.24 | 1.21 | 1.25 | 0.89 | 72.0 |
| 13000 | -2.5 | 100 | 107 | 91 | 78 | 92 | 83 | 86 | 96 | 87 | 89 | 83 | 86 | 1.30 | 1.28 | 1.31 | 0.95 | 69.1 |
| 13000 | 0 | 97 | 105 | 89 | 74 | 93 | 75 | 82 | 92 | 85 | 92 | 83 | 90 | 1.29 | 1.27 | 1.30 | 0.94 | 69.8 |
| 13000 | 2.5 | 95 | 101 | 87 | 69 | 93 | 64 | 77 | 88 | 82 | 94 | 83 | 95 | 1.28 | 1.26 | 1.29 | 0.93 | 70.5 |
| 13000 | 5 | 93 | 99 | 84 | 65 | 91 | 54 | 73 | 83 | 79 | 94 | 83 | 97 | 1.27 | 1.25 | 1.28 | 0.92 | 71.2 |
| 17000 | -2.5 | 100 | 108 | 89 | 77 | 93 | 86 | 89 | 98 | 88 | 88 | 85 | 85 | 1.39 | 1.37 | 1.41 | 1.03 | 66.9 |
| 17000 | 0 | 98 | 105 | 87 | 72 | 94 | 78 | 85 | 93 | 86 | 91 | 85 | 90 | 1.38 | 1.36 | 1.40 | 1.02 | 67.6 |
| 17000 | 2.5 | 95 | 101 | 85 | 67 | 94 | 67 | 81 | 89 | 83 | 94 | 85 | 94 | 1.38 | 1.35 | 1.39 | 1.01 | 68.2 |
| 17000 | 5 | 93 | 98 | 83 | 62 | 92 | 56 | 76 | 84 | 80 | 94 | 85 | 97 | 1.37 | 1.35 | 1.39 | 1.00 | 68.9 |
| 20000 | -2.5 | 100 | 108 | 88 | 76 | 93 | 87 | 91 | 98 | 88 | 88 | 86 | 85 | 1.44 | 1.42 | 1.46 | 1.07 | 65.9 |
| 20000 | 0 | 98 | 104 | 86 | 71 | 94 | 79 | 87 | 94 | 86 | 91 | 86 | 90 | 1.43 | 1.41 | 1.45 | 1.06 | 66.5 |
| 20000 | 2.5 | 95 | 101 | 84 | 67 | 94 | 68 | 83 | 89 | 84 | 93 | 86 | 94 | 1.42 | 1.40 | 1.44 | 1.05 | 67.1 |
| 20000 | 5 | 93 | 98 | 82 | 61 | 93 | 57 | 78 | 84 | 81 | 94 | 86 | 96 | 1.42 | 1.39 | 1.44 | 1.04 | 67.8 |

EP 2 281 606 A1

FIG. 52B

FIG. 52A

FIG. 53

(CMZ phosphor emission spectrum)

EP 2 281 606 A1

FIG. 54

| Tc | Duv | Color rendering indexes | | | | | | | | | | | | Action power for per unit luminous flux | | | | Efficiency |
|---|---|---|---|---|---|---|---|---|---|---|---|---|---|---|---|---|---|---|
| | | Ga4 | Ga | M | PS | Ra | R9 | R10 | R11 | R12 | R13 | R14 | R15 | Br | Th | $c(\lambda)$ | $z(\lambda)$ | [lm/W] |
| 7100 | -2.5 | 102 | 114 | 107 | 86 | 87 | 81 | 81 | 80 | 86 | 84 | 78 | 81 | 0.99 | 0.97 | 0.97 | 0.69 | 73.9 |
| 7100 | 0 | 100 | 111 | 105 | 84 | 88 | 74 | 77 | 83 | 83 | 87 | 78 | 85 | 0.97 | 0.96 | 0.96 | 0.68 | 74.9 |
| 7100 | 2.5 | 99 | 109 | 103 | 81 | 88 | 67 | 72 | 86 | 79 | 91 | 78 | 89 | 0.96 | 0.95 | 0.95 | 0.66 | 75.9 |
| 7100 | 5 | 97 | 106 | 101 | 78 | 88 | 59 | 68 | 87 | 76 | 93 | 78 | 93 | 0.94 | 0.93 | 0.93 | 0.65 | 76.9 |
| 8000 | -2.5 | 104 | 115 | 106 | 86 | 86 | 88 | 84 | 77 | 88 | 83 | 78 | 79 | 1.06 | 1.05 | 1.05 | 0.75 | 72.0 |
| 8000 | 0 | 102 | 112 | 104 | 83 | 88 | 82 | 79 | 80 | 84 | 86 | 78 | 84 | 1.05 | 1.03 | 1.03 | 0.74 | 72.9 |
| 8000 | 2.5 | 100 | 110 | 102 | 80 | 88 | 74 | 75 | 83 | 81 | 90 | 78 | 88 | 1.03 | 1.02 | 1.02 | 0.72 | 73.8 |
| 8000 | 5 | 98 | 107 | 100 | 77 | 88 | 66 | 70 | 85 | 77 | 92 | 79 | 92 | 1.02 | 1.01 | 1.01 | 0.71 | 74.7 |
| 10000 | -2.5 | 107 | 117 | 105 | 85 | 85 | 88 | 88 | 71 | 89 | 81 | 80 | 76 | 1.18 | 1.17 | 1.17 | 0.85 | 68.8 |
| 10000 | 0 | 105 | 114 | 103 | 82 | 87 | 90 | 84 | 75 | 86 | 84 | 80 | 81 | 1.17 | 1.16 | 1.16 | 0.84 | 69.6 |
| 10000 | 2.5 | 103 | 111 | 101 | 79 | 88 | 85 | 79 | 79 | 83 | 88 | 80 | 85 | 1.16 | 1.15 | 1.15 | 0.83 | 70.4 |
| 10000 | 5 | 100 | 108 | 98 | 75 | 89 | 77 | 75 | 81 | 80 | 91 | 80 | 89 | 1.15 | 1.14 | 1.14 | 0.82 | 71.2 |
| 11000 | -2.5 | 108 | 117 | 105 | 85 | 85 | 85 | 90 | 69 | 90 | 80 | 80 | 75 | 1.23 | 1.22 | 1.22 | 0.89 | 67.7 |
| 11000 | 0 | 106 | 114 | 103 | 82 | 87 | 91 | 86 | 73 | 87 | 83 | 80 | 80 | 1.22 | 1.21 | 1.21 | 0.88 | 68.4 |
| 11000 | 2.5 | 103 | 111 | 100 | 78 | 88 | 88 | 81 | 77 | 84 | 87 | 81 | 84 | 1.21 | 1.20 | 1.20 | 0.87 | 69.2 |
| 11000 | 5 | 101 | 108 | 98 | 74 | 89 | 80 | 76 | 80 | 80 | 90 | 81 | 89 | 1.20 | 1.19 | 1.19 | 0.86 | 69.9 |
| 12000 | -2.5 | 109 | 118 | 105 | 85 | 84 | 82 | 92 | 68 | 90 | 79 | 81 | 74 | 1.27 | 1.26 | 1.26 | 0.92 | 66.7 |
| 12000 | 0 | 106 | 115 | 102 | 82 | 86 | 89 | 88 | 72 | 87 | 83 | 81 | 79 | 1.26 | 1.25 | 1.25 | 0.91 | 67.4 |
| 12000 | 2.5 | 104 | 112 | 100 | 78 | 88 | 90 | 83 | 75 | 84 | 86 | 81 | 83 | 1.25 | 1.24 | 1.24 | 0.90 | 68.2 |
| 12000 | 5 | 102 | 109 | 98 | 74 | 89 | 83 | 78 | 78 | 81 | 89 | 81 | 88 | 1.24 | 1.23 | 1.23 | 0.89 | 68.9 |
| 13000 | -2.5 | 109 | 118 | 104 | 85 | 84 | 79 | 93 | 66 | 91 | 79 | 81 | 73 | 1.30 | 1.29 | 1.30 | 0.95 | 65.9 |
| 13000 | 0 | 108 | 116 | 102 | 82 | 86 | 86 | 90 | 69 | 89 | 82 | 82 | 77 | 1.30 | 1.29 | 1.30 | 0.95 | 66.3 |
| 13000 | 2.5 | 105 | 112 | 100 | 78 | 88 | 91 | 84 | 74 | 85 | 86 | 82 | 83 | 1.28 | 1.27 | 1.28 | 0.93 | 67.3 |
| 13000 | 5 | 102 | 109 | 97 | 74 | 89 | 85 | 80 | 77 | 82 | 89 | 82 | 87 | 1.27 | 1.26 | 1.27 | 0.92 | 68.0 |
| 17000 | -2.5 | 111 | 120 | 104 | 85 | 83 | 71 | 97 | 62 | 92 | 77 | 83 | 71 | 1.39 | 1.39 | 1.39 | 1.03 | 63.7 |
| 17000 | 0 | 109 | 117 | 101 | 81 | 86 | 81 | 94 | 67 | 90 | 81 | 83 | 76 | 1.39 | 1.38 | 1.38 | 1.02 | 64.3 |
| 17000 | 2.5 | 106 | 113 | 99 | 78 | 88 | 90 | 89 | 70 | 87 | 84 | 83 | 81 | 1.38 | 1.37 | 1.38 | 1.01 | 64.9 |
| 17000 | 5 | 103 | 110 | 97 | 73 | 89 | 91 | 84 | 74 | 83 | 87 | 84 | 86 | 1.37 | 1.36 | 1.37 | 1.00 | 65.6 |
| 20000 | -2.5 | 112 | 120 | 104 | 85 | 83 | 68 | 97 | 60 | 93 | 76 | 83 | 70 | 1.44 | 1.44 | 1.44 | 1.07 | 62.6 |
| 20000 | 0 | 109 | 117 | 101 | 81 | 85 | 78 | 96 | 65 | 90 | 80 | 84 | 75 | 1.43 | 1.43 | 1.43 | 1.06 | 63.2 |
| 20000 | 2.5 | 107 | 114 | 99 | 78 | 88 | 88 | 91 | 69 | 87 | 83 | 84 | 80 | 1.43 | 1.42 | 1.42 | 1.05 | 63.8 |
| 20000 | 5 | 104 | 110 | 96 | 73 | 89 | 92 | 86 | 72 | 84 | 87 | 84 | 85 | 1.42 | 1.41 | 1.42 | 1.04 | 64.4 |

EP 2 281 606 A1

FIG. 55B

FIG. 55A

94

FIG. 56

Wavelength [nm]

Relative value

(SAE phosphor emission spectrum)

FIG. 57

| Tc | Duv | Color rendering indexes | | | | | | | | | | | | Action power for per unit luminous flux | | | | Efficiency [lm/W] |
|---|---|---|---|---|---|---|---|---|---|---|---|---|---|---|---|---|---|---|
| | | Ga4 | Ga | M | PS | Ra | R9 | R10 | R11 | R12 | R13 | R14 | R15 | Br | Th | c(λ) | z(λ) | |
| 7100 | -2.5 | 98 | 108 | 99 | 93 | 89 | 81 | 95 | 96 | 93 | 80 | 87 | 80 | 1.03 | 0.99 | 1.05 | 0.69 | 77.3 |
| 7100 | 0 | 97 | 106 | 97 | 91 | 91 | 74 | 99 | 97 | 95 | 83 | 87 | 84 | 1.02 | 0.98 | 1.03 | 0.68 | 78.2 |
| 7100 | 2.5 | 95 | 104 | 96 | 88 | 93 | 67 | 94 | 94 | 94 | 86 | 86 | 88 | 1.00 | 0.96 | 1.01 | 0.66 | 79.2 |
| 7100 | 5 | 94 | 101 | 94 | 85 | 94 | 59 | 89 | 90 | 91 | 89 | 86 | 92 | 0.99 | 0.95 | 1.00 | 0.65 | 80.2 |
| 8000 | -2.5 | 99 | 109 | 97 | 92 | 88 | 87 | 90 | 94 | 90 | 78 | 89 | 78 | 1.11 | 1.07 | 1.13 | 0.75 | 75.5 |
| 8000 | 0 | 98 | 106 | 96 | 90 | 90 | 82 | 95 | 97 | 93 | 82 | 88 | 82 | 1.09 | 1.05 | 1.11 | 0.74 | 76.4 |
| 8000 | 2.5 | 96 | 104 | 94 | 87 | 92 | 74 | 99 | 95 | 94 | 85 | 88 | 86 | 1.08 | 1.04 | 1.10 | 0.72 | 77.3 |
| 8000 | 5 | 94 | 102 | 93 | 85 | 94 | 66 | 94 | 91 | 93 | 88 | 88 | 90 | 1.07 | 1.03 | 1.08 | 0.71 | 78.3 |
| 10000 | -2.5 | 102 | 110 | 95 | 92 | 86 | 85 | 81 | 90 | 87 | 75 | 91 | 75 | 1.24 | 1.20 | 1.27 | 0.85 | 72.6 |
| 10000 | 0 | 100 | 107 | 94 | 90 | 89 | 88 | 86 | 94 | 90 | 79 | 91 | 79 | 1.23 | 1.18 | 1.26 | 0.84 | 73.4 |
| 10000 | 2.5 | 98 | 105 | 92 | 87 | 91 | 85 | 91 | 96 | 92 | 82 | 91 | 83 | 1.21 | 1.17 | 1.24 | 0.83 | 74.2 |
| 10000 | 5 | 96 | 102 | 90 | 83 | 92 | 77 | 96 | 94 | 93 | 86 | 91 | 87 | 1.20 | 1.16 | 1.23 | 0.82 | 75.1 |
| 11000 | -2.5 | 102 | 110 | 94 | 92 | 86 | 82 | 77 | 89 | 85 | 74 | 92 | 73 | 1.29 | 1.24 | 1.32 | 0.89 | 71.5 |
| 11000 | 0 | 100 | 108 | 93 | 89 | 88 | 88 | 82 | 93 | 89 | 78 | 92 | 78 | 1.28 | 1.23 | 1.31 | 0.88 | 72.3 |
| 11000 | 2.5 | 98 | 105 | 91 | 86 | 90 | 87 | 88 | 96 | 91 | 81 | 92 | 82 | 1.27 | 1.22 | 1.30 | 0.87 | 73.1 |
| 11000 | 5 | 96 | 102 | 89 | 83 | 92 | 80 | 93 | 94 | 93 | 85 | 92 | 86 | 1.25 | 1.21 | 1.28 | 0.86 | 73.9 |
| 12000 | -2.5 | 103 | 111 | 94 | 92 | 85 | 79 | 74 | 87 | 84 | 73 | 93 | 72 | 1.33 | 1.29 | 1.37 | 0.92 | 70.6 |
| 12000 | 0 | 101 | 108 | 92 | 89 | 87 | 86 | 79 | 92 | 87 | 77 | 93 | 77 | 1.32 | 1.27 | 1.35 | 0.91 | 71.4 |
| 12000 | 2.5 | 99 | 105 | 90 | 86 | 90 | 88 | 85 | 95 | 90 | 80 | 93 | 81 | 1.31 | 1.26 | 1.34 | 0.90 | 72.1 |
| 12000 | 5 | 97 | 103 | 89 | 83 | 91 | 83 | 90 | 95 | 92 | 84 | 94 | 85 | 1.30 | 1.25 | 1.33 | 0.89 | 72.9 |
| 13000 | -2.5 | 103 | 111 | 93 | 92 | 84 | 77 | 71 | 86 | 83 | 72 | 94 | 72 | 1.37 | 1.32 | 1.40 | 0.95 | 69.9 |
| 13000 | 0 | 101 | 108 | 92 | 89 | 87 | 85 | 77 | 91 | 86 | 76 | 94 | 76 | 1.35 | 1.31 | 1.39 | 0.94 | 70.6 |
| 13000 | 2.5 | 99 | 106 | 90 | 86 | 89 | 89 | 82 | 95 | 89 | 80 | 94 | 80 | 1.34 | 1.30 | 1.38 | 0.93 | 71.3 |
| 13000 | 5 | 97 | 103 | 88 | 83 | 91 | 85 | 87 | 95 | 91 | 83 | 94 | 85 | 1.34 | 1.29 | 1.37 | 0.92 | 72.1 |
| 17000 | -2.5 | 104 | 112 | 92 | 92 | 82 | 69 | 63 | 84 | 80 | 70 | 95 | 69 | 1.47 | 1.42 | 1.51 | 1.03 | 67.8 |
| 17000 | 0 | 102 | 109 | 90 | 90 | 85 | 78 | 68 | 89 | 84 | 74 | 95 | 74 | 1.46 | 1.41 | 1.50 | 1.02 | 68.4 |
| 17000 | 2.5 | 100 | 106 | 89 | 86 | 88 | 87 | 74 | 93 | 87 | 78 | 96 | 78 | 1.45 | 1.40 | 1.49 | 1.01 | 69.1 |
| 17000 | 5 | 98 | 103 | 87 | 82 | 90 | 89 | 79 | 96 | 89 | 81 | 97 | 83 | 1.44 | 1.39 | 1.48 | 1.00 | 69.8 |
| 20000 | -2.5 | 105 | 113 | 91 | 93 | 81 | 65 | 58 | 83 | 79 | 69 | 95 | 68 | 1.52 | 1.47 | 1.56 | 1.07 | 66.8 |
| 20000 | 0 | 103 | 110 | 90 | 90 | 84 | 75 | 64 | 88 | 82 | 73 | 95 | 73 | 1.51 | 1.46 | 1.55 | 1.06 | 67.4 |
| 20000 | 2.5 | 100 | 107 | 88 | 86 | 87 | 85 | 70 | 92 | 85 | 77 | 96 | 77 | 1.50 | 1.45 | 1.55 | 1.05 | 68.0 |
| 20000 | 5 | 98 | 104 | 86 | 83 | 89 | 89 | 76 | 95 | 88 | 80 | 97 | 82 | 1.49 | 1.44 | 1.54 | 1.04 | 68.7 |

FIG. 58B

FIG. 58A

FIG.59

EP 2 281 606 A1

FIG.60B

FIG.60A

FIG.61

1000

600

500

200

300

400

<table>
<tr><td colspan="2" align="center">**INTERNATIONAL SEARCH REPORT**</td><td>International application No.<br>PCT/JP2009/001874</td></tr>
</table>

A. CLASSIFICATION OF SUBJECT MATTER
*A61N5/06*(2006.01)i

According to International Patent Classification (IPC) or to both national classification and IPC

B. FIELDS SEARCHED

Minimum documentation searched (classification system followed by classification symbols)
A61N5/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2007-532724 A (Patent Treuhand Gesellschaft fur elektrische Gluhlampen mbH), 15 November, 2007 (15.11.07), Full text; all drawings & US 2008/0265207 A1 & WO 2005/100508 A1 | 1-14 |
| A | JP 2007-299714 A (Osram-Melco Ltd.), 15 November, 2007 (15.11.07), Full text; all drawings (Family: none) | 1-14 |
| A | JP 2006-299097 A (Matsushita Electric Works, Ltd.), 02 November, 2006 (02.11.06), Full text; all drawings (Family: none) | 1-14 |

☐ Further documents are listed in the continuation of Box C.       ☐ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search<br>03 July, 2009 (03.07.09) | Date of mailing of the international search report<br>14 July, 2009 (14.07.09) |
|---|---|
| Name and mailing address of the ISA/<br>Japanese Patent Office | Authorized officer |
| Facsimile No. | Telephone No. |

Form PCT/ISA/210 (second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005529462 A
- JP 2004508106 A
- JP H6314595 B
- JP H4284347 B

**Non-patent literature cited in the description**

- **Kenjiro Hashimoto.** New Method for Specifying Color-Rendering Properties of Light Sources Based on Feeling of Contrast. *Color research and application,* October 2007, vol. 32 (5), 361
- **Kenjiro Hashimoto.** Preference Index for Japanese Complexion Color under Illumination. *Journal of the Illuminating Engineering Institute of Japan,* 1998, vol. 82 (11), 895
- **Seishi Sekine.** Spectral Distributions of Clear Sky and Their Chromaticities. *Journal of the Illuminating Engineering Institute of Japan,* 1989, vol. 73 (2), 3
- **Okada Kiyoshi.** Report of the Investigative Committee for Daylight Standard Establishment. *Journal of the Illuminating Engineering Institute of Japan,* 1970, vol. 54 (3), 15
- New Edition Color Science Handbook. The Color Science Association of Japan, 1985, 67
- Effects of High Color Temperature Illumination on Psychological and Physiological Function in Working. **Takashi Kanai.** Master's Thesis. Chiba University Graduate School of Science and Technology, 2000